(19) Europäisches Patentamt
European Patent Office
Office européen des brevets

(11) Publication number: **0 298 466 A2**


# (12) EUROPEAN PATENT APPLICATION


(21) Application number: 88110814.6

(22) Date of filing: 06.07.88

(51) Int. Cl.4: C07D 213/40 , C07D 213/38 , C07D 239/26 , C07D 409/12 , C07D 405/12 , C07D 401/12 , A61K 31/44 , A61K 31/505

(30) Priority: 10.07.87 US 72199
11.04.88 US 179616

(43) Date of publication of application:
11.01.89 Bulletin 89/02

(84) Designated Contracting States:
AT BE CH DE ES FR GB GR IT LI LU NL SE



(71) Applicant: F. HOFFMANN-LA ROCHE & CO. Aktiengesellschaft

CH-4002 Basel(CH)

(72) Inventor: Guthrie, Robert William
102 Alberta Drive
Saddle Brook, N.J. 07665(US)
Inventor: Kierstead, Richard Wightman
30 Willowbrook Drive
North Caldwell, N.J. 07006(US)
Inventor: Mullin, Jr. John Guilfoyle
519 Goffle Hill Road
Hawthorne, N.J.(US)
Inventor: Tilley, Jefferson Wright
19 Evergreen Drive
North Caldwell, N.J.(US)

(74) Representative: Lederer, Franz, Dr. et al
Patentanwält Dr. Franz Lederer Lucile Grahnstrasse 22
D-8000 München 80(DE)


## (54) Substituted alkene carboxylic acid amides and derivatives.


(57) There are described compounds of the general formula

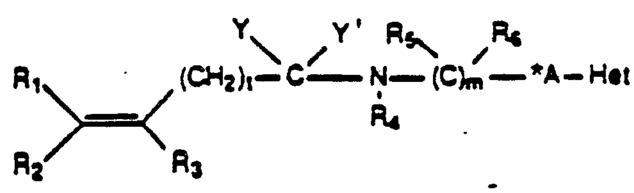

I

wherein Y and Y′ are hydrogen or taken together are O or S, *A is paraphenylene or $^*\text{-}(CH_2)_n\text{-}(X)_s\text{-}(CH_2)_r\text{-}$, X is O, S or -CH=CH-, n and r, independently are integers from 0 to 3, m is the integer 0 or 1, s is the integer 0 or 1, provided that when s is 1, n+m is at least 2, t is an integer from 0 to 10, $R_1$ and $R_2$, independently, are lower alkyl, lower alkenyl or aryl, or one of $R_1$ and $R_2$ is hydrogen and the other is


EP 0 298 466 A2

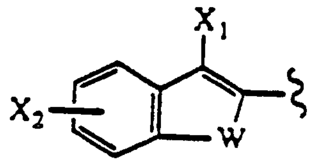

(a)

W is $-CX_3=CX_4-$, $-CH_2-CH_2-$, $-CH_2-$, O, S or $-NX_5-$, $X_1$ is lower alkyl, phenyl or phenyl with up to 3 substituents selected from lower alkoxy, lower alkyl and halogen, $X_2$, $X_3$ and $X_4$, independently, are hydrogen, lower alkyl, lower alkoxy or halogen, $X_5$ is lower alkyl, $R_3$ is hydrogen, lower alkyl or aryl, $R_4$ is hydrogen, lower alkyl, aryl, aryl-lower alkyl or acyl, $R_5$ is hydrogen or lower alkyl, $R_6$ is hydrogen,

lower alkyl, lower cycloalkyl, Het-lower alkyl or aryl, Het is a monocyclic 6-membered heteroaromatic radical containing one or two nitrogen atoms, which radical may be substituted by lower alkyl, halogen or aryl, and the asterisk denotes the point of attachment,

when $R_5$ and $R_6$ are different, enantiomers and racemic mixtures thereof, when $R_1$ and $R_2$ are different, geometric isomers thereof, and pharmaceutically acceptable acid addition salts thereof.

These compounds exhibit activity as platelet activating factor (PAF) antagonists and are, therefore, useful in disease states characterized by excess platelet activating factor or for the prevention and treatment of cardiovascular diseases, pulmonary diseases, immunological disorders, inflammatory diseases, dermatological disorders, shocks or transplant rejections.

## Substituted Alkene Carboxylic Acid Amides and Derivatives

The present invention relates to compounds of the general formula

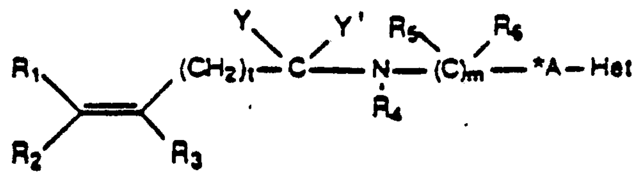

I

wherein Y and Y′ are hydrogen or taken together are O or S, *A is paraphenylene or *-$(CH_2)_n$-$(X)_s$-$(CH_2)_r$-, X is O, S or -CH=CH-, n and r, independently, are integers from 0 to 3, m is the integer 0 or 1, s is the integer 0 or 1, provided that when s is 1, n+m is at least 2, t is an integer from 0 to 10, $R_1$ and $R_2$, independently, are lower alkyl, lower alkenyl or aryl, or one of $R_1$ and $R_2$ is hydrogen and the other is

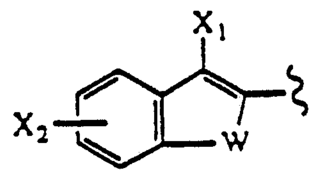

(a)

W is -$CX_3$=$CX_4$-, -$CH_2$-$CH_2$-, -$CH_2$-, O, S or -$NX_5$-, $X_1$ is lower alkyl, phenyl or phenyl with up to 3 substituents selected from lower alkoxy, lower alkyl or halogen, $X_2$, $X_3$ and $X_4$, independently, are hydrogen, lower alkyl, lower alkoxy or halogen, $X_5$ is lower alkyl, $R_3$ is hydrogen, lower alkyl or aryl, $R_4$ is hydrogen, lower alkyl, aryl, aryl-lower alkyl or acyl, $R_5$ is hydrogen or lower alkyl, $R_6$ is hydrogen, lower alkyl, lower cycloalkyl, Het-lower alkyl or aryl, Het is a 6-membered heteroaromatic radical containing one or two nitrogen atoms, which radical may be substituted by lower alkyl, halogen or aryl, and the asterisk denotes the point of attachment,

when $R_5$ and $R_6$ are different, to enantiomers and racemic mixtures thereof, when $R_1$ and $R_2$ are different, to geometric isomers thereof, and pharmaceutically acceptable acid addition salts thereof.

The compounds of formula I exhibit activity as platelet activating factor (PAF) antagonists and are, therefore, useful in disease states characterized by excess platelet activating factor or for the prevention or treatment of cardiovascular diseases, pulmonary diseases, immunological disorders, inflammatory diseases, dermatological disorders, shocks or transplant rejections.

Objects of the present invention are the aforementioned compounds per se and as therapeutically active substances, a process for their manufacture, pharmaceutical compositions on their basis and a process for the manufacture of said pharmaceutical compositions, as well as the use of these compounds in the prevention or treatment of diseases and in the manufacture of pharmaceutical compositions.

As used herein, the term "lower alkyl" denotes a straight or branched chain saturated hydrocarbon containing 1 to 7 carbon atoms, for example, methyl, ethyl, propyl, isopropyl, butyl, t-butyl, neopentyl, pentyl, heptyl, and the like. The term "lower cycloalkyl" denotes a cyclic alkyl group of 3 to 6 carbon atoms, for example, cyclopropyl, cyclohexyl, and the like. The term "lower alkoxy" denotes an alkyl ether group in which the alkyl group is as described above, for example, methoxy, ethoxy, propoxy, pentoxy and the like. The term "lower alkenyl" denotes a straight or branched chain unsaturated hydrocarbon containing 3 to 7 carbon atoms, for example, propenyl, butenyl and the like.

The term "halogen" denotes the four halogens bromine, chlorine, fluorine and iodine. The term "aryl" preferably denotes naphthalenyl or phenyl which may be mono-, di- or trisubstituted by halogen, trifluoromethyl, lower alkyl, lower alkoxy, nitro, phenyl or phenyl with up to 3 substituents selected from lower alkoxy, lower alkyl or halogen.

The term "acyl" denotes a "lower alkanoyl" group derived from an aliphatic carboxylic acid of 1 to 7 carbon atoms, for example, formyl, acetyl, propionyl, and the like, an aryl-lower alkanoyl group or an aroyl group derived from an aromatic carboxylic acid, such as benzoyl and the like.

The term "Het" denotes a monocyclic 6-membered heteroaromatic radical containing one or two nitrogen atoms, which radical may be substituted by lower alkyl, halogen or phenyl, for example, 3-pyridinyl, 2-pyridinyl, 6-methyl-3-pyridinyl, 2-methyl-3-pyridinyl, 5-pyrimidinyl and the like.

As already indicated the compounds of formula I, when $R_5$ and $R_6$ are different can exist as enantiomers or racemic mixtures.

The invention encompasses all the isomers and mixtures thereof.

A preferred group of compounds of formula I are those wherein $R_1$ and $R_2$, independently, are lower alkyl or aryl, or $R_1$ is hydrogen and $R_2$ is

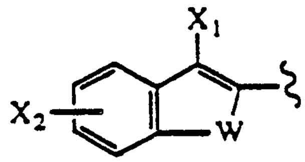

(a)

$X_1$, $X_2$ and W are as previously described, $R_3$ is hydrogen or lower alkyl, $R_4$ and $R_5$ are hydrogen, $R_6$ is hydrogen, lower alkyl or lower cycloakyl, *A is $^*-(CH_2)_n-(X)_s-(CH_2)_r-$, n+r is an integer from 2 to 6, s is the integer 0, Het is a monocyclic 6 membered heteroaromatic radical containing one or two nitrogen atoms, which radical may be substituted by lower alkyl, Y and Y′ are hydrogen or taken together are oxygen or sulfur, m is the integer 1 and t is an integer from 0 to 4.

A more preferred group of compounds of formula I are those wherein $R_1$ is hydrogen, $R_2$ is

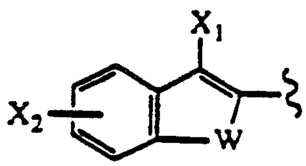

(a)

$X_1$, $X_2$ and W are as previously described, $R_3$ is hydrogen or lower alkyl, *A is $^*-(CH_2)_n-(X)_s-(CH_2)_r-$, n + r is 3, s is the integer 0, Het is pyridinyl or pyrimidinyl unsubstituted or substituted by lower alkyl, $R_4$ and $R_5$ are hydrogen, $R_6$ is hydrogen, lower alkyl or cyclopropyl, m is the integer 1, t is the integer 0, and Y and Y′ are hydrogen or taken together are oxygen.

Another more preferred group of compounds of formula I are those wherein $R_1$ is lower alkyl or aryl, $R_2$ is aryl, $R_3$ is hydrogen or lower alkyl, *A is $^*-(CH_2)_n-(X)_s-(CH_2)_r-$, n+r is 3, s is the integer 0, Het is pyridinyl or pyrimidinyl unsubstituted or substituted by lower alkyl, $R_4$ and $R_5$ are hydrogen, $R_6$ is hydrogen, lower alkyl or cyclopropyl, m is the integer 1, t is the integer 0 or 2, and Y and Y′ are hydrogen or taken together are oxygen,

A most preferred group of compounds of formula I are those wherein $R_1$ is hydrogen, $R_2$ is

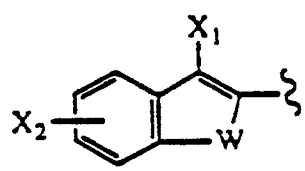

(a)

W is $-CX_3=CX_4-$, $-CH_2-$, O, S or $-NX_5-$, $X_1$ is butyl, pentyl, hexyl, phenyl or phenyl with up to 3 substituents selected from halogen or lower alkoxy, $X_2$, $X_3$ and $X_4$, independently, are hydrogen, lower alkoxy or halogen, $X_5$ is lower alkyl, $R_3$, $R_4$ and $R_5$ are hydrogen, $R_6$ is hydrogen, lower alkyl or cyclopropyl, m is the integer 1, t is the integer 0, Y and Y′ taken together are oxygen, and Het is 3-pyridinyl or 2-methyl-3-pyridinyl.

Another most preferred group of compounds of formula I are those where $R_1$ is butyl, pentyl, hexyl, phenyl or phenyl with up to 3 substituents selected from halogen and lower alkoxy, $R_2$ is phenyl or phenyl with up to 3 substituents selected from halogen and lower alkoxy, $R_3$, $R_4$ and $R_5$ are hydrogen, $R_6$ is hydrogen, lower alkyl or cyclopropyl, m is the integer 1, t is the integer 2, Y and Y′ are hydrogen or taken together are oxygen and Het is 3-pyridinyl or 2-methyl-3-pyridinyl.

Preferred compounds of the invention are:

5,5-diphenyl-N-[4-(3-pyridinyl)butyl]-4-pentenamide;
5,5-bis(4-methoxyphenyl)-N-[4-(3-pyridinyl)butyl]-4-pentenamide;
5,5-bis(4-fluorophenyl)-N-[4-(3-pyridinyl)butyl]-4-pentenamide;
N-(5,5-diphenyl-4-pentenyl)-3-pyridinebutanamine;
[R-(E)]-3-(1-butyl-6-methoxy-2-naphthalenyl)-N-[1-methyl-4-(3-pyridinyl)butyl]-2-propenamide;
[R-(E)]-3-[6-methoxy-1-(4-methoxyphenyl)-2-naphthalenyl]-N-[1-methyl-4-(3-pyridinyl)butyl]-2-propenamide;
[R-(E)]-3-(1-butyl-4-methoxy-2-naphthalenyl)-N-[1-methyl-4-(3-pyridinyl)butyl]-2-propenamide;
(E)-3-(1-butyl-4-methoxy-2-naphthalenyl)-N-[6-(3-pyrimidinyl)hexyl]-2-propenamide;
[R-(E)]-3-(6-methoxy-3-pentylinden-2-yl)-N-[1-methyl-4-(3-pyridinyl)butyl]-2-propenamide;
[R-(E)]-3-(6-methoxy-3-pentylbenzo[b]thien-2-yl) N-[1-methyl-4-(3-pyridinyl)butyl]-2-propenamide;
[R-(E)]-3-(6-methoxy-3-pentylbenzofuran-2-yl)-N-[1-methyl-4-(3-pyridinyl)butyl]-2-propenamide and
[R-(E)]-3-(6-methoxy-1-methyl-3-pentylindol-2-yl)-N-[1-ethyl-4-(3-pyridinyl)butyl]-2-propenamide.

Exemplary compounds of formula I of the invention are:

N-(4,4-diphenyl-3-butenyl)-3-pyridinebutanamine;
N-[4,4-bis(4-methoxyphenyl)-3-butenyl]-3-pyridinebutanamine;
N-[5,5-bis(4-methoxyphenyl)-4-pentenyl]-3-pyridinebutanamine;
N-[5,5-bis(4-fluorophenyl)-4-pentenyl]-3-pyridinebutanamine;
N-[5,5-bis(3-methoxyphenyl)-4-pentenyl]-3-pyridinebutanamine;
N-(5,5-bis(4-methoxyphenyl)-4-pentenyl]-5-pyrimidinebutanamine;
N-[5,5-bis(3-methoxyphenyl)-4-pentenyl]-5-pyrimidinebutanamine;
N-[5,5-bis(3-fluorophenyl)-4-pentenyl]-5-pyrimidinebutanamine;
N-[5,5-bis(3-nitrophenyl)-4-pentenyl]-5-pyrimidinebutanamine;
N-[5,5-bis(4-nitrophenyl)-4-pentenyl-5-pyrimidinebutanamine;
N-[5,5-bis(3-chlorophenyl)-4-pentenyl]-3-pyridinebutanamine;
N-[5,5-bis(4-bromophenyl)-4-pentenyl]-3-pyridinebutanamine;
N-(5,5-diphenyl-4-pentenyl)-5-pyrimidinebutanamine;
N-(5,5-diphenyl-4-pentenyl)-5-pyrimidinehexanamine;
N-(5,5-diphenyl-4-pentenyl)-5-pyrimidinepropanamine;
N-(5,5-diphenyl)-4-pentenyl]-5-pyrimidinepentanamine;
N-(5,5-diphenyl)-4-pentenyl]-N-methyl-5-pyrimidinebutanamine;
N-[5,5-bis(4-methoxyphenyl)-4-pentenyl]-N-methyl-3-pyridinebutanamine;
(E)-N-(5-phenyl-4-octenyl)-5-pyrimidinebutanamine;
(Z)-N-(5-phenyl-4-nonenyl)-5-pyrimidinebutanamine;
(E)-N-[5-(4-methoxyphenyl)-4-pentenyl]-3-pyridinebutanamine;
(E)-N-[5-(4-methoxyphenyl)-4-hexenyl]-3-pyridinebutanamine;
(E)-N-[5-(4-methoxyphenyl)-4-octenyl]-3-pyridinebutanamine;
(Z)-N [5-(4-methoxyphenyl)-4-octenyl]-3-pyridinebutanamine;
(E)-N-(5-ethyl-4-nonenyl)-3-pyridinebutanamine;
(E)-N-(5 methyl-4-octenyl)-3-pyridinebutanamine;
N-(5-butyl-4-nonenyl)-3-pyridinebutanamine;
N-(5-butyl-4-nonenyl)-N-methyl-3-pyridinebutanamine;
N-[5,5-bis(4-methoxyphenyl)-4-pentenyl]-N-[4-(3-pyridinyl)butyl]formamide;
N-[5,5-bis(3-methoxyphenyl)-4-pentenyl]-N-[4-(3-pyridinyl)butyl]formamide;
N-[5,5-bis(4-methoxyphenyl)-4-pentenyl]-N-[4-(3-pyrimidinyl)butyl]formamide;
N-[5,5-bis(4-fluorophenyl)-4-pentenyl]-N-[4-(3-pyridinyl)butyl]formamide;
(E)-N-[5-(4-methoxyphenyl)-4-octenyl]-N-[4-(3-pyridinyl)butyl]formamide;
N-[6,6-bis(4-methoxyphenyl)-5-hexenyl]-N-[4-(3-pyridinyl)butyl]formamide;
N-[4,4-bis(4-methoxyphenyl)-3-butenyl]-N-[4-(3-pyridinyl)butyl]formamide;
(Z)-N-[5-(4-methoxyphenyl)-4-octenyl]-N-[4-(3-pyridinyl)butyl]formamide;
N-[5,5-bis(4-methoxyphenyl)-4-pentenyl]-N-[4-(3-pyridinyl)butyl]acetamide;
N-[5,5-bis(4-methoxyphenyl)-4-pentenyl]-N-[4-(3-pyridinyl)butyl]benzamide;
N-[5,5-bis(4-methoxyphenyl)-4-pentenyl]-N-[4-(3-pyridinyl)butyl]-4-fluorobenzamide;
N-[5,5-bis(4-methoxyphenyl)-4-pentenyl]-N-[4-(3-pyridinyl)butyl]-3-methoxybenzamide;
N-[5,5-bis(4-bromophenyl)-4-pentenyl]-N-[4-(3-pyridinyl)butyl]benzamide;
N-(5-butyl-4-nonenyl)-N-[4-(3-pyridinyl)butyl]benzamide;
N-(5-butyl-4-nonenyl)-N-[4-(3-pyridinyl)butyl]acetamide;
N-(5-butyl-4-nonenyl)-N-[4-(3-pyridinyl)butyl]formamide;
5,5-diphenyl-N-[4-(3-pyrimidinyl)butyl]-4-pentenamide;

5,5-diphenyl-N-[5-(3-pyrimidinyl)pentyl]-4-pentenamide;
5,5-diphenyl-N-[4-(3-pyrimidinyl)propyl]-4-pentenamide;
(R)-5,5-diphenyl-N-[1-methyl-4-(3-pyridinyl)butyl]-4-pentenamide;
(R)-5,5-bis(4-methoxyphenyl)-N-[1-methyl-4-(3-pyridinyl)butyl]-4-pentenamide;
(rac)-5,5-bis(4-methoxyphenyl)-N-[1-methyl-3-(3-pyridinyl)propyl]-4-pentenamide;
(R)-5,5-bis(4-methoxyphenyl)-N-[1-ethyl-4-(3-pyridinyl)butyl]-4-pentenamide;
(R)-5,5-bis(3-methoxyphenyl)-N-[1-methyl-4-(3-pyridinyl)butyl]-4-pentenamide;
5,5-bis(4-methoxyphenyl)-N-[1,1-dimethyl-4-(3-pyridinyl)butyl]-4-pentenamide;
(R)-5,5-bis(4-methoxyphenyl)-N-[1-propyl-4-(3-pyridinyl) butyl]-4-pentenamide;
(rac)-6,6-bis(4-methoxyphenyl)-N-[1-methyl-4-(3-pyridinyl) butyl]-4-hexenamide;
(R)-5,5-bis(4-methoxyphenyl)-N-[1-propyl-4-(3-pyridinyl)butyl]-4-pentenamide;
(R)(E)-5-(4-methoxyphenyl)-5-phenyl-N-[1-methyl-4-(3-pyridinyl)butyl]-4-pentenamide;
(E)-5-(4-methoxyphenyl)-5-phenyl-N-[4-(3-pyridinyl)butyl]-4-pentenamide;
(R)(E)-5-(4-methoxyphenyl)-5-methyl-N-[1-methyl-4-(3-pyridinyl)butyl]-4-pentenamide;
(R)(Z)-5-(4-methoxyphenyl)-5-methyl-N-[1-methyl-4-(3-pyridinyl)butyl]-4-pentenamide;
(rac)(E)-5-(2-methoxyphenyl)-5-methyl-N-[1-methyl-4-(3-pyridinyl)butyl]-4-pentenamide;
(R)-5,5-bis(3-chlorophenyl)-N-[1-methyl-4-(3-pyridinyl)butyl]-4-pentenamide;
5,5-bis(4-methylphenyl)-N-[4-(3-pyridinyl)butyl]-4-pentenamide;
(R)-5,5-bis(3,4-dimethoxyphenyl) N-[1-methyl-4-(3-pyridinyl)butyl]-4-pentenamide;
(R)-5,5-bis(3,4-dichlorophenyl)-N-[1-methyl-4-(3-pyridinyl)butyl]-4-pentenamide;
(R)(E)-5-(3-methoxyphenyl)-5-(4-fluorophenyl)-N-[1-methyl-4-(3-pyridinyl)butyl]-4-pentenamide;
(R)-5,5-bis(3-methylphenyl)-N-[1-methyl-4-(3-pyridinyl)butyl]-4-pentenamide;
(R)-5,5-bis(3-methoxyphenyl)-N-[1-methyl-4-(3-pyridinyl)butyl]-5-hexenamide;
(R)-5,5-bis(3-methoxyphenyl)-N-[1-methyl-4-(3-pyridinyl)butyl]-3-butenamide;
(R)-5,5-bis(4-methoxyphenyl)-N-[1-methyl-4-(2-methyl-3-pyridinyl)butyl]-4-pentenamide;
(R)-5,5-bis(3-methoxyphenyl)-N-[1-methyl-4-(6-methyl-3-pyridinyl)butyl]-4-pentenamide;
(rac)-5,5-bis(3-methoxyphenyl)-N-[1-methyl-4-(2-ethyl-3-pyridinyl)butyl]-4-pentenamide;
[R-(E)]-3-(6-methoxy-1-propyl-2-naphthalenyl)-N-[1-methyl-4-(3-pyridinyl)butyl]-2-propenamide;
[R-(E)]-3-(6-methoxy-1-pentyl-2-naphthalenyl)-N-[1-methyl-4-(3-pyridinyl)butyl]-2-propenamide;
(E)-3-(1-butyl-6-methoxy-2-naphthalenyl)-N-[4-(3-pyridinyl)-butyl]-2-propenamide;
(E)-3-(1-butyl-6-methoxy-2-naphthalenyl)-N-[4-(3-pyrimi dinyl)-butyl]-2-propenamide;
[R-(E)]-3-(1-butyl-6-methoxy-2-naphthalenyl)-N-[1-ethyl 4-(3-pyridinyl)butyl]-2-propenamide;
[R-(E)]-3-(3,4-dihydro-6-methoxy-1-propyl-2-naphthalenyl)-N-[1-methyl-4-(3-pyridinyl)butyl]-2-propenamide;
[R-(E)]-3-(3,4-dihydro-6-methoxy-1-pentyl-2-naphthalenyl)-N-[1-methyl-4-(3-pyridinyl)butyl]-2-propenamide;
(E)-3-(1-butyl-3,4-dihydro-6-methoxy-2-naphthalenyl)-N-[4-(3-pyridinyl)butyl]-2-propenamide;
(E)-3-(1-butyl-3,4-dihydro-6-methoxy-2-naphthalenyl)-N-[4-(3-pyrimidinyl)butyl]-2-propenamide;
[R-(E)]-3-(1-butyl-3,4-dihydro-6-methoxy-2 naphthalenyl)-N-[1-ethyl-4-(3-pyridinyl)butyl]-2-propenamide;
[R-(E)]-3-[3,4-dihydro-6-methoxy-1-(4-methylphenyl)-2-napht-alenyl]-N-[1-methyl-4-(3-pyridinyl)butyl]-2-propenamide;
[R-(E)]-3 [3,4-dihydro-6 methoxy-1-(3-methoxyphenyl)-2-naphthalenyl]-N-[1-methyl-4-(3-pyridinyl)butyl]-2-propenamide;
[R-(E)]-3-[3,4-dihydro-6-methoxy-1-(4-methoxyphenyl)-2-naphthalenyl]-N-[1-methyl-4-(3-pyridinyl)butyl]-2-propenamide;
[R-(E)]-3-[1-(4-chlorophenyl)-3,4-dihydro-6-methoxy)-2-naphthalenyl]-N-[1-methyl-4-(3-pyridinyl)butyl]-2-propenamide;
(E)-3-[6-methoxy-1-(4-methoxyphenyl)-2-naphthalenyl]-N-[4-(3-pyridinyl)butyl]-2-propenamide;
[R-(E)]-3-[6-methoxy-1-(4-methylphenyl)-2-naphthalenyl]-N-[1-methyl-4-(3-pyridinyl)butyl]-2-propenamide;
[R-(E)]-3-[6-methoxy-1-(3-methoxyphenyl)-2-naphthalenyl]-N-[1-methyl-4-(3-pyridinyl)butyl]-2-propenamide;
[R-(E)]-3-[6-methyl-1-(4-methoxyphenyl)-2-naphthalenyl] N-[1-methyl-4-(3-pyridinyl)butyl]-2-propenamide;
[R-(E)]-3-[1-(4-chlorophenyl)-6-methoxy)-2-naphthalenyl]-N-[1-methyl-4-(3-pyridinyl)butyl]-2-propenamide;
[R-(E)]-3-(1-butyl-5-methoxy-2-naphthalenyl)-N-[1-methyl-4-(3-pyridinyl)butyl]-2-propenamide;
[R-(E)]-3-(5-methoxy-1-pentyl-2 naphthalenyl)-N-[1- methyl- 4-(3-pyridinyl)butyl]-2-propenamide;
(E)-3-(1-butyl-5-methoxy-2-naphthalenyl)-N-[4-(3-pyridinyl)-butyl]-2-propenamide;
(E)-3-(1-butyl-5-methoxy-2-naphthalenyl)-N-[4-(3-pyrimidinyl)-butyl]-2-propenamide;
[R-(E)]-3-(1-butyl-5-methoxy-2-naphthalenyl)-N-[1-ethyl-4-(3-pyridinyl)butyl]-2-propenamide;
[R-(E)]-3-(1-butyl-7-methoxy-2-naphthalenyl)-N-[1-methyl-4-(3-pyridinyl)butyl]-2-propenamide;
[R-(E)]-3-(7-methoxy-1-pentyl-2-naphthalenyl)-N-[1-methyl-4-(3-pyridinyl)butyl]-2-propenamide;

(E)-3-(1-butyl-7-methyl-2-naphthalenyl)-N-[4-(3-pyridinyl)butyl]-2-propenamide;
(E)-3-(1-butyl-7-methoxy-2-naphthalenyl)-N-[4-(3-pyrimidinyl)-butyl]-2-propenamide;
[R-(E)]-3-(1-butyl-7-methoxy-2-naphthalenyl)-N-[1-ethyl-4-(3-pyridinyl)butyl]-2-propenamide;
[(S-(E)]-3-(1-butyl-4,7-dimethoxy-2-naphthalenyl)-N-[1-methyl-4-(3-pyridinyl)butyl]-2-propenamide;
[R-(E)]-3-(4,7-dimethoxy-1-pentyl-2-naphthalenyl)-N-[1-methyl-4 (3-pyridinyl)butyl]-2-propenamide;
(E)-3-(1-butyl-4,7-dimethoxy-2-naphthalenyl)-N-[4-(3-pyridinyl)butyl]-2-propenamide;
(E)-3-(1-butyl-4,7-dimethoxy-2-naphthalenyl)-N-[4-(3-pyrimi-dinyl)butyl]-2-propenamide;
[R-(E)]-3-(1-butyl-4,7-dimethoxy-2-naphthalenyl)-N-[1-ethyl-4-(3-pyridinyl)butyl]-2-propenamide;
[R-(E)]-3-(1-butyl-4-methyl-2-naphthalenyl)-N-[1-methyl-4-(3-pyridinyl)butyl]-2-propenamide;
(E)-3-(1-butyl-4 methoxy-2-naphthalenyl)-N-[6-(3-pyrimidinyl)-hexyl]-2-propenamide;
[R-(E)]-3-(4-methoxy-1-pentyl-2-naphthalenyl)-N-[1-methyl-4-(3-pyridinyl)butyl]-2-propenamide;
(E)-3-(1-butyl-4-methoxy-2-naphthalenyl)-N-[4-(3-pyridinyl)-butyl]-2-propenamide;
(E)-3-(1-butyl-4-methoxy-2-naphthalenyl)-N-[4-(3-pyrimidinyl)-butyl]-2-propenamide;
[R-(E)]-3-(1-butyl-4-methoxy-2-naphthalenyl)-N-[1-ethyl-4-(3-pyridinyl)butyl]-2-propenamide;
(E)-3-[7-methyl-1-(3-methoxyphenyl)-2 naphthalenyl]-N-[4-(3-pyridinyl)butyl]-2-propenamide;
[R-(E)]-3-(6-methoxy-3-pentylinden-2-yl)-N-[1-methyl-4-(3-pyridinyl)butyl]-2-propenamide;
(E)-3-(6-methoxy-3-pentylinden-2-yl)-N-[4-(3-pyridinyl)butyl]-2-propenamide;
(E)-3-(6-methoxy-3-pentylinden-2-yl)-N-[4-(3-pyrimidinyl)butyl]-2-propenamide;
[R-(E)]-3-(6-methoxy-3-propylinden-2-yl)-N-[1-methyl-4-(3-pyridinyl)butyl]-2-propenamide;
[R-(E)]-3-(6-chloro-3-pentylinden-2-yl)-N-[1-methyl-4-(3-pyridinyl)butyl]-2-propenamide;
[R-(E)]-3-(6-methyl-3-methylinden-2-yl)-N-[1-methyl-4-(3-pyridinyl)butyl]-2-propenamide;
[R-(E)]-3-(6-methoxy-3-pentylbenzo[b]thien-2-yl)-N-[1-ethyl-4-(3-pyridinyl)butyl]-2-propenamide;
[R-(E)]-3-(6-methyl-3-pentylbenzo[b]thien-2-yl)-N-[1-methyl-4-(3-pyridinyl)butyl]-2-propenamide;
[R-(E)]-3-(6-methoxy-3-propylbenzo[b]thien-2-yl)-N-[1-methyl-4-(3-pyridinyl)butyl]-2-propenamide;
[R-(E)]-3-(3-butyl-6-methoxybenzo[b]thien-2-yl)-N-[1-methyl-4-(3-pyridinyl)butyl]-2-propenamide;
(E)-3-(6-methoxy-3-pentylbenzo[b]thien-2-yl)-N-[4-(3-pyridinyl)butyl]-2-propenamide;
(E)-3-(6-methoxy-3-pentylbenzo[b]thien-2-yl)-N-[4-(3-pyrimi-dinyl)butyl]-2-propenamide;
(E)-3-(6-methoxy-3-pentylbenzofuran-2-yl)-N-[4-(3-pyridinyl)-butyl]-2-propenamide;
(E)-3-(6-methoxy-3-pentylbenzofuran-2-yl)-N-[6-(3-pyridinyl)hexyl]-2-propenamide;
(E)-3-(6-methyl-3-pentylbenzofuran-2-yl)-N-[4-(3-pyridinyl)butyl]-2-propenamide;
[R-(E)]-3-(6-chloro-3-pentylbenzofuran-2-yl)-N-[1-methyl-4-(3-pyridinyl)butyl]-2-propenamide;
[R-(E)] 3-(6-methoxy-3 propylbenzofuran-2-yl)-N-[1-methyl-4-(3 -pyridinyl)butyl]-2-propenamide;
[R-(E)]-3-(6-butyl-6-methoxybenzofuran-2-yl)-N-[1-methyl-4-(3-pyridinyl)butyl]-2-propenamide;
[R-(E)]-3-(6-methoxy-1-methyl-3-pentylindol-2-yl)-N-[1-methyl-4-(3-pyridinyl)butyl]-2-propenamide;
[R-(E)]-3-(6-chloro-1-methyl-3-pentylindol-2-yl)-N-[1-ethyl-4-(3-pyridinyl)butyl]-2-propenamide;
[R-(E)]-3-(1,6-dimethyl-3-pentylindol-2-yl)-N-[1-ethyl-4-(3-pyridinyl)butyl]-2-propenamide;
(E)-3-(6-methoxy-1-methyl-3-pentylindol-2-yl)-N-[4-(3-pyridinyl)butyl]-2-propenamide;
(E)-3-(6-methoxy-1-methyl-3-pentylindol-2-yl)-N-[4-(3-pyrim-id inyl)butyl]-2-propenamide;
(E)-3-(6-methoxy-1-methyl-3-pentylindol-2-yl)-N-[6-(3-pyridinyl)hexyl]-2-propenamide and
[R-(E)]-N-[1-ethyl-4-(3-pyridinyl)butyl]-3-(6-methoxy-1-methyl-3-propylindol-2-yl)-2-propenamide.

The compounds of formula I and their pharmaceutically acceptable acid addition salts can be prepared in accordance with the present invention by

a) reacting a carboxylic acid of the general formula

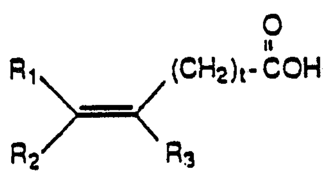

II

wherein $R_1$, $R_2$, $R_3$ and t are as above;

in the presence of a coupling agent or a reactive derivative of a carboxylic acid of the formula II above with an amine of the general formula

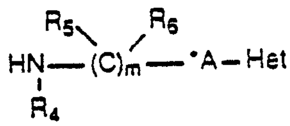

V

wherein $R_4$, $R_5$, $R_6$, *A, Het and m are as above, or

b) reacting a compound of the general formula

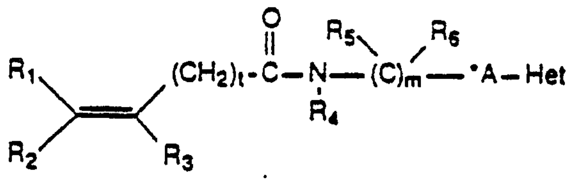

Ia

wherein $R_1$, $R_2$, $R_3$, $R_4$ $R_5$, $R_6$, *A, Het, m and t are as above, with phosphorous pentasulfide, or

c) reacting a compound of the general formula

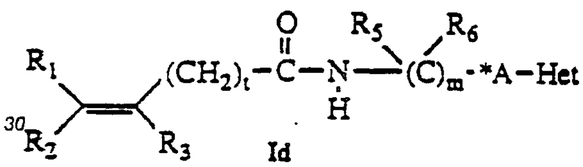

Id

or

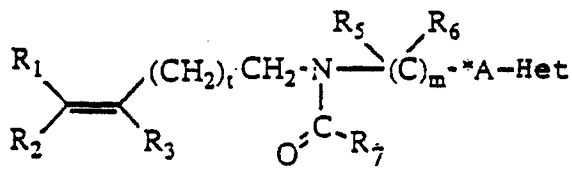

If

wherein $R_1$, $R_2$, $R_3$, $R_5$, $R_6$, *A, Het, m and t are as above and $R_7$ is $C_{1-6}$-alkyl, aryl-$C_{1-6}$-alkyl or aryl, with a reducing agent, or

d) reacting a compound of the general formula

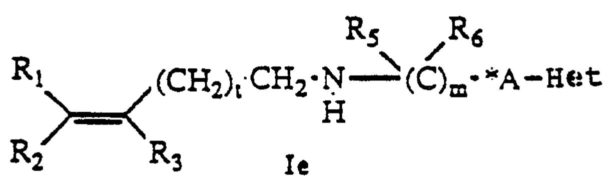

Ie

wherein $R_1$ $R_2$, $R_3$, $R_5$, $R_6$, *A, Het, m and t are as above, with an acylating agent yielding the group $R_7$-CO-, wherein $R_7$ is as defined above, and

e) if desired, converting a compound of formula I obtained into a pharmaceutically acceptable acid addition salt.

More particularly, the compounds of formula I and their pharmaceutically acceptable acid addition salts can be prepared as described hereinafter in more detail.

## Reaction Scheme I

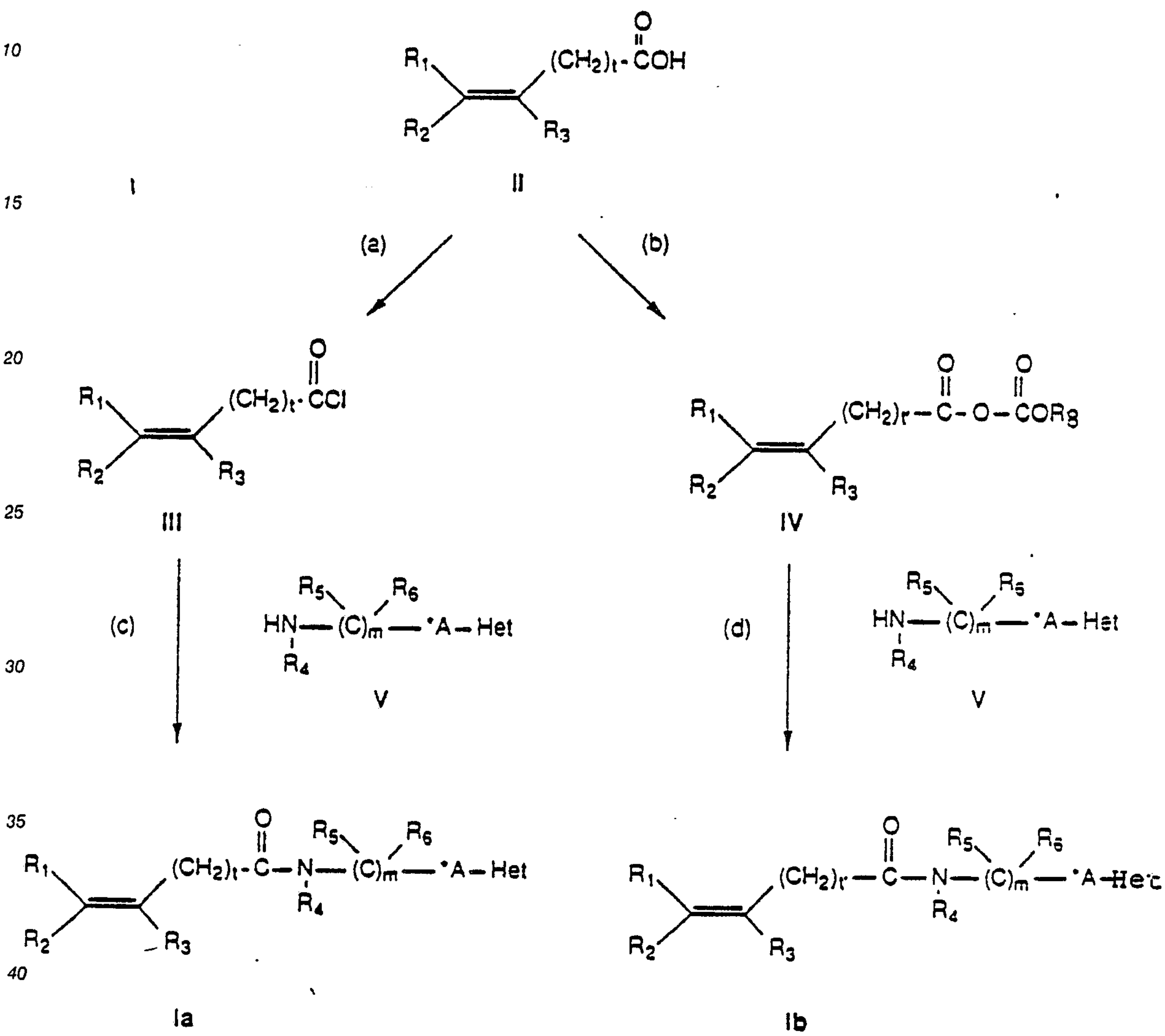


II III IV V Ia Ib

wherein $R_1$, $R_2$, $R_3$, $R_4$, $R_5$, $R_6$, *A, Het, m and t are as previously described and $R_8$ is lower alkyl, aryl-lower alkyl or aryl and $t'$ is 0 or 2-10.

In Reaction Scheme I, step (a), an alkenoic acid of formula II, which is a known compound or can be prepared according to known procedures, is treated with an acyl chloride forming reagent, preferably oxalyl chloride or thionyl chloride, in an inert solvent, preferably dichloromethane or toluene at a temperature of from -80° C to room temperature. The resulting corresponding compound of formula III can be isolated by evaporation of the reaction solvent. In accordance with step (c), the acid chloride of formula III is treated with an amine of formula V, which is a known compound or can be prepared according to known procedures, in the presence of a tertiary amine, preferably triethylamine, in an inert solvent such as dichloromethane or toluene at a temperature of from -80° C to room temperature.

In step (b), an alkenoic acid of formula II is treated with an alkyl chloroformate in the presence of a tertiary amine, preferably triethylamine, in an inert solvent, preferably diethyl ether or tetrahydrofuran, at a temperature of from -20° C to 10° C. In accordance with step d), the resulting mixed anhydride of formula IV

is treated in situ with an amine of formula V at a temperature of from -20° C to room temperature.

## Reaction Scheme II

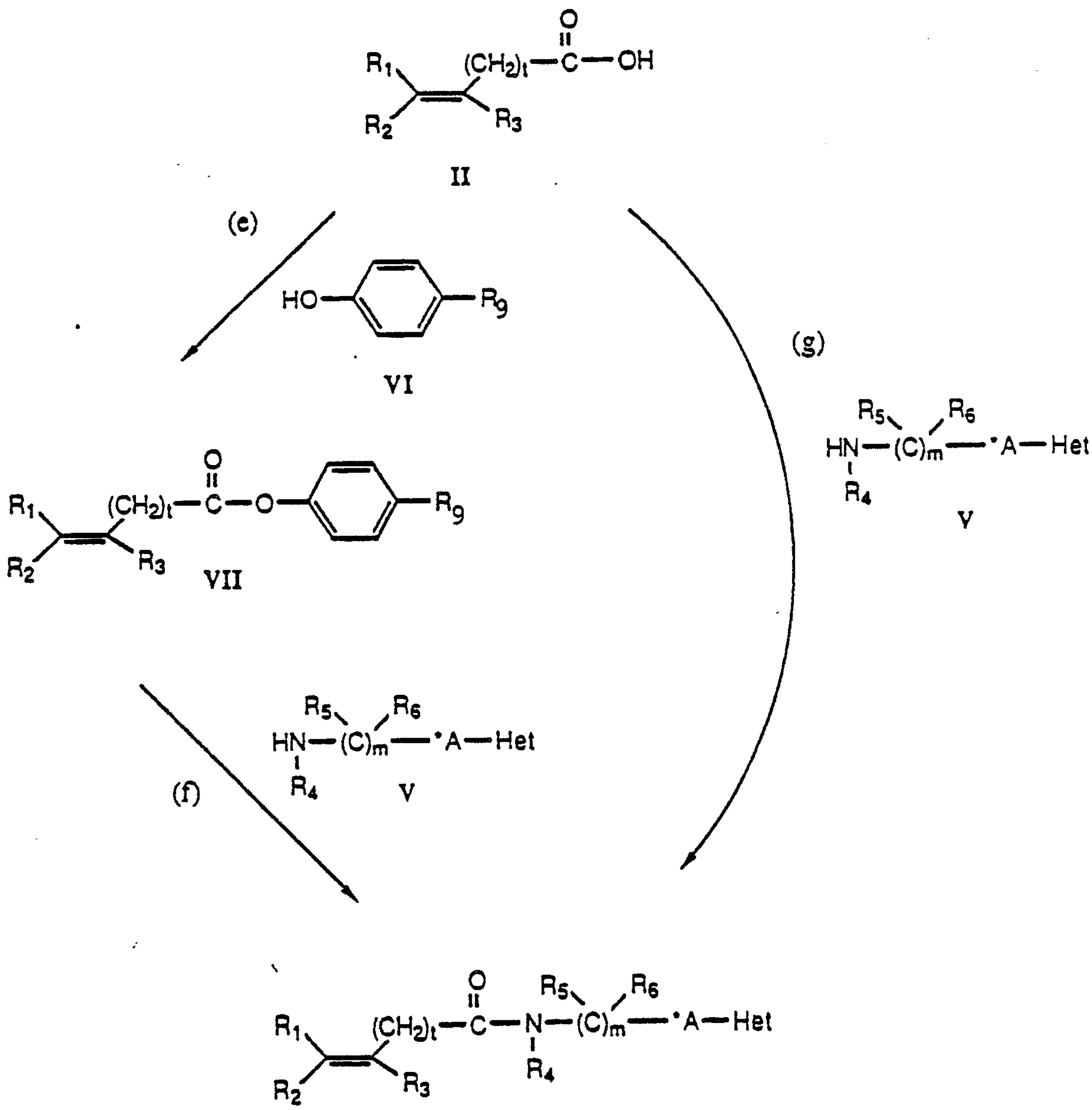


Ia

wherein $R_1$, $R_2$, $R_3$, $R_4$, $R_5$, $R_6$, *A, Het, m and t are as previously described and $R_9$ is hydrogen, halogen, lower alkoxy, lower alkyl, trihaloalkyl or nitro.

In Reaction Scheme II, step (e), a carboxylic acid of formula II is reacted with a phenol of formula VI, which is a known compound or can be prepared according to known procedures, in the presence of a coupling agent, preferably dicyclohexylcarbodiimide, in an inert solvent such as dichloromethane, diethyl ether and dimethylformamide, at a temperature of from -80° C to room temperature.

In step (f), an ester of formula VII is reacted with an amine of formula V in an inert solvent, preferably

tetrahydrofuran or diethyl ether, at a temperature of from -80° C to 100° C. $R_9$ is preferably nitro.

In step (g), a carboxylic acid of formula II is reacted with an amine of formula V in the presence of a suitable coupling agent, for example, dicyclohexylcarbodiimide, optionally in the presence of a promotor, for example, 1-hydroxybenzotriazole in a polar aprotic solvent, for example, dimethylformamide or pyridine at a temperature of from -10° C to 80° C.

## Reaction Scheme III

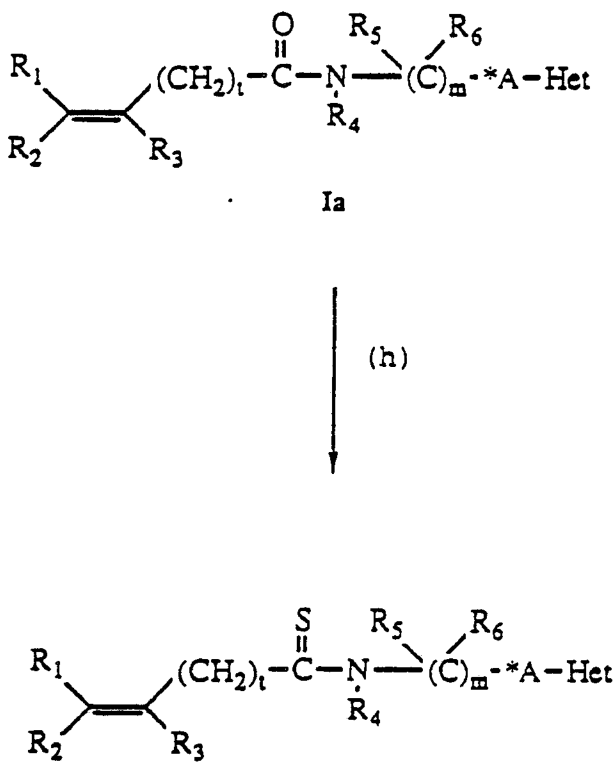


Ia

Ic

wherein $R_1$, $R_2$, $R_3$, $R_4$, $R_5$, $R_6$, *A, Het, m and t are as previously described.

In Reaction Scheme III, step (h), a compound of formula Ia is reacted with phosphorous pentasulfide in an inert solvent, for example, tetrahydrofuran or pyridine, at a temperature of from room temperature to reflux temperature.

Reaction Scheme IV

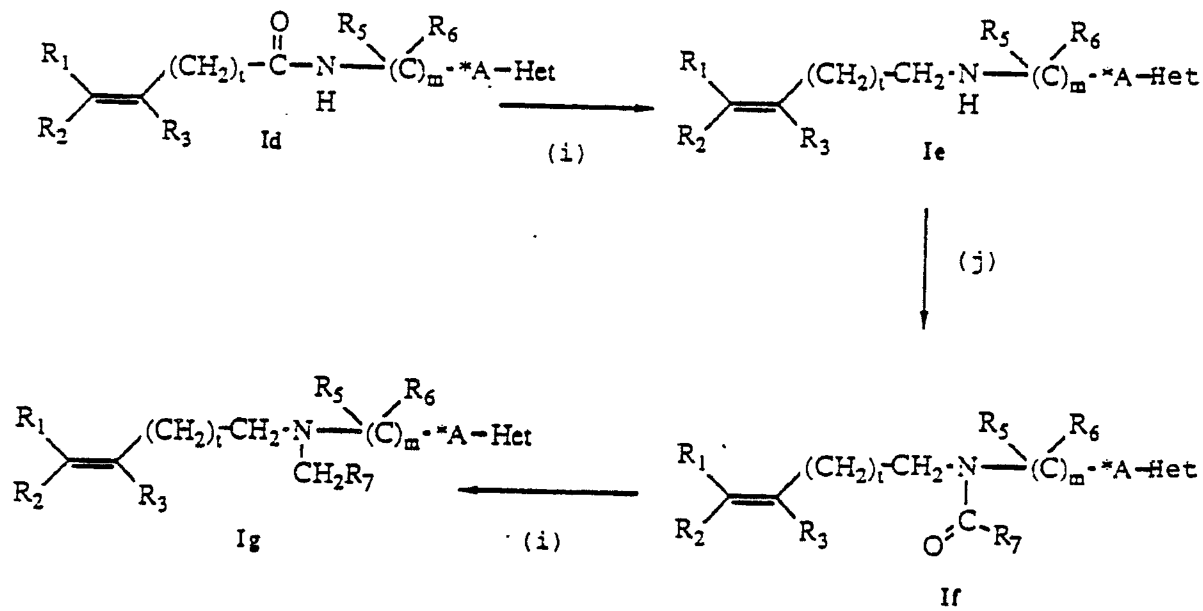


wherein $R_1$, $R_2$, $R_3$, $R_5$, $R_6$, *A, Het, m and t are as previously described and $R_7$ is hydrogen, $C_{1-6}$ alkyl, aryl-$C_{1-6}$-alkyl or aryl.

In Reaction Scheme IV, step (i), a compound of formula Id is reacted with a reducing agent, preferably sodium bis(2-methoxyethoxy)aluminum hydride, in an inert solvent, for example toluene, at a temperature of from room temperature to 100° C.

In step (j), an amine of formula Ie is reacted with an acylating agent yielding the group $R_7$-CO-, for example, an acyl halide, in an inert solvent, for example, toluene, in the presence of a proton acceptor, for example, triethylamine, at a temperature of from -50° C to room temperature, or when $R_7$ is hydrogen, with formic acid in an inert solvent, preferably toluene, at reflux temperature, to give the corresponding compound of formula If.

A compound of formula If, may then be converted to the corresponding compound of formula Ig, by repetition of the reaction conditions described in step (i) above.

## Reaction Scheme V

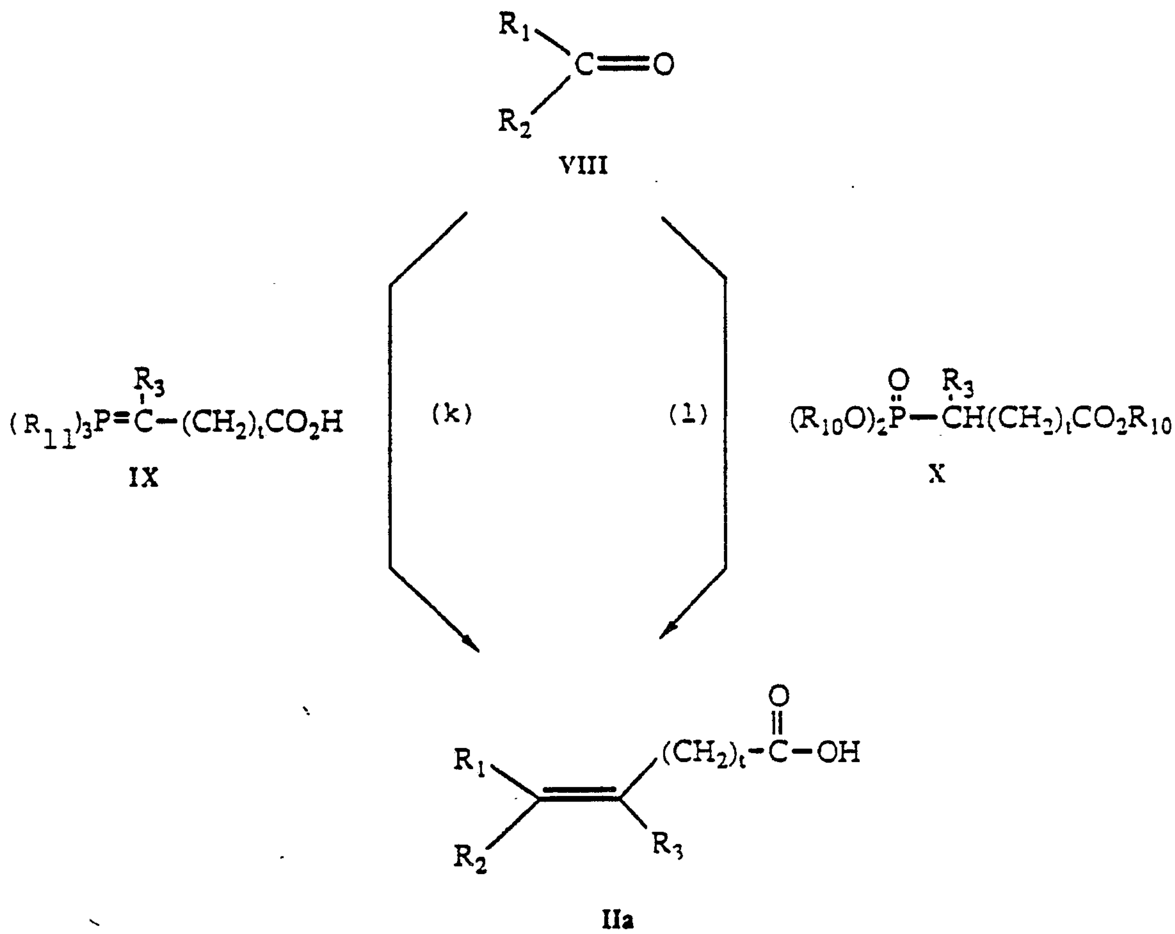


where $R_1$, $R_2$, $R_3$ and t are as previously described and $R_{10}$ and $R_{11}$ are, independently, lower alkyl, lower cycloakyl or aryl.

In Reaction Scheme V, step (1), a phosphonoacetate triester of formula X, which is a known compound or can be prepared according to known procedures, is converted to its corresponding carbanion utilizing a strong base, preferably sodium hydride or sodium amide, in an inert solvent, preferably dimethylsulfoxide, dimethylformamide or tetrahydrofuran, at temperatures of from 0° to 80° C, and then is reacted in situ with a carbonyl derivative of formula VIII.

In step (k), a carbonyl compound of formula VIII, which is a known compound or can be prepared according to known procedures, is reacted with a (carboxyalkylidene)triarylphosphorane in an appropriate solvent, for example dimethylformamide or dimethylsulfoxide, at a temperature of from -20° C to 80° C.

## Reaction Scheme VI

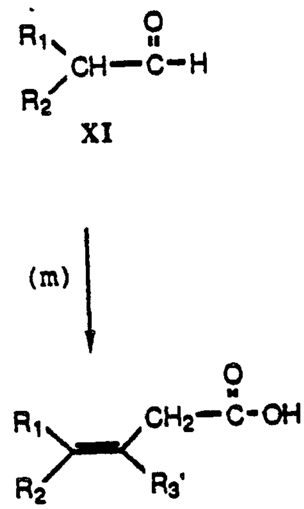


XI

IIb

wherein $R_1$ and $R_2$ are as previously described and $R_3'$ is hydrogen.

In Reaction Scheme VI, step (m), a carboxaldehyde of formula XI, which is a known compound or can be prepared according to known procedures, is reacted with malonic acid in an aprotic polar basic solvent, for example, pyridine, optionally in the presence of a catalytic amount of a secondary amine, for example, pyrrolidine or morpholine, at a temperature of from 50 °C to 110 °C.

## Reaction Scheme VII

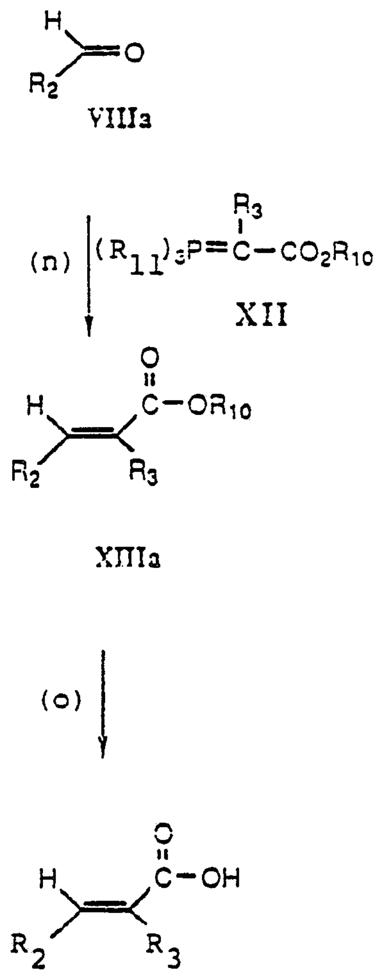


VIIIa

XII

XIIIa

IIc

wherein $R_2$, $R_3$, $R_{10}$ and $R_{11}$ are as previously described.

In Reaction Scheme VII, step (n), a carboxaldehyde of formula VIIIa is reacted with a (carbalkoxyalkylidene)triarylphosphonate of formula XII, which is a known compound or can be prepared according to known procedures, in an appropriate solvent at a temperature of from -20° C to 50° C. Use of an aprotic solvent, preferably dichloromethane, carbon tetrachloride or dimethylformamide, leads to an isomer ratio of >10:1 in favor of the (E)-isomer around the newly formed double bond. When a protic solvent is used as the reaction solvent, preferably methanol or ethanol, a higher proportion of the (Z)-isomer is formed, The resulting corresponding compound of formula XIIIa may be separated, if necessary, into the (E) and (Z)-isomers utilizing conventional methods, for example, crystallization, chromatography or the like.

In step (o), the resulting carboxylic acid ester of formula XIIIa is reacted with an excess of alkali metal hydroxide in a solvent mixture, preferably methanol water or ethanol water, at a temperature of from room temperature to 85° C.

## Reaction Scheme VIII

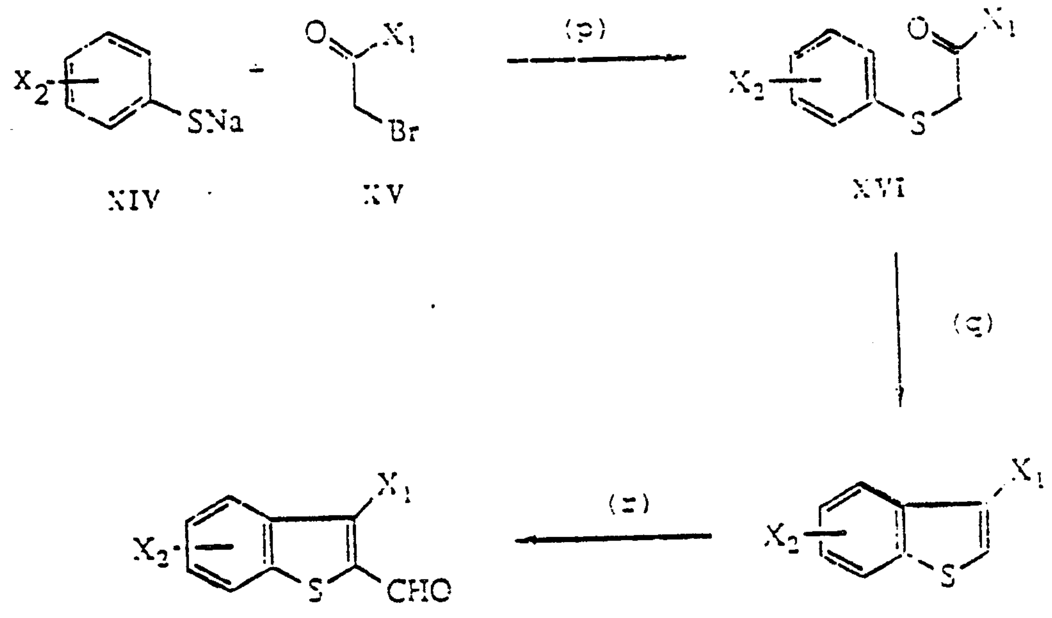


XIV XV XVI

XVII

VIIIb

wherein $X_1$ and $X_2$ are as previously described.

In Reaction Scheme VIII, step (p), a thiophenolate of formula XIV, which is a known compound or can be prepared according to known procedures, is reacted with a bromomethyl ketone of formula XV, which is a known compound or can be prepared according to known procedures, in an appropriate solvent, for example, water, at a temperature of from -20° C to 50° C.

In step (q), a resulting compound of formula XVI is treated with a strong mineral acid, preferably sulfuric acid, at a temperature of from -40° C to room temperature.

In step (r), a resulting benzo[b]thiophene of formula XVII is reacted with Vilsmeier reagent (prepared in the usual manner from phophorus oxychloride and dimethylformamide) in dimethylformamide as solvent at a temperature of from -20° C to 50° C. The resulting intermediate imine is converted to the carboxaldehyde of formula VIIIb by treatment of the reaction mixture with an excess of a solution of an alkali metal hydroxide, for example, sodium hydroxide, at a temperature of from 50° C to 120° C.

## Reaction Scheme IX

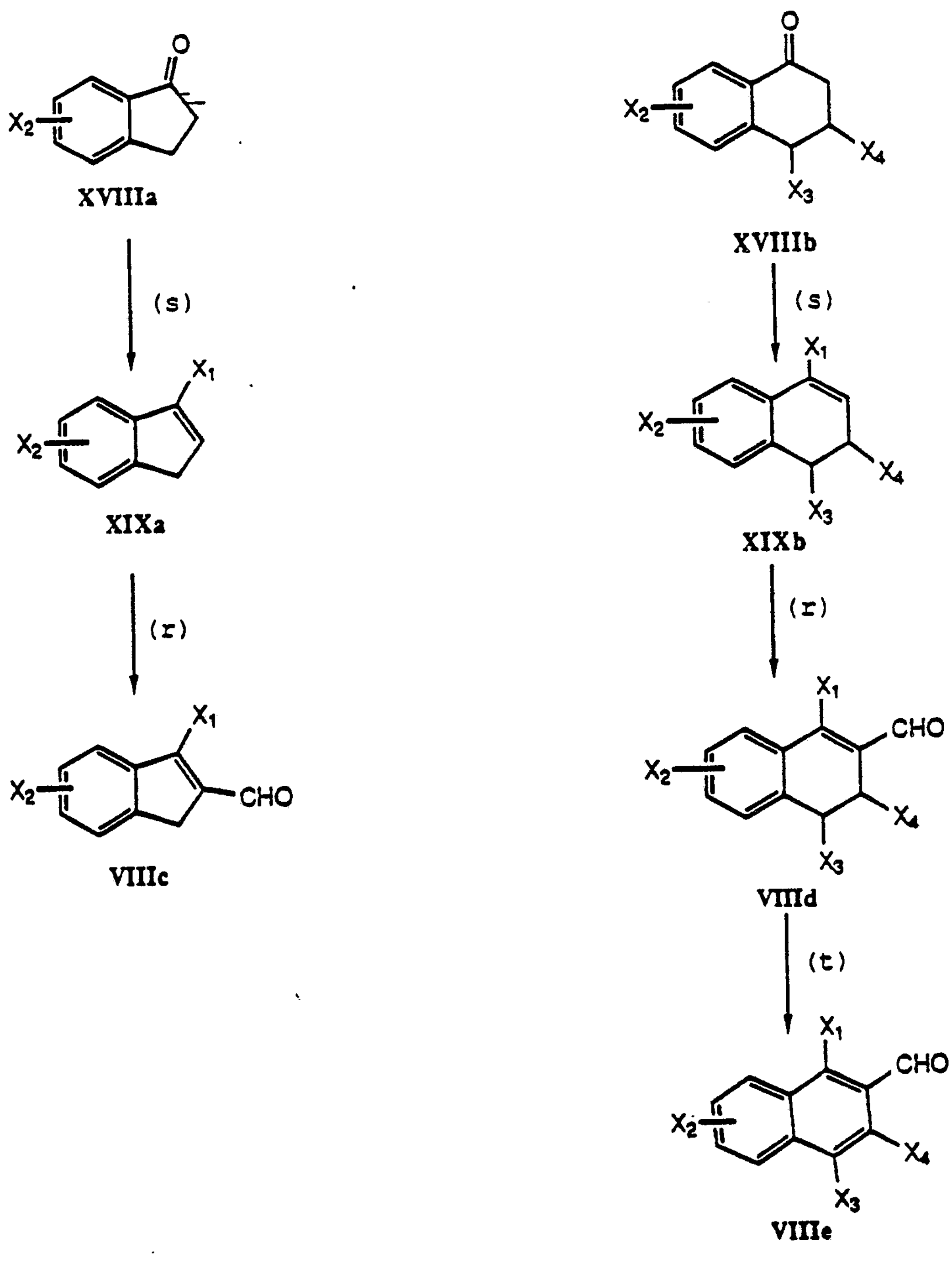


XVIIIa

XIXa

VIIIc

XVIIIb

XIXb

VIIId

VIIIe

wherein $X_1$, $X_2$, $X_3$ and $X_4$ are as previously described.

In Reaction Scheme IX, step (s), a carbonyl compound of formula XVIIIa or XVIIIb, which are known compounds or can be prepared according to known methods, is reacted with the appropriate alkyl or aryl magnesium halide or alkyl lithium or aryl lithium in an inert solvent such as diethyl ether or tetrahydrofuran at a temperature of from -80 ° C to 65 ° C.

Step (r), is the same as step (r) in Reaction Scheme VIII to give the carboxaldehyde of formula VIIIc or VIIId.

In step (t), a dihydronaphthalene of formula VIIId is reacted with a dehydrogenating agent, for example,

tetrachloro-1,4-benzoquinone or 2,3-dichloro-4,5-dicyano-1,4-benzoquinone, in an appropriate solvent, for example, benzene, toluene or t-butanol, at a temperature of from room temperature to 100° C.

Reaction Scheme X

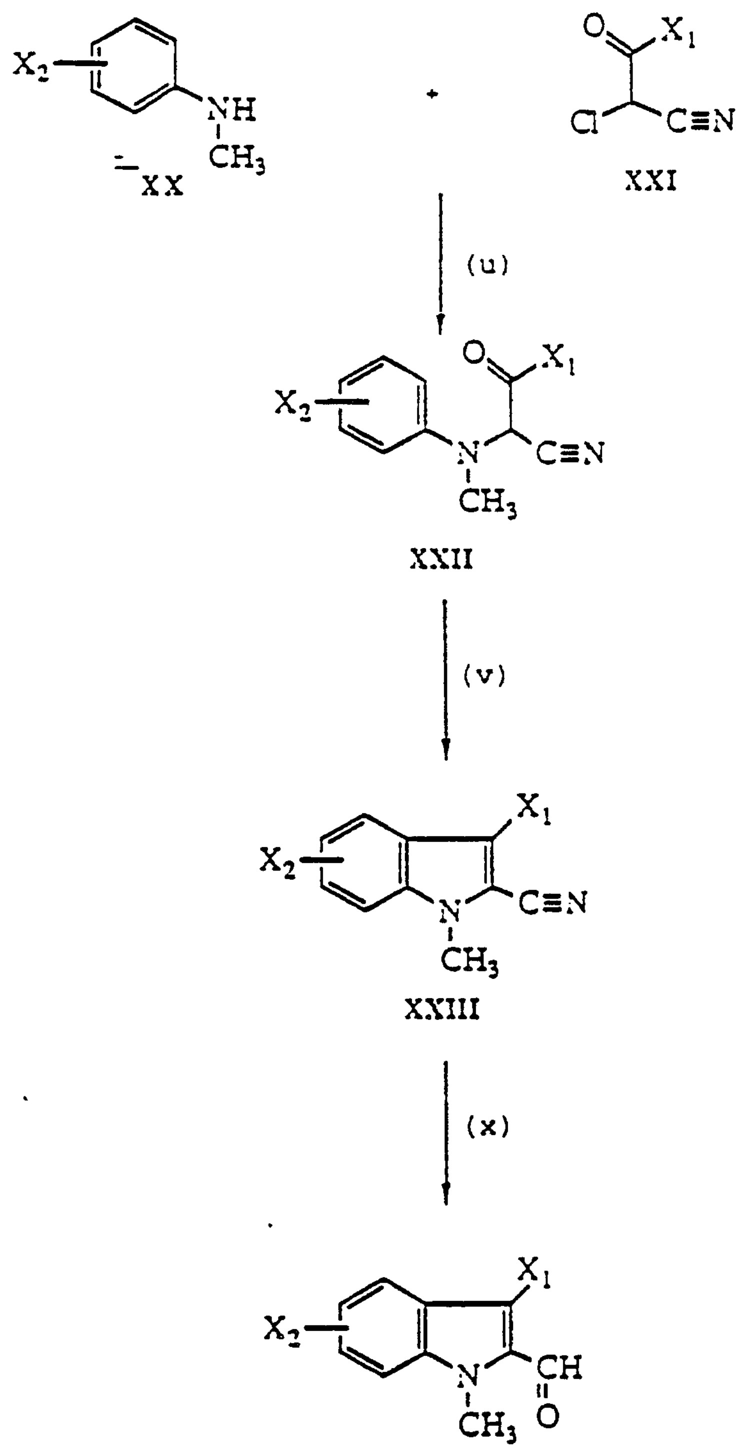

XX XXI XXII XXIII VIIIf

wherein $X_1$ and $X_2$ are as previously described.

In Reaction Scheme X, step (u), an α-chloro-β-ketonitrile of formula XXI, which is a known compound or

can be prepared according to known procedures, is reacted with a N-methylaniline of formula XX, which is a known compound or can be prepared according to known procedures, in an suitable solvent, preferably methanol or ethanol, at a temperature of from 50° C to the reflux temperature of the solvent.

In step (v), a compound of formula XXII is treated with a strong acid, preferably trifluoroacetic acid, at a temperature of from 0° C to 40° C to furnish the corresponding compound of formula XXIII.

In step (x), a nitrile of formula XXIII is reacted with a reducing agent, preferably diisobutylaluminum hydride in an inert solvent, for example, benzene or toluene, at a temperature of from -40° C to room temperature. The intermediate imine is treated with a dilute solution of a mineral acid, preferably sulfuric acid, to furnish the corresponding carboxaldehydes of formula VIIIf.

## Reaction Scheme XI

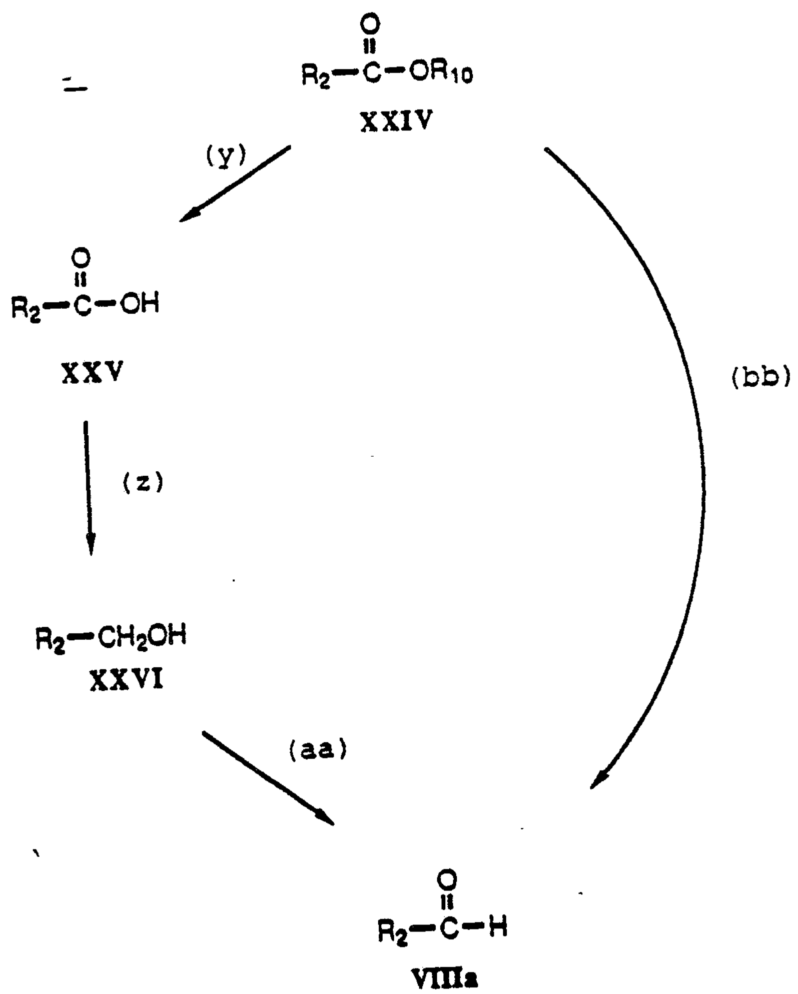

XXIV
XXV
XXVI
VIIIa

wherein $R_2$ and $R_{10}$ are as previously described.

In Reaction Scheme XI, step (y), a carboxylic acid ester of formula XXIV, which is a known compound or can be prepared according to known procedures, is reacted with an excess of alkali metal hydroxide in an solvent mixture, preferably methanol water or ethanol water, at a temperature of from room temperature to 85° C.

In step (z), a carboxylic acid of formula XXV is treated with a reducing agent, for example, borane in tetrahydrofuran, at a temperature of from -10° C to room temperature to furnish the corresponding alcohol of formula XXVI.

In step (aa), an alcohol of formula XXVI is oxidized to a carboxaldehyde of formula VIIIa, A preferred

method for this transformation is that described by K. Omura and D. Swern in Tetrahedron 1978, 34, (1651), which involves the reaction of the alcohol with a slight excess of the reagent, prepared from oxalyl chloride and dimethylsulfoxide, in an inert solvent, preferably dichloromethane at a temperature of from -50°C to -80°C. After the reaction is complete, the mixture is treated with excess triethylamine while the reaction temperature is maintained at from -50°C to -80°C. Then, after 30 minutes, the reaction is allowed to equilibrate to room temperature and the resulting corresponding carboxaldehyde of formula VIIIa is isolated.

In step (bb), the ester of formula XXIV is reacted with a reducing agent, preferably sodium bis(2-methoxyethoxy)aluminum hydride which has been modified with N-methylpiperazine, in an inert solvent, for example, toluene at a temperature of from -20°C to room temperature to produce a carboxaldehyde of formula VIIIa.

## Reaction Scheme XII

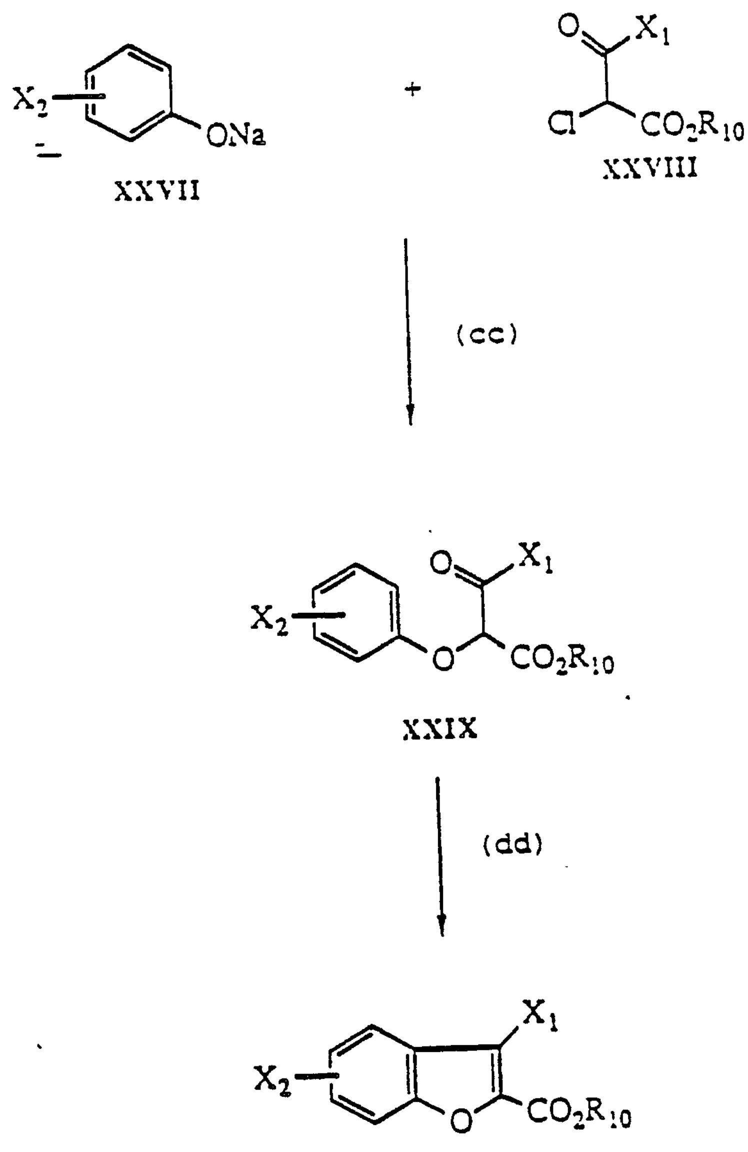


XXVII
XXVIII
XXIX
XXIVa

wherein $R_{10}$, $X_1$ and $X_2$ are as previously described.

In Reaction Scheme XII, step (cc), a sodium phenolate of formula XXVII, which is a known compound or

can be prepared according to known procedures, is reacted with an α-chloro-β-keto ester of formula XXVIII, which is a known compound or can be prepared according to known procedures, in an inert solvent, for example, benzene or toluene, at a temperature of from 20 °C to 50 °C.

In step (dd), a compound of formula XXIX is cyclized by treatment with a strong mineral acid, preferably sulfuric acid, at a temperature of from -40 °C to room temperature to furnish the corresponding benzofurancarboxylic acid ester of formula XXIVa.

Reaktion Scheme XIII

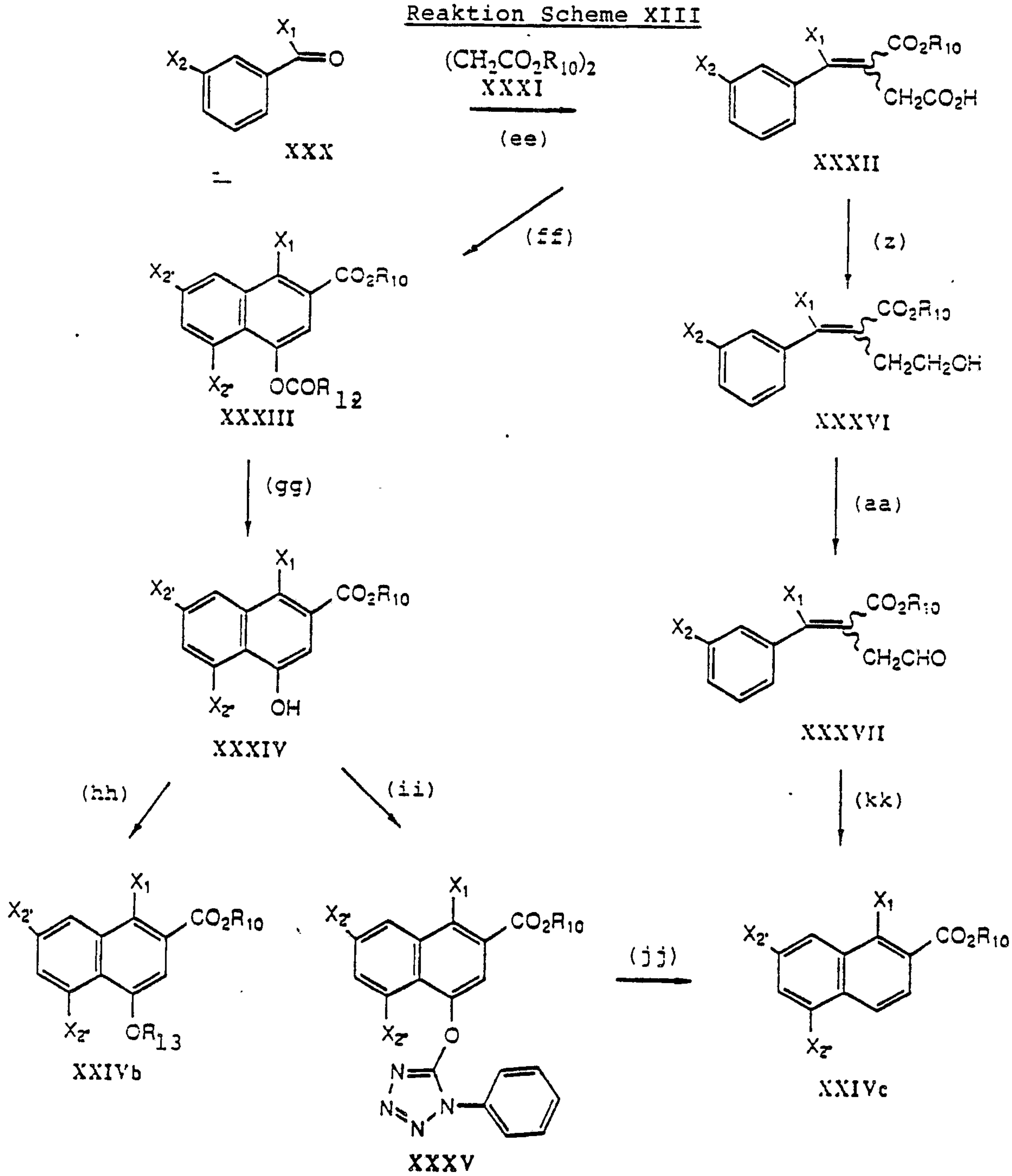


wherein $X_1$ and $X_2$ and $R_{10}$ are as previously described, $R_{12}$ is hydrogen, lower alkyl or trihalomethyl, $R_{13}$ is lower alkoxy and $X_2'$ and $X_2''$ are as previously described for $X_2$ with the proviso that at least one of

$X_2'$ or $X_2''$ is hydrogen.

In Reaction Scheme XIII, step (ee), a carbonyl compound of formula XXX, which is a known compound or can be prepared according to known procedures, is condensed with a succinic acid diester of formula XXXI, which is a known compound or can be prepared according to known procedures, in a protic solvent, preferably ethanol or t-butanol, in the presence of an alkali metal alkoxide, for example, potassium t-butoxide, at a temperature of from 50° C to the reflux temperature of the solvent.

In step (ff), the mixture of acids of formula XXXII is cyclized by treatment with an alkali metal carboxylate, for example, sodium acetate, in a carboxylic acid anhydride, preferably acetic anhydride, at a temperature of from 50° C to the reflux temperature of the mixture. The resulting isomeric mixture of naphthalenecarboxylic acid esters of formula XXXIII may be separated utilizing conventional methods, for example, chromatography, crystallization or the like.

In step (gg), the carbalkoxy function present in the naphthalenecarboxylic acid ester of formula XXXIII may be selectively hydrolyzed under acidic conditions, preferably, by using a dilute solution of hydrogen chloride in anhydrous ethanol or methanol, at a temperature of from room temperature to the reflux temperature of the mixture to furnish the corresponding 4-hydroxynaphthalenecarboxylic acid ester of formula XXXIV.

In step (hh), a 4-hydroxynaphthalenecarboxylic acid ester of formula XXXIV is reacted with an alkyl halide, for example, methyl iodide, in an inert solvent such as acetone or dimethylsulfoxide, in the presence of an alkali metal carbonate, preferably potassium carbonate, at a temperature of from room temperature to 60° C to furnish the corresponding compound of formula XXIVb.

In step (ii), a 4-hydroxynaphthalenecarboxylic acid ester of formula XXXIV is reacted with 5-chloro-1-phenyl-1H-tetrazole, in an inert solvent, preferably, dimethylsulfoxide, in the presence of an alkali metal alkoxide, for example, potassium t-butoxide, at a temperature of from 10° C to 40° C to furnish the corresponding compound of formula XXXV.

In step (jj), a compound of formula XXXV is hydrogenolyzed over a suitable catalyst, for example, palladium on carbon or platinum oxide, in a suitable solvent, for example, ethanol, or in a mixture of solvents, for example, tetrahydrofuran and ethanol, at a hydrogen pressure of one to three atmospheres until the theoretical amount of hydrogen is taken up to produce a corresponding compound of formula XXIVc.

Step (z) is the same as step (z) in Reaction Scheme XI and gives an alcohol of formula XXXVI.

Step (aa), is the same as step (aa) in Reaction Scheme XI and gives a carboxaldehyde of formula XXXVII.

In step (kk), a compound of formula XXXVII or a mixture of geometric isomers thereof is treated with a strong acid, preferably trfluoroacetic acid, at a temperature of from 0° C to 40° C to furnish the corresponding compound or the mixture of compounds of formula XXIVc.

Reaction Scheme XIV

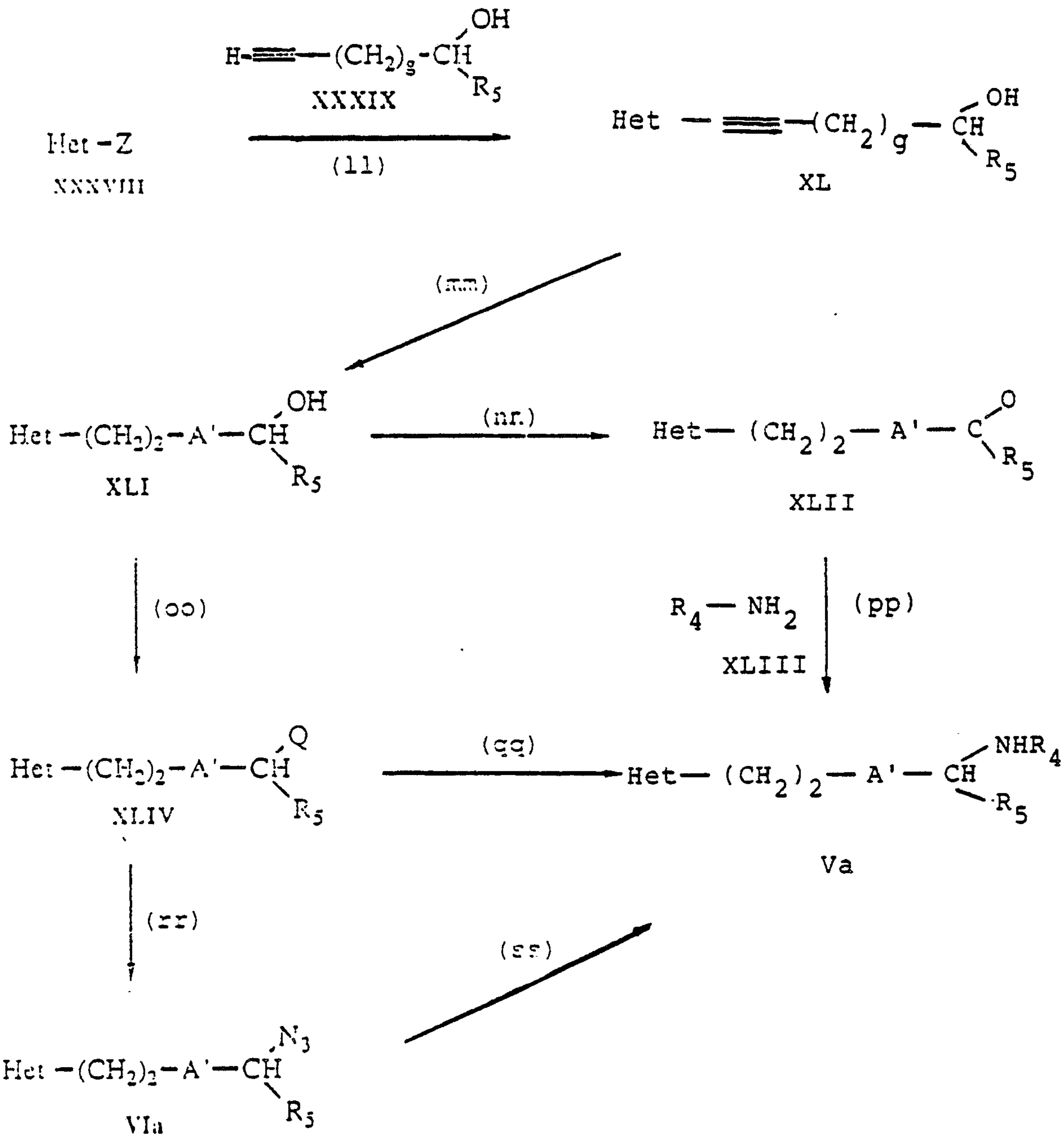


Het is as previously described, Z is iodine, bromine or a perfluoroalkylsulfonate and is attached at a position of the heteroaromatic ring such that it is active in transition metal catalyzed aryl/alkynyl coupling reactions. The compound of formula XIII becomes attached to Het at the position of the leaving Z group, G is an integer from 0 to 4, A′ is alkylene of 1 to 4 carbon atoms, Q is bromo, chloro or a lower alkyl- or arylsulfonyloxy radical and $R_4$ and $R_5$ are as previously described.

In Reaction Scheme XIV, step (11), a compound of formula XXXVIII, which is a known compound or can be prepared according to known methods, is reacted with an acetylene of formula XXXIX in the presence of an excess of a proton acceptor, for example, triethylamine, and a suitable palladium catalyst, for example, bis(triphenylphosphine)palladium dichloride, optionally in the presence of an inert solvent, for example, dichloromethane or dimethylformamide, at a temperature of from room temperature to 100° C, depending on the particular choice of Z-, solvent, and heteroaromatic ring, to give a compound of formula XL, which

may be used directly in the next step of the synthesis.

In step (mm), an acetylene of formula XL is dissolved in an inert solvent, for example, a lower alkanol, and hydrogenated over a suitable catalyst, for example, palladium on carbon, platinum oxide or the like, at a hydrogen pressure of from one to five atmospheres, preferably at room temperature, until reduction is complete. A compound of formula XLI in which $R_5$ is other than hydrogen may be resolved into its enantiomers using standard methodology, for example, conversion to an ester of a chiral acid and chromatographic separation followed by ester hydrolysis.

In step (oo) an alcohol of formula XLI is reacted with an alkyl or aryl sulfonyl halide, for example, methanesulfonyl chloride or p-toluenesulfonyl chloride in the presence of a proton acceptor, for example, pyridine or triethylamine to give a compound of formula XLIV wherein Q is an alkyl- or arylsulfonyloxy radical of the same absolute chirality as the starting alcohol XLI. Alternatively, a compound of formula XLI can be reacted with a reagent useful for the conversion of alcohols into halides, for example, thionyl chloride, in the presence of a proton acceptor, for example, pyridine, until conversion to a compound of formula XLIV, Q = Cl or Br, is complete. The resulting compound of formula XLIV generally is not isolated but utilized directly in the next step.

In step (rr), a compound of formula XLIV, is reacted with an alkali metal azide, for example, sodium azide, in the presence of a polar inert solvent, for example, dimethylformamide, N-methylpyrrolidinone, dimethylsulfoxide or the like at a temperature of from about room temperature to 100°C until azide formation is complete. This transformation generally proceeds with inversion of chirality at the carbon atom of the compound of formula XLIV bearing Q.

In step (qq), a compound of formula XLIV is reacted with an amine anion equivalent to give an intermediate which can be deprotected to give an amine of formula Va. For example, a compound of formula XLIV can be reacted with an alkali metal phthalimide, for example, potassium phthalimide, in a polar aprotic solvent, for example, dimethylformamide, dimethyl sulfoxide, N-methylpyrrolidinone or the like at a temperature of from about 60°C to 120°C until reaction is complete to give an intermediate of formula XLIV, Q = phthalimido, which can be converted to a compound of formula Va, by conventional means, for example, by treatment with hydrazine in a lower alkanol solvent or with methylamine in a polar aprotic solvent such as dimethylformamide. Alternatively, a compound of formula XLIV can be reacted with a perfluoroalkylsulfonamide derived from a primary amine, for example, N-alkyltrifluoromethanesulfonamide, in a polar aprotic solvent, for example, acetone, dimethylformamide, dimethylsulfoxide or the like, in the presence of a base, for example, an alkali metal hydroxide or, as appropriate, an alkali metal hydride, for example, sodium hydride, at a temperature of from room temperature to 100°C. When a compound of formula XLIV is chiral, this transformation will generally proceed with inversion of configuration at the carbon atom bearing Q in a compound of formula XLIV.

In step (nn), an alcohol of formula XLI is oxidized to a carbonyl derivative of formula XLII. Reagents which are useful for this transformation include chromium based oxidizing reagents, for example, pyridinium chlorochromate. A preferable procedure is described in K. Omura and D.Swern, Tetrahedron 1978, <u>34</u>, 1651, which involves dissolution of a slight excess of an acid halide, for example, oxalyl chloride in an inert halogenated hydrocarbon solvent, for example, dichloromethane, cooling to a reaction temperature of from -50°C to -80°C, addition of excess dimethylsulfoxide, stirring for 0.25 to 0.5 hours and addition of one equivalent of an alcohol of formula XLI. After an additional 0.25 to 0.5 hours, addition of excess triethylamine while maintaining the reaction temperature at from -50°C to -80°C, and allowing the reaction mixture to warm for 0.5 to 1 hour before quenching with water and excess inorganic base to produce a carbonyl derivative of formula XLII.

In step (pp), a carbonyl derivative of formula XLII is reacted with an amine of formula XLIII, which is a known compound or can be prepared according to known procedures, to form a Schiff's base which is reduced in the presence of an appropriate reducing agent to produce a corresponding amine of formula Va in either a one step or two step process. For example, a compound of formula XLII is treated with a large excess of an amine of formula XLIII and an equivalent amount of a weak organic acid, for example, acetic acid, in the presence of a reducing agent such as sodium cyanoborohydride in a suitable solvent, preferably a lower alkanol, for example, methanol, at room temperature until the starting material is consumed,

Alternatively, an amine of formula XLIII and a carbonyl derivative of formula XLII are heated together in an aromatic solvent in an apparatus fitted with a water separator until water formation is complete. The resulting Schiff's base can be hydrogenated over a suitable catalyst, preferably Raney nickel, at a hydrogen pressure of from one to five atmospheres to give a compound of formula Va. When $R_5$ is not hydrogen and $R_4$ is chiral, the resulting amine Va may be enriched in one diastereomer over the other. For example, when $R_4$ is a chiral benzyl group, for example, R-α-methylbenzyl, and $R_5$ is lower alkyl, for example, methyl, the compound of formula Va may be diastereomerically enriched, and the chiral benzyl moiety may be

removed, for example by hydrogenation over palladium on carbon to give an enantiomerically enriched amine of formula Va, $R_4$ = hydrogen and $R_5$ = lower alkyl.

In step $(ss)_L$ an azide of formula VIa is dissolved in a solvent, preferably, a lower alkanol, and hydrogenated at a hydrogen pressure of from one to five atmospheres over a nobel metal catalyst, for example, palladium on carbon or platinum oxide. This transformation to a compound of formula Va proceeds without alteration of the chirality of the carbon atom bearing the azido group in a compound of formula VIa.

## Reaction Scheme XV

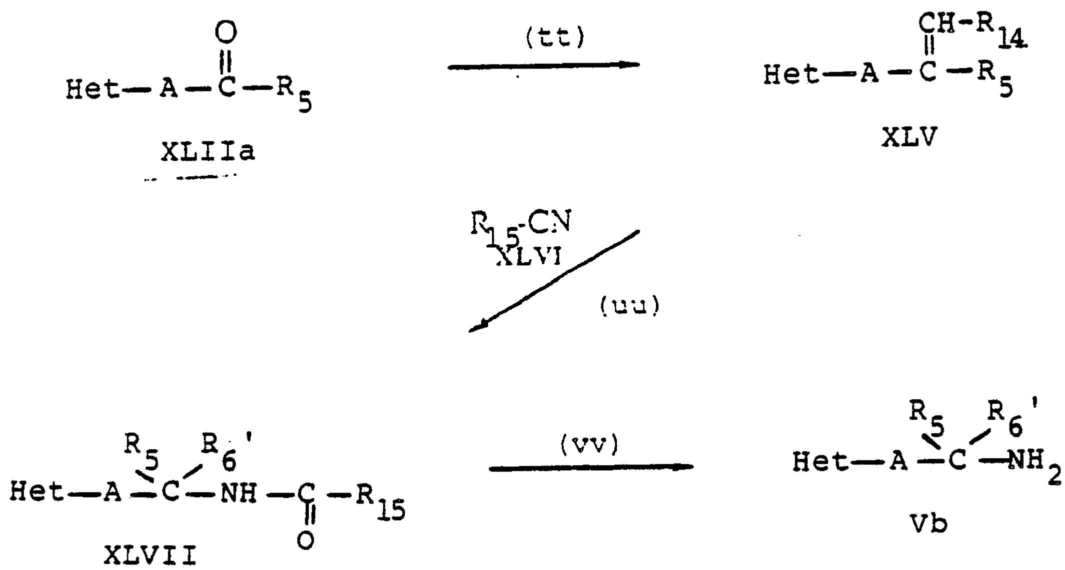


XLIIa XLV XLVII Vb

wherein Het, A and $R_5$ are as previously described. $R_6'$ is lower alkyl, $R_{14}$ is hydrogen or lower alkyl, and $R_{15}$ is lower alkyl, aryl or aryl-lower alkyl.

In Reaction Scheme XV, step (tt), a carbonyl derivative of formula XLIIa, which is a known compound or can be prepared according to known methods, is treated with an alkylidene triarylphosphorane in an inert solvent, preferably tetrahydrofuran, dimethylsulfoxide or diethyl ether, at a temperature of from -80° C to room temperature.

In step (uu), a nitrile of formula XLVI is reacted with a compound of formula XLV in the presence of a strong mineral acid, preferably sulfuric acid and a small amount of water.

In step (vv), a compound of formula XLVII is hydrolyzed to a corresponding amine of formula Vb, The hydrolysis is advantageously carried out, when $R_{15}$ is 2-nitrobenzyl, by catalytic reduction of the nitro benzyl group, for example, over palladium on carbon at one atmosphere hydrogen pressure, and heating of the residue in the absence of solvent or in the presence of a solvent, for example, acetic acid.

## Reaction Scheme XVI

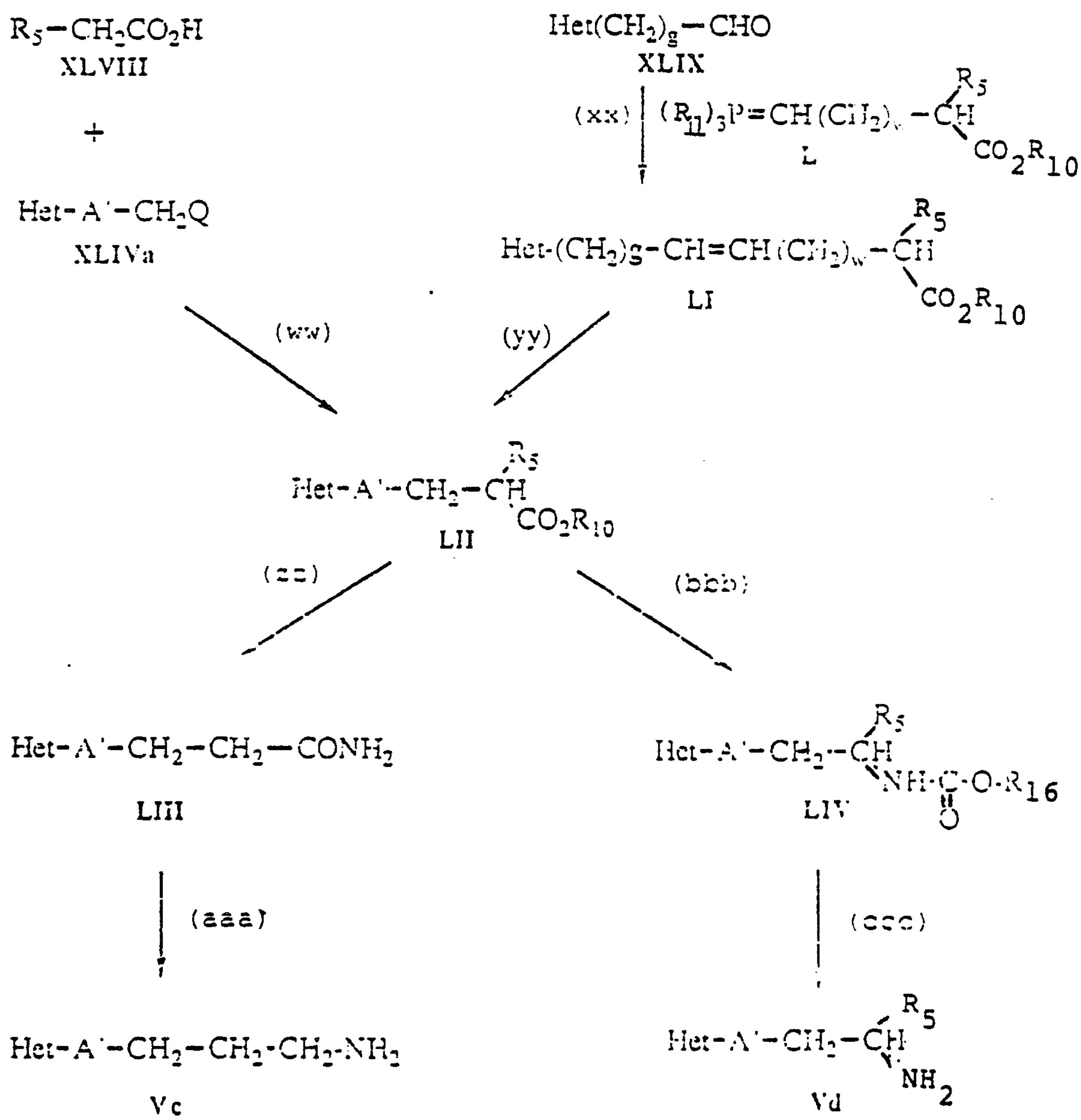


wherein Het, $R_5$, $R_{11}$, A′, Q and g are as previously described, and $R_{10}$ is hydrogen or lower alkyl, $R_{16}$ is lower alkyl or aryl, and w is an integer from 0 to 3.

In Reaction Scheme XVI, step (ww), the dilithium salt derived from a compound of formula XLVIII, which is a known compound or can be prepared according to known methods, for example, by treatment with lithium diisopropylamide, is reacted with a compound of formula XLIVa, which is a known compound or can be prepared according to known methods, in a suitable inert solvent, for example, tetrahydrofuran, to give the corresponding compound of formula LII, $R_{10}$ = hydrogen.

A compound of formula LII, in which $R_5$ is other than hydrogen and $R_{10}$ is hydrogen, may be resolved into its enantiomers by conversion to a salt with a chiral, enantiomerically pure amine, for example, cinchonine, brucine, α-methylbenzylamine or the like. The pure diastereomeric salts are obtained by fractional crystallization from an appropriate solvent, for example, a lower alkanol. A chiral, enantiomerically

pure acid of formula LII, $R_{10}$ = hydrogen, may be recovered from its salt by conventional means, for example extraction from an aqueous, acidic solution.

In step (xx), a heteroaromatic carboxaldehyde of formula XLIX, which is a known compound or can be prepared according to known methods, is reacted with a (carboxyakylidene)triarylphosphorane of formula L, which is a known compound or can be prepared according to known methods, in a suitable solvent, for example, tetrahydrofuran, dichloromethane, methanol or dimethylsulfoxide, to give a compound of formula LI.

In step (yy), a compound of formula LI is hydrogenated over a suitable catalyst, for example, palladium on carbon or platinum oxide, in a suitable solvent, for example, a lower alkanol, at a hydrogen pressure of from one to five atmospheres until the theoretical amount of hydrogen is taken up to give a corresponding compound of formula LII.

In step (zz), a compound of formula LII is converted into an amide of formula LIII using conventional techniques for the conversion of carboxylic acids and esters into the corresponding primary amides. For example, a compound of formula LII, $R_{10}$ = hydrogen, may be converted to the corresponding acid chloride by treatment with thionyl chloride and then reacted with an excess of ammonia to give a corresponding compound of formula LIII. Alternatively, a compound of formula LII, $R_{10}$ = lower alkyl, may be converted into a corresponding compound of formula LIII by treatment with excess ammonia, optionally in the presence of co-solvent, for example, a lower alkanol at a temperature of from -33°C to room temperature. The reaction may be run in a pressure vessel, when appropriate.

In step (aaa), a compound of formula LIII is treated with a reducing agent, for example, borane in tetrahydrofuran at a temperature of from room temperature to the reflux temperature of the solvent for 4 to 24 hours or until reduction is complete to give a corresponding compound of formula Vc.

In step (bbb), an acid of formula LII, $R_{10}$ = hydrogen, which may be obtained from the corresponding ester by hydrolysis, is subjected to conditions leading to a Curtius rearrangement in the presence of a lower alkanol. In a preferred procedure, an acid of formula LII ($R_{10}$ = hydrogen), is treated with one equivalent of diphenylphosphoryl azide in the presence of a proton acceptor, for example, triethylamine or the like, and an excess of a lower alkanol or a phenol to give a corresponding compound of formula LIV.

In step (ccc), a compound of formula LIV is treated with an excess of a mineral acid in water and optionally a co-solvent, for example, a lower alkanol, at a temperature of from room temperature to 100°C or with a strong base in water, optionally in the presence of a co-solvent, for example, a lower alkanol at a temperature of between 60°C and 100°C to form a compound of formula Vd.

## Reaction Scheme XVII

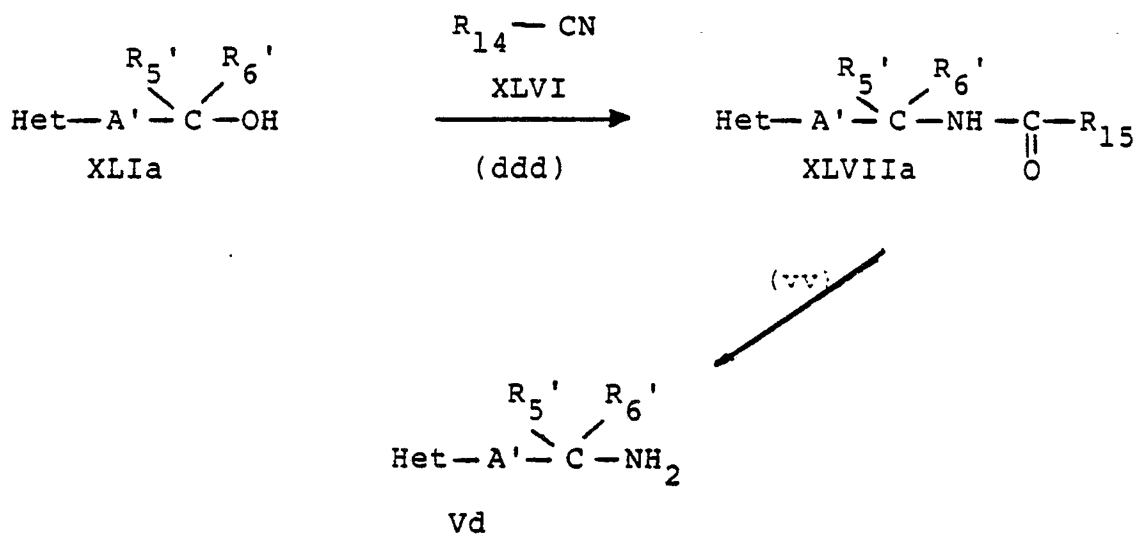


XLIa

XLVIIa

Vd

wherein Het, A′, $R_6$, and $R_{15}$, are as previously described, and $R_5'$ is lower alkyl.

In Reaction Scheme XVII, step (ddd), an alcohol of formula XLIa is reacted with a nitrile of formula XLVI in the presence of a mineral acid, for example, sulfuric acid, and water at a temperature of from -20°C to room temperature to give a compound of formula XLVIIa.

Step (vv) is the same as step (tt) in Reaction Scheme XV.

## Reaction Scheme XVIII

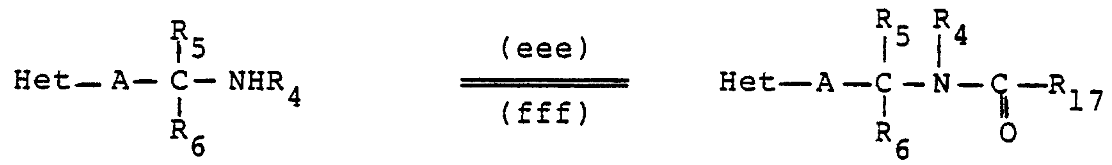


V

LV

wherein Het, A, $R_4$, $R_5$ and $R_6$ are as previously described, $R_{17}$ is a chiral moiety, for example, an open chain lower alkyl group or a lower alkyl group substituted with one or two groups selected from hydroxy, lower alkoxy, lower alkylcarbonyloxy, trifluoromethyl or aryl.

In Reaction Scheme XVIII, step (eee), a compound of formula V which is chiral may be resolved into its enantiomers by conversion to amides of chiral, enantiomerically pure acids using common techniques of peptide coupling. For example, a chiral amine of formula V may be coupled with a chiral, enantiomerically pure acid, for example, (R)-mandelic acid, in the presence of a suitable coupling reagent, for example, dicyclohexylcarbodiimide optionally in the presence of a promoter, for example, 1-hydroxybenzotriazole in a polar, aprotic solvent, for example, dimethylformamide to give a corresponding amide of formula LVI. Amides of formula LVI may be separated into pure diastereomers by fractional crystallization, chromatography or the like.

In step (fff), an enantiomerically pure compound of formula V may be recovered by hydrolysis of a diastereomerically pure amide of formula LV, for example with an aqueous mineral acid at a temperature of from 60°C to 120°C.

The compounds of formula I can form acid addition salts with inorganic or organic acids. Thus, they form pharmaceutically acceptable acid addition salts with both pharmaceutically acceptable organic and inorganic acids, for example, with hydrohalic acids, such as, hydrochloric acid, hydrobromic acid, hydroiodic acid, other mineral acid salts, such as, sulfuric acid, nitric acid, phosphoric acid, perchloric acid or the like, alkyl and mono-aryl sulfonic acids such as, ethanesulfonic acid, p-toluenesulfonic acid, benzenesulfonic acid, or the like, other organic acids such as tartaric acid, maleic acid, citric acid, salicylic acid, ascorbic acid and the like. Non-pharmaceutically acceptable acid addition salts of compounds of formula I can be converted into pharmaceutically acceptable acid addition salts via conventional metathetic reactions whereby the non-pharmaceutically acceptable anion is replaced by a pharmaceutically acceptable anion; or alternatively, by neutralizing the non-pharmaceutically acceptable acid addition salt and then reacting the so-obtained free base with a reagent yielding a pharmaceutically acceptable acid addition salt.

The compounds of formula I exhibit activity as platelet activating factor (PAF) antagonists and are, therefore, useful in disease states characterized by excess platelet activating factor or for the prevention and treatment of cardiovascular diseases, pulmonary diseases, immunological disorders, inflammatory diseases, dermatological disorders, shocks or transplant rejections.

The useful activity of the compounds of formula I can be demonstrated by the following procedures:

Binding Assay

a) Assay

The binding assay was done in 400 µl polyethylene microcentrifuge tubes (Beckman) containing 50 µl of an oil mixture of 2 parts of Siliconol AR200 (Serva) and 1 part of Silicone Fluid (Arthur H. Thomas). Buffer, standards, or analogs (150 µl total volume) were added to the tubes. Radiolabelled $^{3}H$-PAF (50 µl) was then added to the tubes. The reaction was started by the addition of 50 µl of dog platelets ($2 \times 10^{7}$ platelets). The tubes were capped, inverted several times to mix, and incubated for 10 minutes at room temperature. The platelets were separated from the incubation mixture by centrifuging 1 minute in a Beckman Microfuge B centrifuge. The tip of the tubes were cut off, and the platelets were washed out of the tips with 200 µl of 50% methanol (Burdick and Jackson). Aquasol (NEN, 10 ml) was added and the radioactivity in the samples was determined using an LS 8100 Beckman liquid scintillation counter linked to a Techtran tape recorder. Data was processed through an in-house computer system. Alternatively, radioactivity was determined using a Searle Mark III liquid scintillation counter linked to a Iso-Data microprocessor. The results are set forth in Table I.

b) Preparation of Platelets

Blood was collected from anesthesized or unanesthesized dogs into 50 ml plastic centrifuge tubes containing 3.8% sodium citrate as the anticoagulant (1 volume of citrate/9 volumes of blood). The red cells were removed by centrifugation for 15 minutes at 600 rpm (100-125 g) at room temperature. An aliquot of the supernatant platelet rich plasma (PRP) was saved for cell counting and the remainder was acidified to pH 6.5 with 0.15 M citric acid. The platelet pellet was obtained after a 10 minute centrifugation at 2000 rpm (1000 g) at room temperature. Washed platelets were prepared by resuspending the platelet pellet once with PBS containing 1 mM of EDTA, centrifuging as noted, and then resuspending the platelets in 0.1% of BSA/PBS. An aliquot of the washed platelets was counted. Platelets used for binding assays were diluted to $2 \times 10^{7}$ platelets/assay tube ($4 \times 10^{8}$ platelets/ml). Platelet counting was done using a Royco Cell-Crit 921.

PAF Induced Bronchoconstriction Assay

Male guinea pigs (Hartley Strain, 400-500 g) were anesthetized with urethane (2 g/kg, i.p.). Each animals' trachea was cannulated and the guinea pigs were respirated using a Harvard small animal rodent respirator (3.0 cc stroke volume, 40 breaths per min.). Tracheal pressure was recorded from a cannula inserted in the trachea and connected to a Statham pressure Transducer.

The jugular vein was cannulated for administering compounds. Spontaneous breathing was arrested with succinylcholine (1.2 mg/kg, i.v.) administered 2 minutes prior to intravenous injection of platelet activating factor (PAF). Since propranolol has been shown to enhance bronchoconstrictor responses, all animals were pretreated five minutes prior to challenge with propranolol (0.1 mg/kg, i.v.).

For the intravenous testing, the guinea pig is given a 5-minute pretreatment with propranolol at a dose of 0.1 mg/kg intravenously. The test compound is administered with a 1 minute pretreatment prior to intravenous challenge with PAF. The animal is then challenged with a 1.0µg/kg intravenous dose of PAF and the change in tracheal pressure is measured.

For the oral testing, the procedure includes a 2-hour pretreatment period with the test compound administered through an oral gavage tube. Propranolol or succinylcholine and PAF are administered intravenously, and the change in tracheal pressure is measured.

The change in tracheal pressure is determined by subtracting the steady state baseline achieved after administration of succinylcholine from the peak bronchoconstriction seen after challenge with PAF. The mean is calculated for each test compound and compared to the mean of the control animals to give the percent inhibition of bronchoconstriction. The standard error is calculated as the standard error of the mean.

The compounds of formula I also have Thromboxane Synthase ($TXA_2$Syn.) Inhibitory Activity, which can be demonstrated as follows.

TXA2 Synthesis Inhibition

Thromboxane Synthase ($TXA_2$ syn.) inhibitory activity is measured by following the conversion of $^{14}C$-thromboxane $A_2$ ($TXA_2$) using microsomal fractions from human platelets as enzyme source. In the aqueous incubation medium, the $TXA_2$ decomposes rapidly into $TXB_2$. The amount of $TXA_2$ syn, is adjusted so that under the conditions of the assay approximately 80-90% of the substrate, $PGH_2$, is converted to product in control tubes. To prepare $^{14}C$-$PGH_2$, $^{14}C$-AA(50-60mCi/mmole) is incubated with sheep seminal vesicular gland microsomes for 1.5 min, at 37° and then the $^{14}C$-$PGH_2$ is extracted with diethylether, purified on columns of Sephadex LH-20 or silicic acid, and stored in acetone at -70° C. Incubations are done as follows, Sufficient $^{14}C$-$PGH_2$ to yield a final substrate concentration of 10µM (about 30,000 cpm) is added to the incubation tubes and then the acetone is removed under nitrogen. The tubes are placed in an ice bath and then 215 µl of ice cold phosphate buffered saline, 10 µl of ethanol (control) or of test drug in ethanol and 25 µl of the microsomal suspension are added with mixing in that order as rapidly as possible. The tubes are incubated at 22° for 2 minutes, the reaction is stopped and then the radioactive products and the unconverted $PGH_2$ are extracted and analyzed by thin layer chromatography. The amount of $^{14}C$-$PGH_2$ converted to products is analyzed by thin layer chromatography. The amount of $^{14}C$-$PGH_2$ converted to products was used as a measure of $TXA_2$ synthase activity. Inhibitors were tested initially at a concentration of 100 µM. $IC_{50}$ values were calculated by linear regression analysis of successive 10 fold dilutions of the test compound concentration.

Test results obtained with compounds of formula I in the described tests are set forth in Table I which follows:

Table I

| Compound | Inhibition of PAF Binding $IC_{50}$ (nM) | Inhibition of PAF Induced Bronchoconstriction 1mg/Kg (iv) |
|---|---|---|
| [R-(E)]-3-(1-Butyl-6-methoxy-2-naphthalenyl)-N-[1-methyl-4-(3-pyridinyl)butyl]-2-propenamide | 7 | 95 ± 1% |
| [R-(E)]-3-(1-Butyl-3,4-dihydro-6-methoxy-2-naphthalenyl)-N-[1-methyl-4-(3-pyridinyl)butyl]-2-propenamide | 90 | 94 ± 2% |
| [R-(E)]-3-[3,4-Dihydro-6-methoxy-1-(4-methoxyphenyl)-2-naphthalenyl]-N-[1-methyl-4-(3-pyridinyl)butyl]-2-propenamide | 30 | 95 ± 2% |
| [R-(E)]-3-[6-Methoxy-1-(4-methoxyphenyl)-2-naphthalenyl]-N-[1-methyl-4-(3-pyridinyl)butyl]-2-propenamide | 55 | 92 + 3% |
| [R-(E)]-3-(1-Butyl-5-methoxy-2-naphthalenyl)-N-[1-methyl-4-(3-pyridinyl)butyl]-2-propenamide | 6 | 50 + 12% |
| [R-(E)]-3-(1-Butyl-7-methoxy-2-naphthalenyl)-N-[1-methyl-4-(3-pyridinyl)butyl]-2-propenamide | 15 | -- |

Table I (con't)

| Compound | Inhibition of PAF Binding, $IC_{50}$ (nM) | Inhibition of PAF Induced Bronchoconstriction 1mg/Kg (iv) |
|---|---|---|
| [R-(E)]-3-(1-Butyl-4,7-dimethoxy-2-naphthalenyl)-N-[1-methyl-4-(3-pyridinyl)butyl]-2-propenamide | 3 | 80 + 12% |
| [R-(E)]-3-(1-Butyl-4-methoxy-2-naphthalenyl)-N-[1-methyl-4-(3-pyridinyl)butyl]-2-propenamide | 1 | 90 + 5% |
| (E)-3-[7-Methoxy-1-(3-methoxyphenyl) 2-naphthalenyl]-N-[4-(3-pyridinyl)butyl]-2-propenamide | 8 | 62 + 8% |
| [R-(E)]-3-(6-Methoxy-3-pentylinden-2-yl)-N-[1-methyl-4-(3-pyridinyl)butyl]-2-propenamide | 33 | 96 + 1% |
| [R-(E)]-3-(6-Methoxy-3-pentylbenzo-[b]thien-2-yl)-N-[1-methyl-4-(3-pyridinyl)butyl]-2-propenamide | 24 | 98 + 1% |
| [R-(E)]-3-(6-Methoxy-3-pentylbenzofuran-2-yl)-N-[1-methyl-4-(3-pyridinyl)butyl]-2-propenamide | 40 | 89 + 1% |

Table II

| Compound | Inhibition of $TXA_2$ Synthetase $IC_{50}$ ($\mu M$) |
|---|---|
| 3,3-Diphenyl-N-[4-(3-pyridinyl)butyl]-2-propenamide | 0.1 - 1 |
| 4,4-Diphenyl-N-[4-(3-pyridinyl)butyl]-3-butenamide | 1 |
| 5,5-Diphenyl-N-[4-(3-pyridinyl)butyl]-4-pentenamide | 0.1 - 1 |
| 5,5-bis(4-Methoxyphenyl)-N-[4-(3-pyridinyl)-butyl]-4-pentenamide | 1 - 10 |
| 5,5-bis(4-Fluorophenyl)-N-[4-(3-pyridinyl)-butyl]-4-pentenamide | 1 - 10 |
| 6,6-Diphenyl-N-[4-(3-pyridinyl)butyl]-5-hexenamide | 0.1 |
| 7,7-Diphenyl-N-[4-(3-pyridinyl)butyl]-6-heptenamide | 0.1-10 |
| 8,8-Diphenyl-N-[4-(3-pyridinyl)butyl]-7-octenamide | 0.1 - 1 |
| 9,9-Diphenyl-N-[4-(3-pyridinyl)butyl]-8-nonenamide | 0.01-0.1 |

A compound of formula I, an enantiomer thereof or a salt thereof or a composition containing a therapeutically effective amount of a compound of formula I, an enantiomer thereof or a salt thereof can be administered by methods well known in the art. Thus, a compound of formula I, an enantiomer thereof or a salt thereof can be administered either singly or with other pharmaceutical agents, for example, antihistamines, mediator release inhibitors, methyl xanthines, beta agonists or antiasthmatic steroids such as prednisone and prednisolone, orally, parenterally, rectally, or by inhalation, for example, in the form of an aerosol, micropulverized powder or nebulized solution. For oral administration they can be administered in the form of tablets, capsules, for example, in admixture with talc, starch, milk sugar or other inert ingredients, that is, pharmaceutically acceptable carriers, or in the form of aqueous solutions, suspensions, elixirs or aqueous alcoholic solutions, for example, in admixture with sugar or other sweetening agents, flavoring agents, colorants, thickeners and other conventional pharmaceutical excipients. For parenteral administration, they can be administered in solutions or suspension, for example, as an aqueous or peanut oil solution or suspension using excipients and carriers conventional for this mode of administration. For administration as aerosols, they can be dissolved in a suitable pharmaceutically acceptable solvent, for example, ethyl alcohol or combinations of miscible solvents, and mixed with a pharmaceutically acceptable propellant. Such aerosol compositions are packaged for use in a pressurized container fitted with an aerosol valve suitable for release of the pressurized composition. Preferably, the aerosol valve is a metered valve, that is one which on activation releases a predetermined effective dose of the aerosol composition.

In the practice of the invention, the dose of a compound of formula I or a salt thereof to be administered and the frequency of administration will be dependent on the potency and duration of activity of the particular compound of formula I or salt to be administered and on the route of administration, as well as the severity of the condition, age of the mammal to be treated and the like. Oral doses of a compound of formula I or a salt thereof contemplated for use in practicing the invention are in the range of from about 25 to about 1000 mg per day, preferably about 25 to about 250 mg either as a single dose or in divided doses.

The geometric isomers encompassed by formula I can be separated by conventional means, for example, chromatography, crystallization and the like. It is noted, however, that the separation in most instances is done at an earlier stage in the synthesis of the compounds of formula I.

Furthermore, since compounds of formula I of the invention, when $R_5$ and $R_6$ are different, possess an asymmetric carbon atom, they are ordinarily obtained as racemic mixtures. The resolution of such racemates into the optically active isomers can be carried out by known procedures, Some racemic mixtures can be precipitated as eutectics and can thereafter be separated. Chemical resolution is, however, preferred. By this method, diastereomers are formed from the racemic mixture of a compound of formula I, with an optically active resolving agent, for example, an optically active acid, such as dibenzoyltartaric acid. The formed diastereomers are separated by selective crystallization and converted to the corresponding optical isomer. Thus, the invention covers the racemates of the compounds of formula I as well as their optically active isomers (enantiomers).

The following examples further describe the present invention. All temperatures given are in degrees centigrade.

Example 1

a) In an inert atmosphere, sodium hydride (56% dispersion in oil; 4,3g) was triturated with dry pentane and then was dispersed in dimethylsulfoxide (100 ml). After stirring at 70 °C for 1 hour, the solution was cooled to 0 °C, then trimethyl phosphonoacetate (38,8g) in dry tetrahydrofuran (100 ml) was added over 15 min. The reaction was stirred at 0 °C for 30 min, then benzophenone (15g) was added and the mixture was stirred at room temperature for 16 hours and then at 50 °C for 4 hours to complete the reaction. After dilution with water, the mixture was extracted with ethyl acetate. The organic layer was washed with water, then was dried ($MgSO_4$) and evaporated. The residual oil was dissolved in methanol (150 ml) containing 10N sodium hydroxide (30 ml) and after the mixture was stirred at reflux for 16 hours, it was cooled, acidified with excess 1N hydrochloric acid and extracted with dichloromethane (3 x 150 ml). The combined extracts were washed with water, dried ($Na_2O_4$) and evaporated. The crude product was passed through a short column of silica gel (200g) using dichloromethaneethyl acetate (1:1) as the eluant. The resulting material was crystallized from hexane to furnish 13.1g of 3,3-diphenyl-2-propenoic acid, mp 162-164 °C.

b) To a stirred solution of 3,3-diphenyl-2-propenoic acid (6.23g) and triethylamine (4.25 ml) in dry tetrahydrofuran (40 ml) at 0 °C, was added a solution of ethyl chloroformate (2.74 ml) in tetrahydrofuran (20 ml) dropwise such that the reaction temperature was maintained at 0 °C. After the addition was completed, the reaction was stirred at 0 °C for 15 min, then a solution of 3-pyridinebutanamine (4,38g), in tetrahydrofuran (20 ml) was added dropwise while keeping the reaction at -5 °C. The mixture was stirred at 0 °C for 1 hour and then at room temperature for 2 hours before the precipitated triethylamine hydrochloride was removed by filtration. The evaporated filtrate was partitioned between 1N hydrochloric acid (80 ml) and ether (80 ml). The aqueous layer was basified with 4N sodium hydroxide (30 ml) and extracted with ether (4 x 100 ml). Evaporation of the dried ($K_2CO_3$) organic extracts afforded 11.1g of amide which was purified by HPLC (ethyl acetate) to yield 7.42g of 3,3-diphenyl-N-[4-(3-pyridinyl)- butyl]-2-propenamide. A portion was crystallized from ether to give an analytical sample, mp 78-80 °C. Anal. Calculated for $C_{24}H_{24}N_2O$: C 80.87, H 6.79, N 7.86; Found: C 81.11, H 6.86, N 7.89.

c) The amine (6.8g) in 2-propanol (20 ml) was treated with 2.1N hydrobromic acid in 2-propanol (9 ml) and the solution was diluted with ethyl acetate to give 7.9g of 3,3-diphenyl-N-[4-(3-pyridinyl)butyl]-2-propenamide HBr salt (1:1), mp 173-174.5 °C. Anal. Calculated for $C_{24}H_{24}N_2O{\bullet}HBr$: C 65.91, H 5.76, N 6.40, Br 18.27; Found: C 65.93, H 5.98, N 6.53, Br 18.14.

Example 2

As in Example 1, the mixed anhydride formed from 3,3-diphenyl-2-propenoic acid (4.49g), triethylamine (3 ml; 0.0215 mol) and ethyl chloroformate (1.97 ml) in tetrahydrofuran (44 ml) was reacted with 3-pyridinehexanamine (3.74g). The material obtained after the usual work up was purified by HPLC (etherhexane; 7:3) and then crystallized from ether to give 4.45g of 3,3-diphenyl-N-[6-(3-pyridinyl)hexyl]- 2-propenamide, mp 73-74 °C. Anal. Calculated for $C_{26}H_{28}N_2O$: C 81.21, H 7.34, N 7.29, Found: C 80.98, H 7.42, N 7.07.

Example 3

a) A mixture of diphenylacetaldehyde (19.6g), malonic acid (10.9g) and piperidine (0.4ml) in pyridine (20 ml) was heated at reflux until the evolution of gas had subsided (1.25h). The solution was cooled and poured into a stirred mixture of ice (100g) and 2N hydrochloric acid (150 ml). The resulting solid was recovered by filtration, washed with water and dried to provide 23.1g of 4,4-diphenyl-3-butenoic acid, mp 109-111 °C. A portion was crystallized from ethyl acetate hexane to give the pure acid, mp 112.5-114 °C.

b) A solution of 4,4-diphenyl-3-butenoic acid (10.15g), 3-pyridinebutanamine (6.4g) and 1,3-dicyclohexylcarbodiimide (8.8g) in dichloromethane (100 ml) was stirred at ambient temperature for 10 days. The precipitated dicyclohexylurea was filtered off, then the evaporated filtrate was partitioned between 1N hydrochloric acid (150 ml) and ethyl acetate (2 x 50 ml). The aqueous layer was basified with 10N sodium

hydroxide and extracted with ethyl acetate. Concentration of the dried ($MgSO_4$) organic extract furnished 5.1g of crude amide which was purified by HPLC (ether hexane; 2:1) to yield 3.4g of 4,4-diphenyl-N-[4-(3-pyridinyl)butyl]-3-butenamide. A portion was crystallized from ether to give an analytical sample, 69-70° C. Anal. Calculated for $C_{25}H_{26}N_2O$ : C 81.05, H 7.07, N 7.56; Found: C 80.92, H 6.90, N 7.58.

## Example 4

a) In an inert atmosphere, sodium hydride (56% dispersion in oil; 9.5g) was triturated with pentane and then dispersed in dry dimethylsulfoxide (100 ml). The mixture was stirred at 70° C for 1 hour, then it was cooled to 0° C and (3-carboxy- propyl)triphenylphosphonium chloride (35g) was added in several portions over 5 min. The deep red solution was stirred at 0-5° C for 15 min, then a solution of benzophenone (19.86g) in dry tetrahydrofuran (100 ml) was added at such a rate that the temperature did not exceed 25° C. The light brown reaction mixture was stirred for 16 hours at room temperature, then it was diluted with water (500 ml) and extracted with dichloromethane (3 x 200 ml). The aqueous layer was acidified with 6N hydrochloric acid (40 ml) and extracted with dichloromethane (3 x 200 ml). The organic extracts were washed with water (3 x 100 ml), then combined, dried ($MgSO_4$), decolorized with charcoal and evaporated in vacuo. The residue was passed through a short column of silica gel (150g) made up in dichloromethane and the product was eluted with ethyl acetate dichloromethane (1:1). After the appropriate fractions were concentrated, the residual solid was crystallized from ether hexane to yield 14.3g of 5,5-diphenyl-4-pentenoic acid, mp 80-81.5° C.

b) Treatment of 5,5-diphenyl-4-pentenoic acid (30.3g) with thionyl chloride (30 ml) at room temperature for 15 min followed by evaporation from toluene provided the acid chloride. A solution of the acid chloride in dichloromethane (125 ml) was added over 15 min to a stirred solution of 3-pyridinebutanamine (18 g) in dichloromethane (125 ml) maintained at 0° C. The resulting mixture was allowed to equilibrate to room temperature over 1 hour and then excess 1N sodium hydroxide was added. The phases were separated and the organic layer was washed with water, dried ($Na_2SO_4$) and evaporated. The residue was purified by HPLC (ethyl acetatetriethylamine: 49:1) to afford 45.1 g of 5,5-diphenyl- N-[4-(3-pyridinyl)butyl]-4-pentenamide as a pale yellow oil. Anal. Calculated for $C_{26}H_{28}N_2O$ : C 81.21, H 7.34, N 7.29; Found: C 80.96, H 7.61, N 7.15.

## Example 5

a) The reaction was performed as in Example 4a) using sodium hydride (56% dispersion in oil; 9.5g) dimethylsulfoxide (100 ml), (3-carboxypropyl)triphenylphosphonium chloride (35g); 4,4′-dimethoxybenzophenone (26.4g) and tetrahydrofuran (100 ml). After the usual work up, the crude acid was crystallized from dichloromethanehexane to give 15.6g of 5,5-bis(4-methoxy- phenyl)-4-pentenoic acid, mp 113-114° C.

b) As in Example 1b), the mixed anhydride formed from 5,5-bis(4-methoxyphenyl)-4-pentenoic acid (8.68g), triethylamine (4.25 ml) and ethyl chloroformate (2.74 ml) in tetrahydrofuran (60 ml) was reacted with 3-pyridinebutanamine (4.38g). The material obtained from the usual work up was purified by HPLC (ethyl acetate triethylamine; 49:1) to give 9.4g of 5,5-bis(4-methoxyphenyl)-N-[4-(3-pyridinyl)butyl]-4-pentenamide as an oil. Anal. Calculated for $C_{28}H_{32}N_2O_3$: C 75.65, H 7.26, N 6.30;
Found: C 75.70, H 7.15, N 6.20.

## Example 6

a) The reaction was performed as in Example 4a) using sodium hydride (56% dispersion in oil; 9.5g) dimethylsulfoxide (100 ml), (3-carboxypropyl)triphenylphosphonium chloride (35g), 4,4′-difluorobenzophenone (23.8g) and tetrahydrofuran (100 ml). After the usual work up, the crude product was crystallized from ether hexane to furnish 18.2g of 5,5-bis(4-fluorophenyl)-4-pentenoic acid, mp 100-100° C.

b) As in Example 1b), the mixed anhydride formed from 5,5-bis(4-fluorophenyl)-4-pentenoic acid (8g), triethylamine (4.25 ml) and ethyl chloroformate (2.74 ml) in tetrahydrofuran (60 ml) was treated with 3-pyridinebutanamine (4.38g). The crude product obtained after the usual work up was purified by HPLC (ethyl acetate triethylamine; 49:1) to give 10.1g of 5,5-bis(4-fluorophenyl)-N-[4-(3-pyridinyl)butyl]-4-pentenamide as an oil. Anal. Calculated for $C_6H_{26}F_2N_2O$ : C 74.26, H 6.23, N 6.66; Found: C 73.99, H 6.20, N 6.44.

## Example 7

a) The reaction was carried out as in Example 4a) using sodium hydride (56% dispersion in oil; 8.6g), dimethylsulfoxide (100 ml), (4-carboxybutyl)triphenylphosphonium bromide (36.5g), benzophenone (18.2g) and tetrahydrofuran (100 ml). The crude product was isolated in the usual manner and crystallized from hexane to give 16.5g of 6,6-diphenyl-5-hexenoic acid, mp 111-112.5° C.

b) As in Example 1b), the mixed anhydride formed from 6,6-diphenyl-5-hexenoic acid (7.4g), triethylamine (4.25 ml) and ethyl chloroformate (2.74 ml) in tetrahydrofuran (60 ml) was reacted with 3-pyridinebutanamine (4.38g). The material obtained after the usual work up was purified by HPLC (ethyl acetate triethylamine; 49:1) to provide 8.2g of 6,6-diphenyl- N-[4-(3-pyridinyl)butyl]-5-hexenamide as an oil. Anal. Calculated for $C_{27}H_{30}N_2O$ : C 81.37, H 7.59, N 7.03; Found: C 81.27, H 7.59, N 7.06.

## Example 8

a) A mixture of 6-bromohexanoic acid (19.5g) and triphenylphosphine (26.3g) was heated at 150° for three hours in a closed flask. After the reaction was cooled, the crude product was dissolved in chloroform (200 ml) and the resulting solution was diluted just to the cloud point with ether. The mixture was stored at 0° C for several hours, then the solids were collected by filtration, washed with ether and dried in vacuo to yield 41.3g of (5-carboxypentyl)triphenylphosphonium bromide, mp 197-198° C.

b) The reaction was carried out as in Example 4a) using sodium hydride (56% dispersion in oil; 8.6g), dimethylsulfoxide (100 ml), (5-carboxypentyl)triphenylphosphonium bromide (37.64g), benzophenone (18g) and tetrahydrofuran (100 ml). The acid was isolated in the normal manner and crystallized from hexane to give 16.3g of 7,7-diphenyl-6-heptenoic acid, mp 71.5-72.5° C. A portion was recrystallized from hexane to give an analytical sample, mp 72.5-73.5° C.

c) As in Example 1b), the mixed anhydride formed from 7,7-diphenyl-6-heptenoic acid (7.8g), triethylamine (4.25 ml) and ethyl chloroformate (2.74 ml) in tetrahydrofuran (60 ml) was reacted with 3-pyridinebutanamine (4.38g). The material obtained after the usual work up was purified by HPLC (ethyl acetate triethylamine; 49:1) to furnish 9.85g of 7,7-biphenyl-N-[4-(3-pyridinyl)butyl]-6-heptenamide as an oil. Anal. Calculated for $C_{28}H_{32}N_2O$ : C 81.51, H 7.82, N 6.79; Found: C 81.56, H 7.67, N 6.77.

## Example 9

a) 7-Bromoheptanoic acid (19.7g) and triphenylphosphine (22.1g) were reacted together as in example 8a). Crystallization of the crude salt from chloroform ether afforded 38.9g of (6-carboxyhexyl)-triphenylphosphonium bromide, mp 182-183° C.

b) The reaction was carried out as in Example 4a), using sodium hydride (56% dispersion in oil; 8.36g), dimethylsulfoxide (100 ml), (6-carboxyhexyl)triphenylphosphonium bromide (37.7g), benzophenone (17.5g) and tetrahydrofuran (100 ml). The crude product was isolated in the usual way and recrystallized from hexane to yield 16.6g of 8,8-diphenyl-7-octenoic acid, mp 88-89° C.

c) As in Example 1b), the mixed anhydride formed from 8,8-diphenyl-7-octenoic acid (8.18g), triethylamine (4.25 ml) and ethyl chloroformate (2.74 ml) in tetrahydrofuran (60 ml) was reacted with 3-pyridinebutanamine (4.38g). The material obtained after the usual work up was purified by HPLC (ethyl acetate triethylamine; 49:1) to afford 8.95g of 8,8-diphenyl- N-[4-(3-pyridinyl)butyl]-7-octenamide as an oil.

The product was characterized as its (1:1) hydrobromide salt, mp 98.5-102° C, that had been crystallized from 2-propanol ethyl acetate. Anal. Calculated for $C_{29}H_{34}N_2O.HBr$ : C 68.63, H 6.95, Br 15.75, N 5.52; Found: C 68.48, H 6.68, Br 15.92, N 5.52

## Example 10

a) Triphenylphosphine (26.3g) and 8-bromooctanoic acid (21.3g) were reacted as in Example 8a. The crude salt was crystallized from a mixture of ethanol (100 ml), chloroform (200 ml) and ether (500 ml) to give 41g of (7-carboxyheptyl) triphenylphosphonium bromide, mp 121-123 °C.

b) The reaction was carried out as in Example 4a) using sodium hydride (56% dispersion in oil; 8.6g), dimethylsulfoxide (100 ml), (7-carboxyheptyl)triphenylphosphonium bromide (39.95g), benzophenone (18.2g) and tetrahydrofuran (100 ml). The crude acid was isolated in the usual manner and purified by HPLC (hexane ether; 3:2). The appropriate fractions were combined and evaporated and the residue was crystallized from hexane to provide 14.1g of 9,9-diphenyl-8-nonenoic acid, mp 77.5-78.5 °C.

c) As in Example 1b), the mixed anhydride formed from 9,9-diphenyl-8-nonenoic acid (8.75g), triethylamine (4.25 ml) and ethyl chloroformate (2.74 ml) in tetrahydrofuran (60 ml) was reacted with 3-pyridinebutanamine (4.38g). The material obtained after the usual work up was purified by HPLC (ethyl acetate triethylamine; 49:1) to give 8.3g of 9,9-diphenyl- N-[4-(3-pyridinyl)butyl)-8-nonenamide as an oil. Anal. Calculated for $C_{30}H_{36}N_{20}$ : C 81.78, H 8.24, N 6.36; Found: C 81.89, H 8.21, N 6.40.

## Example 11

A solution of 5,5-diphenyl-N-[4-(3-pyridinyl)butyl]-4-pentenamide (40.1g) in toluene (250 ml) was added in a slow stream with stirring to a 3.5M solution of sodium bis(2-methoxyethoxy)aluminum hydride in toluene (101.5 ml). After the mixture was stirred at 50 °C for 2 hours, it was cooled in an icewater bath and excess 4N sodium hydroxide was added. The separated aqueous layer was extracted with toluene and the organic layers were washed with brine, then were dried ($Na_2SO_4$) and evaporated to furnish 35.9g of crude amine. The material was purified by HPLC (ethyl acetate methanol triethylamine; 95:5:2) to yield 23g of N-(5,5-diphenyl-4-pentenyl)-3-pyridinebutanamine as an oil. The amine (5g) in methanol was treated with fumaric acid (1.6g) and ethyl acetate was added to the boiling solution to the cloud point. The resulting salt was recrystallized from methanol ethyl acetate to provide 6.2g of N-(5,5-diphenyl- 4-pentenyl)-3-pyridinebutanamine (1:1)-(E)-2-butenedioate salt, mp 160-161 °C (dec). Anal. Calculated for $C_{26}H_{30}N_2 \bullet C_4H_4O_4$: C 74.05, H 7.04, N 5.76;
Found: C 74.02, H 7.03, N 5.89.

## Example 12

A mixture of N-(5,5-diphenyl-4-pentenyl)-3-pyridinebutanamine (10g) and 98% formic acid (6 ml) in toluene (50 ml) was refluxed for 2 hours through a Soxhlet extractor charged with 4A molecular sieves. The cooled solution was washed in turn with water, saturated sodium carbonate solution and brine. The dried ($Na_2SO_4$) toluene phase was evaporated to give 9.5g of crude amide which was purified by HPLC (ethyl acetate triethylamine; 49:1) to furnish 8.5g of N-(5,5-diphenyl-4-pentenyl)-N-[4-(3-pyridinyl)butyl]formamide as a colorless oil. Anal. Calculated for $C_{27}H_{30}N_2O$ :
C 81.37, H 7.59, N 6.96; Found: C 81.50, H 7.64, N 6.96.

## Example 13

A solution of N-(5,5-diphenyl-4-pentenyl)-N-[4-(3-pyridinyl)butyl]formamide (4.5g) in toluene (50 ml) was added dropwise with stirring to a 3.5M solution of sodium bis(2-methoxyethoxy)aluminum hydride in toluene (13 ml) at 50 °C. After 30 min the cooled solution was treated with excess 4N sodium hydroxide and the separated aqueous phase was extracted with toluene. The organic phases were washed with brine, then were combined, dried ($Na_2SO_4$) and evaporated to afford 4.2g of crude amine. This material (4.2g) in methanol was treated with oxalic acid dihydrate (1.4g) and ethyl acetate was added to the boiling solution just to the cloud point. The resulting solids were recrystallized from methanol ethyl acetate to provide 4.6g

of N-(5,5-diphenyl- 4-pentenyl)-N-methyl-3-pyridinebutanamine ethanedioate 0.25 molar hydrate, mp 118-119 °C. Anal. Calculated for $C_{27}H_{32}N_2 \bullet C_2H_2O_4 . 0.25H_2O$:
C 72.70, H 7.26, N 5.85; Found: C 72.78, H 7.20, N 5.84.

## Example 14

a) A solution of 2.5M n-butyl lithium in hexane (160 ml) was added to a stirred mixture of 6-methoxytetralone (70.5g) in diethyl ether (500 ml) maintained at 10 °C. The reaction was stirred at room temperature overnight. then water (20 ml) was added dropwise over several minutes followed by 2N HCl (200 ml). The phases were separated and the aqueous layer was extracted with diethyl ether (2 x 250 ml). The combined organic extracts were dried (MgSO₄) and evaporated to give 80g of a mixture (about 1:1) of 1-butyl 1-hydroxy 6-methoxytetralin and the starting ketone as an oil.

A solution of the crude material (80g) in chloroform (250 ml) containing trifluoroacetic acid (25 ml) was stirred at ambient temperature for 16 hours then the solvents were removed under reduced pressure. The residue was partitioned between dichloromethane (500 ml) and 1N sodium hydroxide (200 ml) and the separated aqueous layer was extracted with dichloromethane (100 ml). The organic extracts were washed with brine, then combined, dried (MgSO₄) and concentrated in vacuo. The resulting oil was passed through a column of silica gel (400g) made up in hexane and eluted with hexane. Evaporation of the fractions (3 x 500 ml) containing the non-polar product furnished 34.9g of 1-butyl-3,4-dihydro-6-methoxynaphthalene as an oil.

b) Phosphorus oxychloride (16.6 ml) was added dropwise with stirring to dimethylformamide (70 ml) at -5 °C. After the addition was completed, the mixture was stirred at 0 °C for 15 minutes, then a solution of 1-butyl-3,4-dihydro-6-methoxy- naphthalene (34.9g) in dimethylformamide (30 ml) was added slowly over 15 minutes while the reaction temperature was maintained at 0 °C. The cooling bath was then withdrawn and after the mixture had stirred at room temperature for 1.5 hours, a few chips of ice were added, followed, after 5 minutes, by 10N sodium hydroxide (200 ml). The reaction was heated to 110 °C which caused the vigorous evolution of dimethylamine from the mixture and after 10 minutes the reaction was cooled. diluted with water (750 ml) and extracted with dichloromethane (1 x 500 ml; 2 x 300 ml). The organic layers were washed with water (2 x 200 ml), then combined. dried (MgSO₄) and evaporated to furnish an amber oil which was passed through a short column of silica gel (200g) in dichloromethane. The fractions containing the product were evaporated to yield 39.4g of crude 1-butyl-3,4-dihydro-6-methoxy-2-naphthalene-carboxaldehyde as an oil.

c) 1-Butyl-3,4-dihydro-6-methoxy-2-naphthalenecarboxaldehyde (4.6g) and 2,3-dichloro-4,5-dicyano-1,4-benzoquinone (5.43g) were stirred together in benzene (100 ml) at reflux for 5.5 hours. The reaction was then cooled and after the precipitated hydroquinone was filtered off, the filtrate was washed with 1N sodium hydroxide (3 x 75 ml). Each aqueous layer was backwashed with benzene (50 ml), then the combined extracts were dried (MgSO₄) and evaporated to provide 4.2g of product. Crystallization of the material from 2-propanol afforded 3.1g of 1-butyl-6-methoxy-2-naphthalenecarboxaldehyde as a light tan solid mp 56-57 °C. A sample was recrystallized from the same solvent to yield an analytical specimen, mp 57-58 °C.

d) A solution of 1-butyl-6-methoxy-2-naphthalenecarboxaldehyde (1.6g) and (carbomethoxymethylene)-triphenylphosphorane (2.45g) in dichloromethane (20 ml) was stirred at room temperature for 40 hours. The solvent was removed in vacuo and the residue was triturated with a mixture of diethyl ether (20 ml) and hexane (50 ml). After the resulting solid was filtered off, the filtrate was evaporated and the residue was passed through a column of silica gel (20g) made up in diethyl ether hexane (1:9). Evaporation of the appropriate fractions furnished 1.75g of (E)-3-(1-butyl 6-methoxy-2-naphthalenyl) 2-propenoic acid methyl ester. A sample was crystallized from hexane to give an analytical sample, mp 71-72 °C.

e) A solution of (E)-3-(1-butyl-6-methoxy-2-naphthalenyl)-2-propenoic acid methyl ester (1.7g) in methanol (10 ml) was treated with 2N sodium hydroxide (4.5 ml) and the mixture was stirred at reflux for 1 hour. The warm reaction mixture was acidified by the addition of 1N hydrochloric acid (9.2 ml). After the mixture cooled, the resulting solid was filtered off to provide 1.6g of (E)-3-(1-butyl-6-methoxy-2-naphthalenyl)-2-propenoic acid. Crystallization of a portion from methanol gave an analytical specimen, mp 163-164 °C.

f) A stirred mixture of (E)-3-(1-butyl-6-methoxy-2-naphthalenyl)-2-propenoic acid (1.5g) and 4-nitrophenol (0.81g) in dichloromethane, was cooled in an ice bath, during the addition of a solution of 1,3-dicyclohexylcarbodiimide (1.1g) in dichloromethane (5 ml) then the reaction was stirred at room temperature over the weekend. After the precipitated dicyclohexylurea was removed by filtration, the filtrate was

concentrated and applied to a column of silica gel (25g) made up in dichloromethane hexane (2:1). Elution with the same solvent mixture and concentration of the appropriate fractions yielded 2g of (E)-3-(1-butyl-6-methoxy- 2-naphthalenyl)2-propenoic acid 4-nitrophenyl ester. Crystallization of the material from 2-propanol gave 1.85g of the active ester, mp 140-141 ° C.

g) A solution of (E)-3-(1-butyl 6-methoxy-2-naphthalenyl]-2-propenoic acid 4-nitrophenyl ester (1.8g) and (R)-α-methyl- 3-pyridinebutanamine (0.73g) in tetrahydrofuran (25 ml) was stirred at room temperature for 42 hours. After the solvent was removed in vacuo, the residue was dissolved in dichloromethane (75 ml) and washed with 0.5N sodium hydroxide (3 x 50 ml). The aqueous layers were back washed with dichloromethane (50 ml) and then the combined extracts were dried ($K_2CO_3$) and evaporated. The crude product was purified by HPLC (ethyl acetate) and then was crystallized from dichloromethane hexane to yield 1.5 g of [R-(E)]-3-(1-butyl-6-methoxy-2-naphthalenyl)-N-[1-methyl-4-(3-pyridinyl)butyl]-2-propenamide, mp 159-160 ° C. Anal. Calcd for $C_{28}H_{34}N_2O_2$: C 78.10, H 7.96, N 6.51; Found: C 78.42, H 7.93, N 6.45.

## Example 15

a) As in Example 14 d), 1-butyl-3,4-dihydro-6-methoxy-2-naphthalenecarboxaldehyde (3.05g) and (carbomethoxymethylene)triphenylphosphorane (4.5g) in dichloromethane (25 ml) was stirred at room temperature for 64 hours. The usual work up furnished 2.9g of (E)-3-(1-butyl-3,4-dihydro-6-methoxy-2-naphthalenyl)-2-propenoic acid methyl ester as an oil.

b) As in Example 14 e), (E)-3-(1-butyl-3,4-dihydro-6-methoxy-2-naphthalenyl)-2-propenoic acid methyl ester (2.9g) in methanol (12 ml) was treated with 2N sodium hydroxide (7 ml) at reflux for 1.5 hours. The usual work up provided 2.6g of (E)-3-(1-butyl-3,4-dihydro-6-methoxy- 2-naphthalenyl)-2-propenoic acid as an oil.

c) As in Example 14 f), (E)-3-(1-butyl-3.4-dihydro-6-methoxy-2-naphthalenyl)-2-propenoic acid (1.8g) was reacted with 4-nitrophenol (0.97g) in dichloromethane (30 ml) in the presence of 1,3-dicyclohexylcarbodiimide (1.3g) overnight at room temperature. The normal work up yielded 1.5g of crude ester. Crystallization from diethyl ether/2-propanol furnished (E)-3-(1-butyl-3,4-dihydro-6-methoxy-2-naphthalenyl)-2-propenoic acid 4-nitrophenyl ester, mp 94-95 ° C.

d) As in Example 14g), (E)-3-(1-butyl-3,4-dihydro 6-methoxy-2-naphthalenyl)-2-propenoic acid 4-nitrophenyl ester (1.1g) was treated with (R)-α-methyl-3-pyridinebutanamine (0.45g) in tetrahydrofuran (10 ml) at room temperature for 24 hours and then at 50 ° C for 4 hours. The crude product was purified by HPLC (ethyl acetate) to afford 1.05g of [R-(E)]-3-(1-butyl-3,4-dihydro-6-methoxy-2-naphthalenyl)-N-[1-methyl-4-(3-pyridinyl)butyl]-2-propenamide as an amorphous solid. Anal. Calcd. for $C_{28}H_{36}N_2O_2$: C 77.74, H 8.39, N 6.47; Found: C 77.22, H 8.71, N 6.32.

## Example 16

a) A solution of 4-methoxyphenyl magnesium bromide, freshly prepared in the normal manner in tetrahydrofuran (200 ml) from 4-bromoanisole (101g) and magnesium metal (14.5g), was added over 45 minutes at 0 ° C with stirring to a solution of 6-methoxytetralone (85g) in dry tetrahydrofuran (500 ml). After the addition was completed, the reaction was stirred at room temperature for 2 hours and the excess reagent was destroyed by the careful addition of water (50 ml). Most of the solvent was removed in vacuo and the concentrate was partitioned between ethyl acetate and 1N sodium hydroxide.

After the resultant solids were filtered off, the layers were separated and the organic layer was washed with brine, then dried ($MgSO_4$) and evaporated to give 123g of the crude carbinol as an oil. A solution of this carbinol (100g) in toluene (800 ml) containing p-toluenesulfonic acid (1g) was refluxed for 3 hours in a flask equipped with a Dean-Stark trap. The cooled solution was washed with 10% sodium bicarbonate and with brine, then dried ($MgSO_4$) and evaporated. The residual material was passed though a short column of silica gel (250g) made up in dichloromethane hexane (1:3) and the product was eluted with dichloromethane hexane (1:1). Evaporation of the appropriate fractions and crystallization of the residue from toluene hexane (2x) gave 37.1g of 3,4-dihydro-6-methoxy-1-(4-methoxyphenyl)naphthalene, mp 100.5-102 ° C.

b) As in Example 14b), 3,4-dihydro-6-methoxy-1-(4-methoxy- phenyl)naphthalene (17g) was added to the reagent formed from the addition of phosphorus oxychloride (6.54 ml) to dimethylformamide (35 ml) at -5 ° C. The cooling bath was then withdrawn and after the mixture had stirred at 35 ° C for 1.5 hours, a few

chips of ice were added followed after 5 minutes by 10N sodium hydroxide (70 ml). After the reaction was heated to 110° C for 10 minutes the reaction was cooled and the resulting solid was filtered off, washed with water and dried in vacuo to give 18.75g of 3,4-dihydro-6-methoxy-1-(4-methoxyphenyl)-2-naphthalenecarboxaldehyde, mp 104-105° C. Crystallization of a portion from diethyl ether furnished the pure aldehyde, mp 105-106° C.

c) As in Example 14d), 3,4-dihydro-6-methoxy 1-(4-methoxyphenyl)-2-naphthalenecarboxaldehyde (2.94g) and (carbomethoxymethylene)triphenylphosphorane (3.7g) in dichloro methane (30ml) was stirred at room temperature for 64 hours. After the normal work up, the crude product was crystallized from 2-propanol to afford 2.65g of (E)-3-[3,4 dihydro-6-methoxy-1-(4-methoxyphenyl)-2-naphthalenyl]-2-propenoic acid methyl ester, mp 116-118° C.

d) As in Example 14 e), (E)-3-[3,4-dihydro-6-methoxy-1-(4-methoxyphenyl)-2-naphthalenyl]-2-propenoic acid methyl ester (2.5g) in methanol (15 ml) was heated at reflux with 1N sodium hydroxide (11 ml) at reflux for 1 hour. The usual work up furnished 2g of (E)-3-[3,4-dihydro-6-methoxy-1-(4-methoxyphenyl)-2-naphthalenyl]-2-propenoic acid. Crystallization of a small sample from dichloromethane ethyl acetate gave an analytical specimen, mp 222-224° C.

e) As in Example 14 f), (E)-3-[3,4-dihydro-6-methoxy-1-(4-methoxyphenyl)-2-naphthalenyl]-2-propenoic acid (1.7g) was reacted with 4-nitrophenol (0.775g) in dichloromethane (25 ml) in the presence of 1,3-dicyclohexylcarbodiimide (1.04g) overnight at room temperature. After the normal work up, the crude ester was triturated with diethyl ether and then crystallized from 2-propanol to give 1g of (E)-3-[3,4-dihydro-6-methoxy-1-(4-methoxyphenyl)-2-naphthalenyl]-2-propenoic acid 4-nitrophenyl ester as a yellow solid, mp 151-152° C.

f) As in Example 14 g), (E)-3-[3,4-dihydro-6-methoxy-1-(4-methoxyphenyl)-2-naphthalenyl]-2-propenoic acid 4-nitrophenyl ester (0.719) was treated with (R)-α-methyl-3-pyridinebutanamine (0.33g) in tetrahydrofuran (5 ml) at room temperature for 24 hours. The crude product was purified by HPLC (ethyl acetate) to yield 0.625g of [R-(E)-]-3-[3,4-dihydro-6-methoxy-1-(4-methoxyphenyl)-2-naphthalenyl]-N-[1-methyl-4-(3-pyridinyl)butyl]-2-propenamide as an amorphous solid. Anal. Calcd for $C_{31}H_{32}N_2O_3{\bullet}0.25H_2O$: C 76.43, H 7.14, N 5.75, $H_2O$ 0.93; Found: C 76.51, H 7.15, N 5.70, $H_2O$ 1.04.

## Example 17

a) As in Example 14 c), 3,4-dihydro-6-methoxy-1-(4-methoxy- phenyl)-2-naphthalenecarboxaldehyde (9.7g) and 2,3 dichloro- 4,5 dicyano-1,4-benzoquinone (9.36g) were stirred together in benzene (150 ml) at reflux for 17 hours. The usual workup provided 9.5g of crude product which was crystallized from 2-propanol to give 7.4g of 6-methoxy-1-(4 methoxy- phenyl)-2-naphthalenecarboxaldehyde, mp 99-101° C.

b) As in Example 14 d), 6-methoxy-1-(4-methoxyphenyl)-2-naphthalenecarboxaldehyde (2.92g) and (carbomethoxymethylene)triphenylphosphorane (3.7g) in dichloromethane (30ml) was stirred at room temperature for 18 hours. After the normal work up, the crude product was crystallized from diethyl ether hexane to provide 1.95g of (E)-3-[6-methoxy-1-(4-methoxyphenyl)-2-naphthalenyl]-2-propenoic acid methyl ester. Recrystallization of a sample from the same solvents furnished the pure aldehyde, mp 118-121° C.

c) As in Example 14 e), (E)-3-[6-methoxy-1-(4-methoxyphenyl)-2-naphthalenyl]-2-propenoic acid methyl ester (1.8g) in methanol (10 ml) was heated at reflux with 1N sodium hydroxide (8 ml) for 1 hour. The usual work up provided 1.6g of (E)-3-[6-methoxy-1-(4-methoxyphenyl)-2-naphthalenyl]-2-propenoic acid. Crystallization of a small portion from dichloromethane ethyl acetate gave an analytical sample, mp 248-249° C.

d) As in Example 14 f), (E)-3-[6-methoxy-1-(4-methoxyphenyl)-2-naphthalenyl]-2-propenoic acid (1.4g) was reacted with 4-nitrophenol (0.64g) in dichloromethane (25 ml) in the presence of 1,3-dicyclohexylcarbodiimide (0.87g) overnight at room temperature to give, after the normal work up and crystallization of the crude product from 2-propanol, 1.6g of (E)-3-[6-methoxy-1-(4-methoxyphenyl)-2-naphthalenyl]-2-propenoic acid 4-nitrophenyl ester, mp 160-161° C.

e) As in Example 14 g), (E)-3-[6-methoxy-1-(4-methoxyphenyl)-2-naphthalenyl]-2-propenoic acid 4-nitrophenyl ester (1.37g) was treated with (R)-α-methyl-3-pyridinebutanamine (0.51g) in tetrahydrofuran (10 ml) at room temperature for 24 hours. The crude amide was purified by HPLC (ethyl acetate) to yield 1.15g of [R-(E)]-3-[6-methoxy-1-(4-methoxyphenyl)-2-naphthalenyl]-N-[1-methyl-4-(3-pyridinyl) butyl]-2-propenamide as an amorphous solid. Anal. Calcd for $C_{31}H_{32}N_2O_3{\bullet}0.25H_2O$: C 76.75, H 6.75, N 5.77, $H_2O$, 0.97; Found: C 76.69, H 6.63, N 5.73, $H_2O$ 1.05.

Example 18

a) To a solution of potassium t-butoxide (25g) in t-butanol (180 ml) at 60 °C was added a mixture of diethyl succinate (52.3g) and 1-(3-methoxyphenyl)pentanone (38.5g) at a rapid dropwise rate and the reaction was refluxed with stirring for 2.5 hours. After the solvent was removed under reduced pressure, the residue was dissolved in water (250 ml) and extracted with diethyl ether (3 x 150 ml) to remove neutral materials. The aqueous layer was then acidified and extracted with dichloromethane (3 x 200 ml) to yield, after evaporation of the dried ($MgSO_4$) organic extracts, 61.3g of a mixture of the isomeric 3-(ethoxycarbonyl)-4-(3-methoxyphenyl)-3-octenoic acids as an oil. A solution of the above mixture of acids (15g) and sodium acetate (3.9g) in acetic anhydride (90 ml) was heated at reflux for 4 hours. The solvent was removed in vacuo and the residue was partitioned between dichloromethane (200 ml) and 10% potassium carbonate (100 ml). The dried ($MgSO_4$) organic phase was concentrated and the resulting mixture was separated by HPLC (diethyl ether hexane; 1:3). Evaporation of the appropriate fractions furnished two isomeric compounds weighing 7.2g and 2.7g respectively. Crystallization of the major component from hexane afforded pure 4-acetoxy-1-butyl- 7-methoxy-2-naphthalenecarboxylic acid ethyl ester, mp 70-71.5 °C.

Crystallization of the more polar minor isomer from hexane gave 4-acetoxy-1-butyl-5-methoxy-2-naphthalenecarboxylic acid ethyl ester, mp 86-88 °C.

b) A solution of 4-acetoxy-1-butyl-5-methoxy-2-naphthenecarboxylic acid ethyl ester (3.3g) in 1.05M ethanolic hydrogen chloride (35 ml) was heated at reflux for 1.5 hour and then the solvent was removed under reduced pressure. Crystallization of the residual material from hexane provided 2.5g of 1-butyl-4-hydroxy-5-methoxy-2-naphthalenecarboxylic acid ethyl ester, mp 80-82 °C.

c) Potassium t-butoxide (1.03g) was added to a solution of 1-butyl-4-hydroxy-5-methoxy-2-naphthalenecarboxylic acid ethyl ester (2.4g) and 5-chloro-1-phenyl-1H-tetrazole (1.55g) in dimethylformamide (20 ml) and the reaction was stirred at room temperature for 1.25 hours. After the solvent was removed in vacuo, the residual material was partitioned between dichloromethane (150 ml) and water (75 ml). The separated aqueous layer was re-extracted with dichloromethane, and after each organic extract was washed with water, they were combined, dried ($Na_2SO_4$) and evaporated. Crystallization of the crude product from diethyl ether hexane gave 2.15g of 1-butyl-5-methoxy-4-[(1-phenyl-1H-tetrazole-5-yl)oxy]-2-naphthalenecarboxylic acid ethyl ester, mp 144-146 °C. A portion was recrystallized from the same solvents to provide an analytical sample, mp 145-146 °C.

d) A solution of 1-butyl-5-methoxy-4-[(1-phenyl-1H-tetrazole-5-yl)oxy]-2-naphthalenecarboxylic acid ethyl ester in a mixture of tetrahydrofuran (25 ml) and ethanol (75 ml) was hydrogenated over 10% palladium on carbon (1.3g) at 50 °C and three atmospheres. After the uptake of hydrogen had stopped, the catalyst was removed by filtration and the filtrate was concentrated to dryness. The resulting material was dissolved in diethyl ether (150 ml) and the solution was extracted with 1N sodium hydroxide (2 x 50 ml). After the base extracts were washed with diethyl ether, the combined organic layers were dried ($MgSO_4$) and evaporated to yield 3.6g of 1-butyl-5-methoxy-2-naphthalenecarboxylic acid ethyl ester as an oil.

e) As in Example 14 e), 1-butyl-5-methoxy-2-naphthalenecarboxylic acid ethyl ester (3.45g) in methanol (40 ml) was saponified with 2N sodium hydroxide (10 ml) at reflux for 1.75 hours. 3.1g of crude acid, mp 151-153 °C, was obtained from the usual work up and crystallization of a sample from diethyl ether hexane gave 1-butyl-5-methoxy-2- naphthalenecarboxylic acid, mp 154-155 °C.

f) A solution of borane in tetrahydrofuran (1M; 10 ml) was added to a solution of 1-butyl-5-methoxy-2-naphthalene carboxylic acid in tetrahydrofuran (15 ml) at 0-5 °C and the mixture was stirred at room temperature for 3 hours. After the solvent was evaporated off, the reaction was diluted with 1N sodium hydroxide and extracted with dichloromethane. The organic phase was washed with brine, then dried ($MgSO_4$), evaporated and crystallized from hexane to give 1.58 g of 1-butyl-5-methoxy-2-naphthalenemethanol, mp 100-102 °C. Recrystallization of a portion from diethyl ether hexane afforded the pure alcoholic, 101-102 °C. Recrystallization of a portion from diethyl etherhexane afforded the pure alcohol, 101-102 °C.

g) A solution of dimethylsulfoxide (0.54 ml) in dry dichloromethane (3 ml) was added with stirring to a cooled (below -60 °C) solution of oxalyl chloride (0.59 ml) in dry dichloromethane (15 ml) at such a rate that the reaction temperature did not exceed -60 °C. After 15 minutes a solution of 1-butyl-5-methoxy-2-naphthalenemethanol (1.54g) in dichloromethane (6 ml) was added while the reaction temperature was still maintained below -60 °C. The reaction was allowed to proceed for 15 minutes then triethylamine (2.9 ml) was added to the reaction and, after an additional 20 minutes the cooling bath was removed and the mixture was allowed to equilibrate to room temperature. After 2N hydrochloric acid (225 ml) was added, the

layers were separated and the aqueous phase was extracted with dichloromethane (50 ml), then the combined organic extracts were dried ($MgSO_4$) and evaporated to yield 1.6g of 1-butyl-5-methoxy-2-naphthalenecarboxaldehyde. Sublimation of a portion (50°C; 13.3 pa (0.1 mm)) furnished an analytical sample, mp 61.5-62.5°C.

h) As in Example 14 d), 1-butyl-5-methoxy-2-naphthalene carboxaldehyde (1.5g) and (carbomethoxymethylene) triphenylphosphorane (2.13g) in dichloromethane (20ml) was stirred at room temperature for 42 hours to provide, after the usual work up, 1.75g of (E)-3-(1-butyl-5-methoxy-2-naphthalenyl)-2-propenoic acid methyl ester. Crystallization of a sample from hexane provided the pure ester, mp 53.5-54.5°C.

i) As in Example 14 e), (E)-3-(1-butyl-5-methoxy-2 naphthalenyl)-2-propenoic acid methyl ester (1.65g) in methanol (10 ml) was treated with 2N sodium hydroxide solution (6 ml) at reflux for 2 hours. A sample of the acid (1.55g) obtained from the usual work up was crystallized from diethyl ether to give pure (E)-3-(1-butyl-5-methoxy-2-naphthalenyl)-2-propenoic acid, mp 202-204°C.

j) As in Example 14 f), (E)-3-(1-butyl-5-methoxy-2-naphthalenyl)-2-propenoic acid (1.4g) was reacted with 4-nitrophenol (0.76g) in dichloromethane (25 ml) in the presence of 1,3-dicyclohexylcarbodiimide (1.02g) to give 1.51g of (E)-3-(1-butyl-5-methoxy-2-naphthalenyl)-2-propenoic acid 4-nitrophenyl ester. Crystallization of a portion from diethyl ether afforded an analytical sample, mp 115-116°C.

k) As in Example 14 g), (E)-3-(1-butyl-5-methoxy-2-naphthalenyl)-2-propenoic acid 4-nitrophenyl ester (1.4g) was reacted with (R)-α-methyl-3-pyridinebutanamine (0.575g) in tetrahydrofuran (25 ml) at room temperature for 17 hours. The product was isolated in the usual way and then was crystallized from ethyl acetate hexane to afford 1.18g of [R-(E)]-3-(1-butyl-5-methoxy-2-naphthalenyl)-N-[1-methyl-4-(3-pyridinyl)-butyl]-2-propenamide, mp 138-139°C. Anal. Calcd for $C_{29}H_{36}N_2O_3$: C 78.10, H 7.96, N 6.51; Found: C 77.81, H 7.96, N 6.60.

## Example 19

a) As in Example 18 b), a solution of 4-acetoxy-1-butyl-7-methoxy-2-naphthenecarboxylic acid ethyl ester (9.1g) in 1.05M ethanolic hydrogen chloride (100 ml) was heated at reflux for 2.5 hours. After removal of the solvent the residue was crystallized from hexane to give 6.5g of 1-butyl-4-hydroxy-7-methoxy-2-naphthalenecarboxylic acid ethyl ester, mp 112-115°C. Recrystallization of a sample from hexane afforded an analytical specimen, mp 114-115°C.

b) As in Example 18 c), 1-butyl-4-hydroxy-7-methoxy-2-naphthalenecarboxylic acid ethyl ester (2.9g) was reacted with 5-chloro-1-phenyl-1H-tetrazole (1.87g) in dimethylformamide (25 ml) in the presence of potassium t-butoxide (1.25g). After 0.5 hours at room temperature, the reaction was worked up in the described manner and the crude product was crystallized from diethyl ether hexane to afford 2.9g of 1-butyl-7-methoxy-4-[(1-phenyl-1H-tetrazole-5-yl)oxy]-2-naphthalenecarboxylic acid ethyl ester, mp 94-96°C. A sample was recrystallized from the same solvents to provide an analytical specimen, mp 95.5-96.5°C.

c) As in Example 18 d), 1-butyl-7-methoxy-4-[(1-phenyl-1H-tetrazole-5-yl)oxy]-2-naphthalenecarboxylic acid ethyl ester (2.85g) was hydrogenolyzed in ethanol (100 ml) over 10% palladium on carbon (0.57g) at 50°C and three atmospheres to give. after the usual work up, 1.5g of crude 1-butyl-7-methoxy-2-naphthalenecarboxylic acid ethyl ester as an oil. The material was combined with 4.8g of crude product from another experiment and was purified by HPLC (diethyl ether hexane; 1:9) to give 5.8g of pure ester.

d) As in Example 14 e), 1-butyl-7-methoxy-2-naphthalenecarboxylic acid ethyl ester (5.4g) in methanol (40 ml) was reacted with 2N sodium hydroxide solution (20 ml) at reflux for 2 hours to give, after the normal work up, 4.9g of crude acid. Crystallization of the material from diethyl ether hexane furnished 4.22g of 1-butyl-7-methoxy-2-naphthalenecarboxylic acid, mp 123-125°C.

e) As in Example 18 f), 1-butyl-7-methoxy-2 naphthalenecarboxylic acid (2.73g) in tetrahydrofuran (15 ml) was treated with a solution of borane in tetrahydrofuran (1M; 22 ml) at 0-5°C and the mixture was stirred at room temperature overnight. The product was isolated in the usual manner and was crystallized from hexane to give 2.32 g of 1-butyl-7-methoxy-2-naphthalenemethanol, mp 52-53°C.

f) As in Example 18 g), 1-butyl-7-methoxy-2-naphthalenemethanol (2.25g) was oxidized by using the reagent prepared from dimethylsulfoxide (0.54 ml) and oxalyl chloride (0.9 ml) in dry dichloromethane (25 ml). The usual work up gave 2g of 1-butyl-7-methoxy-2-naphthalenecarboxaldehyde.

g) As in Example 14 d), 1-butyl-7-methoxy-2-naphthalenecarboxaldehyde (1.7g) and (carbomethoxymethylene)triphenylphosphorane (2.85g) in dichloromethane (20ml) was stirred at room temperature for 42 hours to give after the normal work up, 1.6g of (E)-3-(1-butyl-7-methoxy-2-naphthalenyl)-2-propenoic acid methyl ester as an oil.

h) As in Example 14 e), (E)-3-(1-butyl-7-methoxy-2- naphthalenyl)-2-propenoic acid methyl ester (1.55g) in methanol (20 ml) was treated with 2N sodium hydroxide solution (4 ml) at reflux for 2 hours and the normal isolation procedure afforded 1.45g of (E)-3-(1-butyl-7-methoxy-2-naphthalenyl)-2-propenoic acid. Crystallization of a sample from diethyl ether gave an analytical specimen.

i) As in Example 14 f), (E)-3-(1-butyl-7-methoxy-2-naphthalenyl)-2-propenoic acid (1.35g) was reacted with 4-nitrophenol (0.73g) in dichloromethane (25 ml) in the presence of 1,3-dicyclohexylcarbodiimide (0.99g) to give 1.6g of (E)-3-(1-butyl-7-methoxy-2-naphthalenyl)-2-propenoic acid 4-nitrophenyl ester. Crystallization of a portion from diethyl ether furnished an analytical sample, mp 114-115 °C.

j) As in Example 14 g), (E)-3-(1-butyl-7-methoxy-2-naphthalenyl)-2-propenoic acid 4-nitrophenyl ester (1.73g) was treated with (R)-α-methyl-3-pyridinebutanamine (0.71g) in tetrahydrofuran (25 ml) at room temperature overnight. The product was isolated in the described manner and then crystallized from ethyl acetate hexane to furnish 1.32g of [R-(E)]-3-(1-butyl-7-methoxy-2-naphthalenyl)-N-[1-methyl-4-(3-pyridinyl)-butyl]-2-propenamide, mp 127-128 °C. Anal. Calcd. for $C_{28}H_{34}N_2O_2$: C 78.10, H 7.96, N 6.51; Found: C 77.79, H 8.01, N 6.55.

## Example 20

a) A solution of 1-butyl-4-hydroxy-7-methoxy-2 naphthalenecarboxylic acid ethyl ester (6.5g) and methyl iodide (7 ml) in acetone (65 ml) containing potassium carbonate (5g) was stirred at room temperature for 64 hours. After the reaction mixture was filtered, the filtrate was concentrated to dryness and the residue was partitioned between dichloromethane (150 ml) and water (100 ml). The dried ($MgSO_4$) organic phase was evaporated and the residual material crystallized from 2-propanol to yield 5.35g of 1-butyl-4,7 dimethoxy-2-naphthalenecarboxylic acid ethyl ester, mp 44-45 °C.

b) N-methylpiperazine (8.3 ml) in toluene (15 ml) was added dropwise over 5 minutes to a cooled (-5 °C) mixture of sodium bis(2-methoxyethoxy)aluminum hydride in toluene (3.4M; 20.6 ml) and toluene (25 ml). This solution was added to a stirred solution of 1-butyl-4,7-dimethoxy--2- naphthalenecarboxylic acid ethyl ester (4g) in toluene (50 ml) at -40 °C. After 5 hours the cooling bath was removed and the reaction was allowed to equilibrate to -15 °C over 45 minutes, whereupon conc. hydrochloric acid (30 ml) was added slowly to destroy excess reagent. The mixture was diluted with ice cold 1 N hydrochloric acid (200 ml), and after the phases were separated. the aqueous phase was extracted with toluene (2 x 150 ml). The combined organic extracts were dried ($MgSO_4$) and evaporated to yield 1.35g of crude 1-butyl-4,7-methoxy-2-naphthalenecarboxaldehyde as an oil. This material was used in subsequent reactions without further purification.

c) As in Example 14 d), 1-butyl-4,7-dimethoxy-2-naphthalenecarboxaldehyde (1.35g) and (carbomethoxymethylene)triphenylphosphorane (1.35g) in dichloromethane (10 ml) was stirred at room temperature for 42 hours. The crude ester obtained from the usual work up was dissolved in methanol (25 ml) containing 1N sodium hydroxide (4 ml) and heated at reflux for 2 hours. The normal isolation procedure furnished 0.725g of (E)-3-(1-butyl-4,7-dimethoxy-2-naphthalenyl)-2-propenoic acid. A portion was crystallized from diethyl etherhexane to give an analytical specimen, mp 174-176 °C.

d) As in Example 14 f), (E) 3-(1-butyl-4,7-dimethoxy-2-naphthalenyl)-2-propenoic acid (0.71g) was treated with 4-nitrophenol (0.35g) in dichloromethane (10 ml) in the presence of 1,3-dicyclohexylcarbodiimide (0.475g) to furnish, after the normal work up and crystallization of the crude product from 2-propanol, 1.17g of (E)-3-(1-butyl-4,7-dimethoxy-2-naphthalenyl)-2-propenoic acid 4-nitrophenyl ester., mp 144-145.5 °C.

e) As in Example 14 g), (E)-3-(1-butyl-4,7-dimethoxy-2-naphthalenyl)-2-propenoic acid 4-nitrophenyl ester (1g) was reacted with (R)-α-methyl-3-pyridinebutanamine (0.385g) in tetrahydrofuran (15 ml) at room temperature for 17 hours. The product was isolated in the usual manner and crystallized twice from ethyl acetate hexane to provide 0.65g of [R-(E)]-3-(1-butyl-4,7-dimethoxy-2-naphthalenyl)-N-[1-methyl-4-(3-pyridinyl)butyl]-2-propenamide, mp 168-169 °C. Anal. Calcd. for $C_{29}H_{36}N_2O_3$: C 75.62, H 7.88, N 6.08; Found: C 75.33, H 7.65, N 6.12.

Example 21

a) As in Example 18 g), 1-phenylpentanone (50g) was reacted with diethyl succinate (80.5g) in t-butanol at 60° C for 6 hours in the presence of potassium t-butoxide (38g). The normal work up furnished 81g of a mixture of the isomeric 3-(ethoxycarbonyl)-3-octenoic acids as an oil. The acids (79.4g) were cyclized by refluxing in acetic anhydride (500 ml) containing sodium acetate (22.5g) for 9 hours. After work up, the crude product was passed through a short column of silica gel (650g) made up in dichloromethane hexane (2:1) to give 60g of 4-acetoxy-1-butyl -2-naphthalenecarboxylic acid ethyl ester as an oil.

b) As in Example 18 b), a solution of 4-acetoxy-1-butyl-2-naphthenecarboxylic acid ethyl ester (60g) in 1M ethanolic hydrogen chloride (500 ml) was heated at reflux for 0.75 hours. After the solvent was evaporated, the residue was crystallized from hexane to give 46.6g of 1-butyl-4-hydroxy-2-naphthalenecarboxylic acid ethyl ester, mp 117-120° C. Recrystallization of a portion from hexane gave the pure ester, mp 118-119° C.

c) 1-Butyl-4-hydroxy-2-naphthalenecarboxylic acid ethyl ester (27g) was reacted with methyl iodide (17 ml) in acetone (200 ml) containing potassium carbonate (13.8g) with stirring at room temperature for 24 hours. The crude product obtained after the usual work up was distilled on a Kugelrohr apparatus (135-138° C; 13.3 pa (0.1 mm)) to provide 25.8g of 1-butyl-4-methoxy-2-naphthalenecarboxylic acid ethyl ester as an oil.

d) As in Example 20 b), a solution of 1-butyl-4-methoxy-2-naphthalenecarboxylic acid ethyl ester (8.6g) in toluene (100 ml) was reacted with the reagent formed from the addition of N-methylpiperazine ( 19.6 ml) to a mixture of sodium bis(2-methoxyethoxy)aluminum hydride in toluene (3.4M; 49 ml) and toluene (90 ml) at -45° C for 2 hours and then at -15° C for 1 hour. The usual work up gave 7.5g of crude aldehyde, which was crystallized from 2-propanol to provide 4.1g of 1-butyl-4-methoxy-2-naphthalenecarboxaldehyde, mp 51-52° C.

e) As in Example 14 d), 1-butyl-4-methoxy-2-naphthalenecarboxaldehyde (4.05g) and (carbomethoxymethylene) triphenylphosphorane (6g) in dichloromethane (40ml) was stirred at room temperature for 42 hours. Crystallization of the crude reaction product from the normal work up gave 4.1g of (E)-3-(1-butyl-4-methoxy-2-naphthalenyl)-2-propenoic acid methyl ester, mp 63-64° C.

f) As in Example 14 e), (E)-3-(1-butyl-4-methoxy-2-naphthalenyl)-2-propenoic acid methyl ester (4g) in methanol (20 ml) was treated with 2N sodium hydroxide (5 ml) at reflux for 1 hour and the normal isolation procedure afforded 3.8g of (E)-3-(1-butyl-4-methoxy-2-naphthalenyl)-2-propenoic acid. Crystallization of a sample from diethyl ether gave an analytical specimen, mp 166-167° C.

g) As in Example 14 f), (E)-3-(1-butyl 4-methoxy-2-naphthalenyl)-2-propenoic acid (3.7g) was reacted with 4-nitrophenol (1.99g) in dichloromethane (40 ml) in the presence of 1,3-dicyclohexylcarbodiimide (2.7g). The crude reaction product was crystallized from 2-propanol to give 4.63g of (E)-3-(1-butyl-4-methoxy-2-naphthalenyl)-2-propenoic acid 4-nitrophenyl ester, mp 121.5-122.5° C.

h) As in Example 14 g), (E)-3-(1-butyl-4-methoxy-2-naphthalenyl)-2-propenoic acid 4-nitrophenyl ester (2.03g) was reacted with (R)-α-methyl-3-pyridinebutanamine (0.83g) in tetrahydrofuran (30 ml) at room temperature overnight. The product was isolated in the usual manner and crystallized from ethyl acetate hexane to provide 1.76g of [R-(E)]-3-(1-butyl-4-methoxy-2 naphthalenyl)-N-[1-methyl-4-(3-pyridinyl)butyl]-2-propenamide, mp 133-134° C. Anal. Calcd. for $C_{28}H_{34}N_2O_2$: C 78.10, H 7.96, N 6.51; Found: C 78.18, H 8.06, N 6.49.

Example 22

As in Example 14 g), (E)-3-(1-butyl-4-methoxy-2-naphtha- lenyl)-2-propenoic acid 4-nitrophenyl ester (1.21g) was reacted with 5-pyrimidinehexanamine (0.505g) in tetrahydrofuran (20 ml) at room temperature overnight and then at 50° C for 1.5 hour. The product was isolated in the usual manner then was purified by HPLC (ethyl acetate) and twice crystallized from ethyl acetate hexane to provide 0.86g of (E)-3-(1-butyl-4-methoxy-2-naphthalenyl)-N-[6-(5-pyrimidinyl)hexyl]-2-propenamide, mp 128-129° C. Anal. Calcd. for $C_{28}H_{35}N_3O_2$: C 75.47, H 7.92 N 9.43; Found: C 75.52, H 7.95, N 9.40.

Example 23

a) As described in Example 18 f), a solution of 3-carboethoxy-4,4-bis(3-methoxyphenyl)-3-butenoic acid (20.5g) in dry tetrahydrofuran (100 ml) was treated with a solution of 1M borane in tetrahydofuran (57 ml) and the reaction was stirred at room temperature overnight. After the usual work up, the crude product was purified by HPLC (diethyl ether hexane; 65:35) to provide 15.4g of 2-hydroxyethyl-3,3-bis(3-methoxyphenyl)-2-propenoic acid ethyl ester, mp 102.5-103.5° C.

b) A solution of dimethylsulfoxide (3.8 ml) in dry dichloromethane (10 ml) was added with stirring to a cooled (below -60° C) solution of oxalyl chloride (4 ml) in dry dichloromethane (80 ml) at such a rate that the reaction temperature did not exceed -60° C. After 15 minutes a solution of 2-hydroxyethyl-3,3-bis(3-methoxyphenyl)-2-propenoic acid ethyl ester (15g) in dichloromethane (20 ml) was added while the reaction temperature was still maintained below -60° C. The reaction was allowed to proceed for 15 minutes then triethylamine (27 ml) was added to the chilled reaction and after an additional 20 minutes the cooling bath was removed and the mixture was allowed to equilibrate to room temperature. After 2N hydrochloric acid (100 ml) was added. the layers were separated and the aqueous phase was extracted with dichloromethane (25 ml), then the combined organic extracts were dried ($MgSO_4$) and evaporated to yield 14.8g of the crude aldehyde. The crude material was dissolved in dichloromethane (100 ml) containing trifluoroacetic acid (1 ml) and was left at room temperature for 4 hours. The usual work up gave 13g of crude acid which was purified by HPLC (ethyl acetate hexane; 1:4) to provide 7g of 7-methoxy-1-(3-methoxyphenyl)-2-naphthalenecarboxylic acid ethyl ester contaminated with a minor amount (about 12%) of the corresponding 5-methoxy isomer. This material was used without futher purification.

c) A solution of crude 7-methoxy-1-(3-methoxyphenyl)-2-naphthalenecarboxylic acid ethyl ester (4.1g) in a mixture of methanol (20 ml) and 4N potassium hydroxide (5 ml) was heated at reflux for 3 hours. After the methanol was evaporated off, the reaction was diluted with water and extracted with ether (2 x 25 ml). The aqueous layer was acidified, extracted with dichloromethane (3 x 25 ml) and the dried ($MgSO_4$) extracts evaporated to give 3.9g of crude 7-methoxy-1-(3-methoxyphenyl)-2-naphthalenecarboxylic acid. Crystallization from toluene hexane provided 3.05g of acid, mp 164-168° C.

d) A solution of borane in tetrahydrofuran (1M; 10 ml) was added to a solution of crude 7-methoxy-1-(3-methoxyphenyl)-2-naphthalenecarboxylic acid (2g) in tetrahydrofuran (10 ml) at 0-5° C and the mixture was stirred at room temperature for 3 hours. After the solvent was evaporated off, the reaction was diluted with 1N sodium hydroxide and extracted with dichloromethane. The organic layer was washed with brine then was dried ($MgSO_4$) and evaporated to give 1.75 g of crude 7-methoxy-1-(3-methoxyphenyl)-2-naphthalenemethanol.

e) 7-Methoxy-1-(3-methoxyphenyl)-2-naphthalenemethanol (1 75g) was oxidized using the reagent prepared as in Example 18 g) from oxalyl chloride (0.62 ml) and dimethylsulfoxide (0.9 ml) in dichloromethane (14 ml). The usual work up furnished 1.6g of 7-methoxy-1-(3-methoxyphenyl)- 2-naphthalenecarboxaldehyde.

f) A solution of 7-methoxy-1-(3-methoxyphenyl)-2-naphthalenecarboxaldehyde (1.6g) and (carbethoxymethylene) triphenylphosphorane (2.1g) in dichloromethane (15 ml) was stirred at room temperature overnight. The mixture was evaporated to dryness and the residue was dissolved in a mixture of methanol (15 ml) and 4N potassium hydroxide and heated at reflux for 1.25 hours. After the methanol was removed under reduced pressure, the reaction was diluted with water (15 ml) and was extracted with dichloromethane (3 x 10 ml) to remove neutral impurities. The aqueous phase was acidified and extracted with dichloromethane (6 x 25 ml). Then the combined extracts were dried ($MgSO_4$) and evaporated to furnish 1.35g of crude (E)-3-[7-methoxy-1-(3-methoxyphenyl)-2-naphthalenyl]-2-propenoic acid. Crystallization of the material from toluene hexane yielded 1.03g of the acid, mp 198.5-202° C.

g) As in Example 14 f), (E)-3-[7-methoxy-1-(3-methoxyphenyl)-2-naphthalenyl]-2-propenoic acid (0.982g) was reacted with 4-nitrophenol (0.493g) in dichloromethane (10 ml) in the presence of 1,3-dicyclohexylcarbodiimide (0.606g) at room temperature overnight. The usual work up provided 1.2g of (E)-3-[7-methoxy-1-(3-methoxyphenyl)-2-naphthalenyl]-2-propenoic acid 4-nitrophenyl ester.

h) As in Example 14 g), (E)-3-[7-methoxy-1-(3-methoxyphenyl)-2-naphthalenyl]-2-propenoic acid 4-nitrophenyl ester (1.2g) was reacted with 3-pyridinebutanamine (0.46g) in tetrahydrofuran (12 ml) for 2 hours at room temperature. The crude product, isolated in the usual manner was purified by using HPLC (ethyl acetate) and then was crystallized from diethyl ether to yield 0.83g of (E)-3-[7-methoxy-1-(3-methoxyphenyl)-2-naphthalenyl]-N-[4-(3-pyridinyl)butyl]-2-propenamide, mp 108.5-110° C. Anal. Calcd. for $C_{30}H_{30}N_2O_3 0.15\ H_2O$: C 76.78, H 6.51, N 5.97, $H_2O$ 0.57; Found: C 76.68, H 6.51, N 6.04, $H_2O$ 0.57.

Example 24

a) A solution of pentyl magnesium bromide (2M; 83 ml) was added with stirring to a cooled (-5°C) solution of 5-methoxy-1-indenone (24.33g) in dry tetrahydrofuran (100 ml) and the mixture was stirred at room temperature for 1.5 hours. A few chips of ice were added to destroy excess reagent and after the solvents had been removed under reduced pressure, the residue was taken up in a mixture of dichloromethane (250 ml) and 2N hydrochloric acid and stirred at room temperature for 3 hours. The separated aqueous phase was extracted with dichloromethane (150 ml) then the combined organic layers were dried ($MgSO_4$) and evaporated to provide 30g of crude product as an oil. purification of the mateial by HPLC (dichoromethane hexane; 2:5) gave 21.62g of 6-methoxy-3-pentyl-1H-indene as an oil.

b) As in Example 14 b), 6-methoxy-3-pentyl-1H-indene (21.6g) was reacted with the reagent formed from the addition of phosphorus oxychloride (10.25 ml) to dimethylformamide (70 ml) at -5°C. The cooling bath was withdrawn and after the reaction stirred at room temperature for 1.5 hours, excess reagent was destroyed by the addition of a few ice chips followed after 5 minutes by the addition of 10N sodium hydroxide (70 ml). The reaction was heated to 110°C for 10 minutes, then it was worked up in the described manner to give the crude aldehyde. Purification of the crude product by HPLC (dichloromethane hexane; 3:2) gave 8.6g of 6-methoxy-3-pentyl-1H-2-indenecarboxaldehyde as an oil.

c) As in Example 14 d), 6-methoxy-3-pentyl-1H-2-indenecarboxaldehyde (8.5g) and (carbomethoxymethylene)triphenylphosphorane (18.8g) in dichloromethane (75 ml) was stirred at room temperature for 42 hours. The crude reaction product from the normal work up was purified by HPLC (diethyl ether hexane; 1:3) to provide 6.4g of (E)-3-(6-methoxy-3- pentyl-1H-inden-2-yl)-2-propenoic acid methyl ester as an oil.

d) As in Example 14 e), (E)-3-(6-methoxy-3-pentyl-1H-inden-2-yl)-2-propenoic acid methyl ester (6g) in methanol (40 ml) was treated with 2N sodium hydroxide (20 ml) for 0.5 hours at reflux and worked up in the described manner. Crystallization of the crude product from ethyl acetate furnished 4.2g of (E)-3-(6-methoxy-3-pentyl-1H-inden-2-yl)-2-propenoic acid. A sample was recrystallized from ethyl acetate hexane to give the analytical specimen, mp 165-166°C.

e) A suspension of (E)-3-(6-methoxy-3-pentyl-1H-inden-2-yl)-2-propenoic acid (1.43g) in toluene (15 ml) at 0°C was treated dropwise with a solution of oxalyl chloride (1.09ml) in toluene (5 ml) and the reaction was stirred at room tempertature for 20 minutes. After the mixture was concentrated to ~ about half volume in vacuo, the resulting solution of crude acid chloride was added dropwise with stirring to a cooled (-75°C) solution of (R)-α-methyl- 3-pyridinebutanamine (0.825g) in toluene (25 ml). The cooling bath was removed and after 1.5 hours at room temperature, the reaction was diluted with toluene (50 ml) and washed with 1N sodium hydroxide solution. The dried ($K_2CO_3$) toluene layer was evaporated and the residue crystallized two times from diethyl ether hexane to furnish 1.55g of [R-(E)]-3-(6-methoxy-3-pentylindene-2-yl)-N-[1-methyl-4-(3-pyridinyl)butyl]-2-propenamide, mp 104-105.5°C. Anal. Calcd. for $C_{28}H_{36}N_2O_2$: C 77.74, H 8.39, N 6.47; Found: C 77.73, H 8.40, N 6.55.

Example 25

a) To a stirred solution of sodium 3-methoxythiophenolate (45g) in water (100 ml) at 15°C was added dropwise over 20 minutes 1-bromo-2-heptanone (54.4g). The reaction was stirred for 1 hour at room temperature then was extracted with diethyl ether (2 x 250 ml) and the ether extracts were washed in turn with water (2 x 100 ml) and 1N hydrochloric acid (2 x 100 ml). The dried ($MgSO_4$) organic layers were combined and evaporated to yield crude 1-(3-methoxyphenylthio)-2-heptanone (71g). This material (65g) was added over 30 minutes with stirring to sulfuric acid at -10°C. After 30 minutes at -5°C, the reaction mixture was diluted carefully with ice (200g) and the resulting solid was filtered off, washed with water and dried. Crystallization of the crude product from hexane gave 18.4g of 6-methoxy-3-pentylbenzo[b]thiophene, mp 43-45°C. A small sample was recrystallized from hexane to provide an analytical specimen. mp 45-47°C.

b) As in Example 14 b), 6-methoxy-3 pentylbenzo[b]thiophene (9.37g) was reacted with the reagent formed from the addition of phosphorus oxychloride (4.1 ml) to dimethylformamide (25 ml) at -5°C. The cooling bath was withdrawn and the reaction stirred at room temperature for 3 hours and then at 45°C overnight. excess reagent was destroyed by the addition of a few ice chips followed after 5 minutes by the addition of 10N sodium hydroxide (50 ml). The reaction was heated to 110°C for 10 minutes, then it was

worked up in the described manner to give 9g of crude aldehyde. Crystallization of the reaction product from hexane gave 7.83g of 6-methoxy-3-pentyl-2-benzo[b]thiophenecarboxaldehyde, mp 44-46° C. Recrystallization of a portion from ether furnished the pure aldehyde, mp 48.5-50° C.

c) As in Example 14 d), 6-methoxy-3-pentyl-2-benzo[b]thiophenecarboxaldehyde (7.37g) and (carbomethoxymethylene)triphenylphosphorane (9.7g) in dichloromethane (100 ml) was stirred at room temperature for 42 hours. Crystallization of the crude reaction product, obtained by normal work up, from hexane gave 7.6g of (E)-3-(6-methoxy3-pentyl-benzo[b]thien-2-yl)-2-propenoic acid methyl ester, mp 49-50° C.

d) As in Example 14 e), (E)-3-(6-methoxy-3-pentylbenzo[b]-thien-2-yl)-2-propenoic acid methyl ester (7.45g) in methanol (40 ml) was treated with 2N sodium hydroxide (25 ml) at reflux for 15 minutes and the normal isolation procedure afforded 7.1g of (E)-3-(6-methoxy-3-pentylbenzo[b]- thien 2-yl)-2-propenoic acid. Crystallization of a sample from 2 propanol provided an analytical specimen, mp 209-210° C.

e) As in Example 14 f), (E)-3-(6-methoxy-3-pentylbenzo[b]thien-2-yl)-2-propenoic acid (7g) was reacted with 4-nitrophenol (3.6g) in dichloromethane (70 ml) in the presence of 1,3-dicyclohexylcarbodiimide (4.8g). The crude reaction product was crystallized from dichloromethane/2-propanol to give 8.1g of (E)-3-(6-methoxy-3 pentylbenzo[b]thien-2-yl)-2-propenoic acid 4-nitrophenyl ester, mp 111-113° C.

f) As in Example 14 g), (E)-3-(6-methoxy-3-pentylbenzo[b]thien-2-yl)-2-propenoic acid 4-nitrophenyl ester (4.25g) was reacted with (R)-α-methyl-3-pyridinebutanamine (1.64g) in tetrahydrofuran (40 ml). The product was isolated in the usual manner and the crude amide was then crystallized from ethyl acetate to give 3.7g [R-(E)]-6-methoxy-3-pentylbenzo[b]thien-2-yl)-N-[1-methyl-4-(3-pyridinyl)butyl]-2-propenamide, mp 132-133° C. Anal. Calcd for $C_{27}H_{34}N_2O_2S$: C 71.96, H 7.61, N 6.22, S 7.11; Found: C 72.18, H 7.11, N 6.27, S 7.16.

## Example 26

a) A mixture of 2-chloro-3-oxooctanoic acid (51.5g) and sodium 3-methoxyphenolate (35g) was refluxed with stirring in benzene (250 ml) for 7 hours and then stirred at room temperature overnight. The cooled reaction was washed with water (2 x 250 ml) and the dried ($MgSO_4$) organic phase was evaporated to give 70g of a brown oil. The crude product was purified by HPLC (diethyl etherhexane; 1:19) to give 30.45g of 3-oxo-2-(3-methoxyphenyl)octanoic acid ethyl ester as a colorless oil. This material (21.5g) was added over 1 hour with stirring to sulfuric acid (22 ml) at -10° C. After 1 hour at -10° C, the reaction was diluted carefully with ice (400g) and then extracted with diethyl ether (2 x 400 ml). The organic extracts were washed in turn with saturated sodium bicarbonate solution and with brine, were combined, dried ($MgSO_4$) and evaporated. The crude reaction product was crystallized from hexane to give 10.86g of 6-methoxy-3-pentyl-2-benzofuranecarboxylic acid ethyl ester, mp 38-41° C.

b) As in Example 20 b), sodium bis(2-methoxyethoxy)aluminum hydride in toluene (3.4M; 33.6 ml) and toluene (30 ml) pretreated with N-methylpiperazine (13.5 ml) in toluene (20 ml) was reacted with 6-methoxy-3 pentyl-2-benzofurancarboxylic acid ethyl ester (6g) in toluene (90 ml) for 30 minutes at -45° C. The previously described work up yielded 4.82g of 6-methoxy-3-pentyl-2-benzofurancarboxaldehyde as an oil.

c) As in Example 14 d), 6-methoxy-3-pentyl-2-benzofurancarboxaldehyde (4.82g) and (carbomethoxymethylene)triphenylphosphorane (6.7g) in dichloromethane (75ml) was stirred at room temperature for 42 hours. Purification of the crude reaction product by HPLC (diethyl etherhexane; 1:12) gave 4.55g of (E)-3-(6-methoxy-3-pentylbenzofuran-2-yl)-2-propenoic acid methyl ester as an oil.

d) As in Example 14 e), (E)-3-(6-methoxy-3-pentylbenzofuran-2-yl)-2-propenoic acid methyl ester (4.25g) in methanol (25 ml) was treated with 2N sodium hydroxide solution (15 ml) at reflux for 1.25 hours. The crude reaction product was crystallized from ethyl acetate to provide 3.6g of (E)-3-(6-methoxy-3-pentylbenzofuran-2-yl)-2-propenoic acid, mp 146-147° C.

e) As in Example 14 f), (E)-3-(6-methoxy-3 pentylbenzofuran-2-yl)-2-propenoic acid (3.35g) was reacted with 4-nitrophenol (1.8g) in dichloromethane (35 ml) in the presence of 1,3-dicyclohexylcarbodiimide (2.42g). The crude product was crystallized from 2-propanol to afford 4.1g of (E)-3-(6-methoxy-3-pentylbenzofuran-2-yl)-2-propenoic acid 4-nitrophenyl ester, mp 120-121.5° C.

f) As in Example 14 g), (E)-3-(6-methoxy-3-pentylbenzofuran-2-yl)-2-propenoic acid 4-nitrophenyl ester (2.05g) was reacted with (R)-α-methyl-3-pyridinebutanamine (0.825g) in tetrahydrofuran (25 ml). The product was isolated in the usual manner and the crude amide was then crystallized from ethyl acetate hexane to provide [R-(E)]-3-(6-methoxy-3-pentylbenzofuran-2-yl)-N-[1-methyl-4-(3-pyridinyl)butyl]-2-propenamide, mp 122-124° C. Anal. Calcd. for $C_{27}H_{34}N_2O_3$: C 74.62, H 7.89, N 6.45; Found: C 74.69, H 7.77, N 6.37.

Example 27

a) A solution of 3-methoxy-N-methylaniline (9.8g) and 2-chloro-3-oxooctanenitrile (6.2g) in ethanol (25 ml) was heated at reflux for 17 hours then the solvent was removed under reduced pressure. The residue was dissolved in dichloromethane (100 ml) and washed with 1N hydrochloric acid (3 x 50 ml). The aqueous phases were washed with dichloromethane (50 ml) and the combined organic phases were dried ($K_2CO_3$) and evaporated. The residual oil was purified by HPLC (diethyl etherhexane: 3:7) to yield 6.42g of 2-(3-methoxy-N-methylanilino)-3-oxooctanenitrile as an oil. The above material was dissolved in trifluoroacetic acid (15 ml) and the solution was allowed to stand overnight at ambient temperature. The solvent was then evaporated and the residue was partitioned between dichloromethane (100 ml) and 1N sodium hydroxide (100 ml). The dried ($K_2CO_3$) organic phase was evaporated and the residue was purified by HPLC (diethyl etherhexane; 1:1) to give 3.2g of 6-methoxy-1-methyl-3-pentyl-2-indolecarbonitrile as an oil.

b) A solution of diisobutylaluminum hydride in toluene (1.5M; 8.5 ml) was added with stirring to a cooled (-40°C) solution of 6-methoxy-1-methyl-3-pentyl-2-indolecarbonitrile (2.85g) in dry toluene (25 ml). After 10 minutes, the cooling bath was removed and the reaction was stirred at room temperature for 2 hours followed by the careful addition of 5% sulfuric acid (100 ml). The mixture was heated at 40°C for 45 minutes then was cooled, diluted with toluene (75ml) and the layers were separated. The dried ($Na_2SO_4$) organic phase was evaporated to afford 2.81g of 6-methoxy-1-methyl-3-pentyl-2-indolecarboxaldehyde as an oil.

c) As in Example 14 d), 6-methoxy-1-methyl-3-pentyl-2-indolecarboxaldehyde (1.5g) and (carbomethoxymethylene)triphenylphosphorane (2.13g) in dichloromethane (25ml) was stirred at room temperature for 17 hours. Work up of the reaction in the usual way afforded 2g of (E)-3-(6-methoxy-1-methyl-3-pentylindol-2-yl)-2-propenoic acid methyl ester as an oil.

d) As in Example 14 e), (E)-3-(6-methoxy-1-methyl-3-pentyl- indol-2-yl)-2-propenoic acid methyl ester (2g) in methanol (25 ml) was treated with 4N sodium hydroxide (3 ml) at reflux for 2.5 hours. The crude reaction product was isolated in the usual manner giving 0.91g of (E)-3-(6- methoxy-1- methyl-3-pentylindol-2-yl)-2-propenoic acid. A small sample was crystallized from diethyl ether to give an analytical specimen, mp 157-158°C.

e) As in Example 24 e), (E)-3-(6-methoxy-1-methyl-3-pentylindol-2-yl)-2-propenoic acid in toluene (25 ml) was treated with a solution of oxalyl chloride (0.8 ml) in toluene (3 ml) for 1 hour at 0°C. After the reaction mixture was concentrated to about half volume in vacuo, the resulting solution of the crude acid chloride was added dropwise with stirring to a cooled (-75°C) solution of (R)-α- ethyl-3-pyridinebutanamine (0.535g) and triethylamine (0.85 ml) in toluene (15 ml). The cooling bath was removed and after 1 hour at room temperature the reaction was worked up in the usual manner. Purification of the crude product by HPLC (triethylamineethyl acetate; 1:66) followed by crystallization of the isolated amide from ethyl acetate furnished 0.9g of [R-(E)]-3-(6-methoxy-1-methyl-3-pentylindol-2-yl)-N-[1-ethyl-4-(3-pyridinyl)-butyl]-2-propenamide, mp 147-148°C. Anal. Calcd. for $C_{29}H_{39}N_3O_2$: C 75.45, H 8.52, N 9.10; Found: C 75.32, H 8.75, N 9.30.

Example A

| INHALATION AEROSOL FORMULATION (SOLUTION) | | |
|---|---|---|
| Item | Ingredients | % w/w |
| 1. | [R-(E)]-3-(1-Butyl-6-methoxy-2-naphthalenyl)-N-[1-methyl-4-(3-pyridinyl)butyl]-2-propenamide | 1.0 |
| 2. | Ethyl Alcohol | 30.0 |
| 3. | Ascorbic Acid | 0.5 |
| 4. | Freon 12 | 54.8 |
| 5. | Freon 114 | 13.7 |
| | TOTAL | 100% |

Manufacturing Procedure:

1) Dissolve Items 1 and 3 in Item 2.

2) Fill solution from Step 1 into a suitable glass bottle, insert valve and crimp to seal container.

3) Pressure-fill a 80:20 mixture of Items 4 and 5 into the container. NOTE: A suitable valve may be used to deliver 25 to 100 microliters in volume.

Example B

| INHALATION AEROSOL FORMULATION (SOLUTION) | | |
|---|---|---|
| Item | Ingredients | % w/w |
| 1. | [R-(E)]-3-(1-Butyl-6-methoxy-2-naphthalenyl)-N-[1-methyl-4-(3-pyridinyl)butyl]-2-propenamide | 1.0 |
| 2. | Sorbitan Trioleate | 0.5 |
| 3. | Freon 12 | 64.0 |
| 4. | Freon 11 | 18.5 |
| 5. | Freon 114 | 16.0 |
| | TOTAL | 100% |

Manufacturing Procedure:

1) Mix Items 1 and 2 into 4 and homogenize.

2) Fill the concentrate suspension from Step 1 into a suitable can and place in valve and crimp to seal container.

3) Pressure-fill a 80:20 mixture of Items 3 and 5. NOTE: A suitable valve may be used to deliver 25 to 100 microliters in volume.

Example C

TABLET FORMULATION

| Item | Ingredients | mg/tablet 100 mg | mg/tablet 500 mg |
|---|---|---|---|
| 1. | [R-(E)]-3-(1-Butyl-6-methoxy-2-naphthalenyl)-N-[1-methyl-4-(3-pyridinyl)butyl]-2-propenamide | 100 | 500 |
| 2. | Lactose | 30 | 150 |
| 3. | Pregelatinized Starch | 6 | 30 |
| 4. | Microcrystalline Cellulose | 30 | 150 |
| 5. | Magnesium Stearate | 1 | 6 |
| | TOTAL | 167 | 836 |

Manufacturing Procedure:

1) Mix Items 1, 2, 3 and 4 and granulate with water.
2) Dry the granulation at 50 °C.
3) Pass the granulation through suitable milling equipment.
4) Add Item 5 and mix for three minutes; compress on a suitable press.

Example D

| TABLET FORMULATION | | | | | |
|---|---|---|---|---|---|
| Item | Ingredients | mg/tablet | | | |
| 1. | [R-(E)]-3-(1-Butyl-6-methoxy-2-naphthalenyl)-N-[1-methyl-4-(3-pyridinyl)butyl]-2-propenamide | 0.1 | 0.5 | 5.0 | 25.0 |
| 2. | Lactose Anhydrous | 106.99 | 106.5 | 102.0 | 118.0 |
| 3. | Avicel | 15.0 | 15.0 | 15.0 | 25.0 |
| 4. | Modified Starch | 7.0 | 7.0 | 7.0 | 10.0 |
| 5. | Magnesium Stearate | 1.0 | 1.0 | 1.0 | 2.0 |
| | TOTAL | 130.0 | 130.0 | 130.0 | 180.0 |

Manufacturing Procedure:

1) Dissolve Item 1 in a suitable solvent such as alcohol.
2) Spread the solution in Step 1 over Item 2, dry.
3) Add Items 3 and 4 and mix for 10 minutes.
4) Add magnesium stearate, mix for 3 minutes and compress on a suitable press.

Example E

| CAPSULE FORMULATION | | | |
|---|---|---|---|
| Item | Ingredients | mg/capsule | |
| | | 100 mg | 500 mg |
| 1. | [R-(E)]-3-(1-Butyl-6-methoxy-2-naphthalenyl)-N-[1-methyl-4-(3-pyridinyl)butyl]-2-propenamide | 100 | 500 |
| 2. | Corn Starch (Pregelatinized) | 8 | 40 |
| 3. | Modified Starch | 4 | 20 |
| 4. | Talc | 4 | 20 |
| 5. | Magnesium Stearate | 1 | 2 |
| | TOTAL | 117 | 582 |

Manufacturing Procedure:

1) Mix Items 1, 2 and 3 and wet granulate with water. Dry at 45° C overnight.
2) Mill through suitable screen using appropriate milling equipment.
3) Add Items 4 and 5, mix for five minutes and fill into suitable capsules.

Example F

CAPSULE FORMULATION

| Item | Ingredients | mg/capsule | | | |
|---|---|---|---|---|---|
| 1. | [R-(E)]-3-(1-Butyl-6-methoxy-2-naphthalenyl)-N-[1-methyl-4-(3-pyridinyl)butyl]-2-propenamide | 0.01 | 0.5 | 5.0 | 25.0 |
| 2. | Lactose | 168.99 | 168.5 | 159.0 | 123.0 |
| 3. | Corn Starch | 20.0 | 20.0 | 25.0 | 35.0 |
| 4. | Talc | 10.0 | 10.0 | 10.0 | 15.0 |
| 5. | Magnesium Stearate | 1.0 | 1.0 | 1.0 | 2.0 |
| | TOTAL | 200.0 | 200.0 | 200.0 | 200.0 |

Manufacturing Procedure:

1) Mix Items 1, 2 and 3 in a suitable mixer for 30 minutes.
2) Add Items 4 and 5 and mix for 3 minutes.
3) Fill into suitable capsules.

Example G

| CREAM 0.5% | | |
|---|---|---|
| Ingredients | g/kg | Reasonable Variations |
| [R-(E)]-3-(1-Butyl-6-methoxy-2-naphthalenyl)-N-[1-methyl-4-(3-pyridinyl)butyl]-2-propenamide (3% excess) | 5.15 | ---- |
| Glycerol Monostearate | 100.00 | 80-120 |
| Polysorbate 60 | 20.00 | 15-25 |
| Cetyl Alcohol | 50.00 | 40-60 |
| Petrolatum | 70.00 | 50-90 |
| Methylparaben | 1.50 | 1.25-1.75 |
| Propylparaben | 0.50 | 0.4-0.6 |
| Propylene Glycol | 200.00 | 150-250 |
| Purified Water | 568.05 | 475-575 |
| TOTAL | 1,015.20 | |

The following examples describe the preparation of compounds of formula V:

## Example I

a) In an inert atmosphere, 26g of bis(triphenylphosphine) palladium dichloride and 2.28g of cuprous iodide were added to a stirred solution of 311.2g of (R,S)-4-pentyn-2-ol, 556.8g of 3-bromopyridine and 665 ml of triethylamine in 1.8l of dichloromethane at ambient temperature. After stirring for 75 minutes, the mildly exothermic reaction reached reflux temperature, and when the gentle boiling had subsided (40 minutes), external heat was applied to maintain reflux for 5 additional hours. The cooled reaction was stirred overnight at room temperature, then 1l of water and 500g of ice were added, followed by 420 ml of conc. hydrochloric acid (HCl) and the stirring was continued for several minutes. After the phases were separated, the aqueous layer was extracted with dichloromethane (4 x 1l) and then the organic layers were washed with 1l of 1N HCl. The original aqueous phase was treated with 500 ml of 10N sodium hydroxide (NaOH) and the second aqueous phase was basified with 200 ml of 10N NaOH before each was extracted in turn with dichloromethane (1 x 2l; 3 x 1l). The combined organic extracts were dried over potassium carbonate ($K_2CO_3$) and evaporated to constant weight under reduced pressure to yield 477.3g of crude (R,S)-α-methyl-4-(3-pyridinyl)-3-butyn-1-ol as an amber oil.

b) The crude (R,S)-α-methyl-4-(3-pyridinyl)-3-butyn-1-ol (477.3g) obtained in the previous Example was hydrogenated over 20g of platinum oxide in 3.5l of ethanol at room temperature and atmospheric pressure. After the uptake of hydrogen had stopped, the catalyst was filtered off and the solvent was removed under reduced pressure. The residual oil was distilled on a Kugelrohr apparatus (115-120° C/13.3 Pa (0.1mm)) to yield 420.5g of (R,S)-α-methyl-3-pyridinebutanol.

c) A stirred solution of 218.6g of oxalyl chloride in 1.5l of dry dichloromethane was cooled to -75° C under argon, then a mixture of 141g of dry dimethylsulfoxide in 200 ml of dichloromethane was added dropwise over 75 minutes such that the reaction temperature did not exceed -72° C. The mixture was stirred at -75° C for 10 minutes, then a solution of 271.5g of (R,S)-α-methyl-3-pyridinebutanol in 125 ml of dichloromethane was added dropwise over 55 minutes. while the reaction temperature was maintained below -70° C. After the addition of substrate was completed, the mixture was stirred at -75° C for another 30 minutes, then 520 ml of triethylamine was added over 65 minutes while the reaction temperature was maintained between -65° and -70° C. The cooling bath was removed, and after the reaction was allowed to equilibrate to room temperature over 1 hour, 1l of water was added and the phases were separated. The aqueous layer was extracted with dichloromethane (2 x 800 ml), and then the organic phase and extracts were washed in turn with 800 ml of 1.5N NaOH and with 800 ml of 10% sodium chloride (NaCl). The combined organic layers were dried ($K_2CO_3$) and evaporated to yield 266g of crude ketone. The product was distilled to yield 248.6g of 5-(3-pyridinyl)-2-pentanone (bp 100-102° C/26.7 Pa (0.2mm)).

d) A mixture of 248.5g of 5-(3-pyridinyl)-2-pentanone, 95.85g of sodium cyanoborohydride and 1170g of ammonium acetate in 5.3l of dry methanol was stirred at room temperature for 8 days, then 3l of methanol was removed by distillation under reduced pressure (internal temp. about 30° C). The reaction was cooled in an ice bath as 3.8l of 6N HCL was added dropwise over 2 hours. After the mixture was stirred at room temperature overnight, it was made strongly basic by the addition of 2l of 12.5N NaOH and extracted with dichloromethane (1 x 2l; 2 x 1l). The combined extracts were dried ($K_2CO_3$) and evaporated to yield 244g of a light brown oil, which was distilled to give 205g of (R,S)-α-methyl-3-pyridinebutanamine (bp 95-100° C/20 Pa (0.15 mm))

## Example II

a) In an inert atmosphere, a solution of 2.42 ml of methanesulfonyl chloride in 10 ml of dichloromethane was added over 10 minutes to a stirred mixture of 5.0g of (R,S)-α-methyl-3-pyridinebutanol and 6.2ml of triethylamine in 50 ml of dry dichloromethane maintained at -40° C. After 30 minutes the reaction was warmed to 0° C, then a small piece of ice was added and the mixture was stirred in a an ice bath for another 15 minutes. The solution was then washed in turn with water (3 x 15 ml), 1N NaOH (2 x 15 ml) and brine (10 ml). The dried ($K_2CO_3$) organic layer was evaporated to furnish 7.2g of (R,S)-methanesulfonic acid 5-(3-pyridinyl)-2 pentyl ester.

b) A mixture of 2.5g of (R,S)-methanesulfonic acid 5-(3-pyridinyl)-2-pentyl ester, 0.832g of sodium

azide, 1.5 ml of water and 15 ml of dimethylformamide was stirred at 50° C under argon for 150 minutes. The cooled solution was diluted with 40 ml of water and extracted with dichloromethane (3 x 30 ml). The extracts were washed in turn with water (2 x 20 ml) and then were combined, dried ($K_2CO_3$) and evaporated to furnish 1.76g of (R,S)-3-(4-azidopentyl)pyridine as an oil.

c) A solution of 0.9g of (R,S)-3-(4-azidopentyl)pyridine in 25 ml of ethanol was hydrogenated over 0.05g of 10% Pd/C at 345 kPa (50 psi). After 105 minutes, the catalyst was removed by filtration and the solvent was removed under reduced pressure to yield 0.69g of a colorless oil. Evaporative distillation of the crude product furnished 0.57g of (R,S)-α-methyl-3-pyridinebutanamine.

## Example III

a) A solution of 86.3g of (R,S)-4-bromo-2-methylbutanoic acid ethyl ester and 104.9g of triphenylphosphine in 600 ml of toluene was stirred at reflux for 4 days. As the reaction proceeded, the phosphonium bromide separated from solution as an oil. After the reaction was cooled, the toluene supernatant was decanted and replaced with 500 ml of fresh toluene. The mixture was stirred at reflux for 30 minutes, then was cooled and the toluene layer was again decanted. After this process was repeated a second time. the residual oil was dried in vacuo to give 187g of (R,S)-3-(ethoxycarbonyl)-butyltriphenylphosphonium bromide as a viscous oil.

b) A stirred solution of 10.56g of sodium hydride (60% dispersion in oil) in 1000 ml of dry dimethylsulfoxide was heated at 70° C until the evolution of hydrogen stopped (30 minutes), then the solution was cooled to 0° C and a solution of 103.7g of (R,S)-3-(ethoxycarbonyl)butyltriphenylphosphonium bromide in 200 ml dimethylsulfoxide was added. After the mixture had stirred at room temperature for 30 minutes, a solution of 20.8 ml of 3-pyridinecarboxaldehyde in 100 ml of tetrahydrofuran was added and the reaction was stirred at room temperature overnight. The mixture was diluted with ice-water and extracted with dichloromethane (6 x 150 ml). The combined organic layers were then extracted with 4 x 400 ml of 0.5N HCl. The acidic layers were made basic with 125 ml triethylamine and extracted with dichloromethane (5 x 150 ml). The dried ($K_2CO_3$) extracts were evaporated to furnish 34g of crude product. An initial purification of the material by high pressure liquid chromatography (HPLC) (etherhexane; 3:2) yielded 11.9g of a mixture of (Z)- and (E)-isomers (4:1). A subsequent separation of the mixture by HPLC with recycle gave 6.77g of [(R,S)-Z]-2-methyl-5-(3-pyridinyl)-4-pentenoic acid ethyl ester and 3g of a mixture of [(R,S)-Z]-2-methyl-5-(3-pyridinyl)-4-pentenoic acid ethyl ester and its (E)-isomer.

c) A solution of 5.5g of a mixture (1:1) of [(R,S)-E]- and [(R,S)-Z]-2-methyl-5-(3-pyridinyl)-4-pentenoic acid ethyl ester in 100 ml was ethanol was hydrogenated over 0.4g of 10% palladium on carbon (Pd/C). After the uptake of hydrogen had stopped, the catalyst was removed by filtration and the solvent evaporated to give 5.33g of (R,S)-α-methyl-3- pyridinepentanoic acid ethyl ester.

d) A mixture of 5.3g of (R,S)-α-methyl-3-pyridinepentanoic acid ethyl ester, 35 ml of 1N NaOH and 35 ml of methanol was stirred at reflux for 3 hours, then most of the methanol was removed under reduced pressure. The solution was diluted to 100 ml with water and extracted with dichloromethane (3 x 35 ml). The aqueous layer was then neutralized with 35 ml of 1N HCl and extracted with dichloromethane (3 x 30 ml). The Extracts were then dried over sodium sulfate ($Na_2SO_4$) and evaporated to yield 3.47g of (R,S)-α-methyl-3-pyridinepentanoic acid.

e) A solution of 1.93g of (R,S)-α-methyl-3-pyridinepentanoic acid, 2.21 ml of diphenylphosphorylazide and 1.4 ml of triethylamine in 10 ml of t-butanol was stirred at reflux under argon overnight. After the solvents were removed under reduced pressure, the residue was dissolved in 100 ml of dichloromethane and washed with 2 x 50 ml portions of 1N NaOH. The aqueous layers were washed in turn with 50 ml of dichloromethane. Then the combined organic extracts were dried ($K_2CO_3$) and evaporated to yield 2.45g of crude product. The material was purified by HPLC (ethyl acetate) to furnish 2.2g of (R,S)-[1-methyl-4-(3-pyridinyl)butyl]carbamic acid 1,1-dimethylethyl ester as a colorless oil.

f) A solution of 2.1g of (R,S)-[1-methyl-4-(3-pyridinyl) butyl]carbamic acid 1,1-dimethylethyl ester in 25 ml of 1N HCl was heated on a steam bath for 75 minutes then was cooled and extracted with 50 ml of ether. In an atmosphere of argon, the aqueous layer was treated with 6 ml of 10N NaOH and extracted with 2 x 50 ml portions of dichloromethane. Evaporation of the dried ($K_2CO_3$) extracts and evaporative distillation of the product (95-100° C/26.7 (Pa 0.2mm)) gave 1.25g of (R,S)-α-methyl-3-pyridinebutanamine.

## Example IV

a) To a mixture of 10.6g of (R,S)-α-methyl-3-pyridinebutanol in 30 ml of acetonitrile was added 20 ml of sulfuric acid. After the reaction was stirred at 50°C for 2 hours, it was poured over a mixture of 500g of ice and 400 ml of 4N NaOH and extracted with dichloromethane (2 x 150 ml). Evaporation of the dried ($K_2CO_3$) extracts gave 6g of crude product which was purified by HPLC (methanol ethyl acetate; 1:49) and triturated with ether to yield 2.9g of (R,S)-N-[1-methyl-4-(3-pyridinyl)butyl]acetamide, mp 70-71.5°C.

b) A solution of 2.06g of (R,S)-N-[1-methyl-4-(3-pyridinyl) butyl]acetamide in 50 ml of 6N HCl was stirred at reflux for 22 hours. In an argon atmosphere, the cooled mixture was made basic with the careful addition of 30 ml of 10N NaOH and was extracted with dichloromethane (2 x 75 ml). The extracts were washed with brine, then were combined, dried ($K_2CO_3$) and evaporated to give 1.43g of (R,S)-α-methyl-3-pyridinebutanamine.

## Example V

a) A solution of 281.5g of 1,3-dicyclohexylcarbodiimide in 400 ml of dimethylformamide was added to a stirred solution of 204g of (R,S)-α-methyl-3-pyridinebutanamine, 198.4g of (R)-mandelic acid and 209.75g of 1-hydroxybenzotriazole in 1400 ml of dimethylformamide, maintained at -10°C during the addition by intermittent cooling with a dry ice acetone bath. After stirring at -5° for 4 hours, then at room temperature overnight the mixture was recooled to 0°C for 2 hours. The precipitated solids were filtered and washed in turn with cold dimethylformamide (2 x 150 ml) and ethyl acetate (2 x 300 ml). This material, a mixture of 1.3-dicyclohexylurea (DCU) and the less soluble (R*,R)-mandelamide, was dispersed in 2l of 1N HCl and stirred at room temperature for 3 hours. The undissolved solids (DCU) were removed by filtration and were washed with 200 ml of dilute HCl and with water. The filtrate was basified and the resulting material was collected by filtration, washed with water and dried in vacuo to give 64.4g of [R-(R*,R*)]-α-hydroxy-N-[1-methyl-4-(3-pyridinyl)butyl]benzeneacetamide, the (R*,R)-mandelamide, mp 144-146°C; $[\alpha]_D^{25}$ -27.8° (c 1.0, MeOH).

The original mother liquors and washings were concentrated to dryness under reduced pressure and the residue was dispersed in 2l of 1.5N NaOH and extracted with dichloromethane (1 x 2l; 2 x 1l). The organic extracts were washed in turn with 1N NaOH (2 x 800 ml) and then in turn with 1N HCl (1 x 1.5l; 2 x 750 ml). The combined acidic aqueous layers were basified with 350 ml of 10N NaOH and extracted with dichloromethane (1 x 2; 2 x 1l). The extracts were dried ($K_2CO_3$) and evaporated to give 280g of mandelamide, rich (about 3:2) in the (S*,R)-diastereomer. The residue was crystallized three times from 2-propanol to yield 74.1g of the less soluble (R*,R)-mandelamide. mp 144-146°C.

The mother liquors from the final two crystallizations were combined, evaporated and the residue crystallized twice from 2-propanol to give an additional 7.2g of the (R*,R)-diastereomer, mp 143-145°C. The total yield of [R-(R*,R*)]-α-hydroxy-N-[1-methyl-4-(3-pyridinyl)butyl] benzeneacetamide, obtained in three crops. was 145.6g (78.5%).

b) A solution of 70.5 g of 5-(3-pyridinyl)-2-pentanone and 53.5g of (R)-(+)-alpha-methylbenzylamine in 700 ml of toluene containing 1.8g of p-toluenesulfonic acid was heated at reflux for 17 hours. Water was removed from the reaction as it was formed using a Dean Stark trap. The cooled solution was hydrogenated over 70g of Raney Nickel at room temperature and 345 kpa (50 psi). When approximately 50% of the theoretical amount of hydrogen had been taken up, the reaction essentially stopped. The spent catalyst was removed and replaced with 70g of fresh Raney Nickel and the hydrogenation was continued until the absorption of hydrogen ceased. After the catalyst was removed by filtration, the filtrate was washed with 250 ml of 1N sodium hydroxide solution, then was dried and evaporated to give 106g of an oil. HPLC analysis of the product showed that the main component (about 68%) was [R-(R*,R)]-N-[1-methyl-(3-pyridinyl)-butyl]-α-methylbenzylamine along with 13% of the related (S*,R)-diastereomer.

The above mixture (105g) in 1l of ethanol was hydrogenolysed over 21g of 20% $Pd(OH)_2$ on charcoal (50°C; 172 kPa (25 psi) for a total of 51 hours. After the catalyst was removed by filtration, the solvent was evaporated and the residue distilled to provide 33.6g of α-methyl-3-pyridinebutanamine enriched in the (R)-enantiomer.

To a cooled (-5°C) solution of 32g of the above enriched amine, 33g of 1-hydroxybenzotriazole and 31.22g of (R)-mandelic acid in 350 ml of dimethylformamide, was added a solution of 44.26g of 1,3-dicyclohexylcarbodiimide in 150 ml of dimethylformamide and the mixture was stirred at -5°C for 18 hours.

After the precipitated dicyclohexylurea was removed by filtration, the filtrate was evaporated and the residue dispersed in 300 ml of cold 2N sodium hydroxide. The resulting solids were removed by filtration, washed with dilute sodium hydroxide solution and with water and then dissolved in 500 ml +f 2N hydrochloric acid. The acidic solution was extracted with dichloromethane (3 x 150 ml) to remove neutral impurities, then was basified with 10N sodium hydroxide and extracted with dichloromethane (6 x 300 ml). The dried extracts were evaporated to give 55g of residual solid. Crystallization of the product from ethanol gave 25.6g of [R-(R*,R)]-α-hydroxy-N-[1-methyl-4-(3-pyridinyl)butyl]benzeneacetamide, mp 141-143° C. An additional 1.3g of product, mp 141-143° C. was obtained from the mother liquors.

c) A solution of 145g of [R-(R*,R*)]-α-hydroxy-N-[1-methyl-4-(3-pyridinyl)butyl]benzeneacetamide in 900 ml of 6N HCl was treated with 80 ml of conc. HCl and then was heated at reflux for 2 days. After most of the solvent was removed under reduced pressure, the residue was made decidedly basic with 10N NaOH in an argon atmosphere, and extracted with dichloromethane (1 x 1.21; 2 x 600 ml). The dried ($K_2CO_3$) extracts were evaporated and the crude product was distilled to give 78.5g of (R)-αa-methyl-3-pyridinebutanamine, (bp 95° C/26.7 Pa (0.2mm)).

## Example VI

a) Under the conditions described in Example I a, 395g of 3-bromopyridine and 259.3g of (R,S)-5-hexyn-3-ol were reacted together in 1.51 of dichloromethane in the presence of 418 ml of triethylamine, 17.56g of bis(triphenylphosphine)palladium dichloride and 1.7g of cuprous iodide. The usual work-up furnished 361.5g of crude (R,S)-alpha-ethyl-4-(3-pyridinyl)- 3-butyn-1-ol as a brown oil.

b) As in Example Ib, hydrogenation of 361.5g of crude (R,S)-α-ethyl-4-(3-pyridinyl)-3-butyn-1-ol over 15g of platinum oxide in 31 of ethanol at room temperature and atmospheric pressure and distillation of the product furnished 357g of (R,S)-α-ethyl-3-pyridinebutanol (bp 120-130° /13.3 Pa (0.1mm)) as a colorless oil.

c) As in Example I c, 356.6g of (R,S)-α-ethyl-3-pyridinebutanol was added to a mixture prepared in the prescribed manner from 211.7g of oxalyl chloride and 170g of dimethylsulfoxide in 1.751 of dichloromethane. After the addition of 630 ml of triethylamine, the reaction was worked up in the usual way to yield 347.6g of crude product which was distilled to give 327.9g of 5-(3-pyridinyl)-3-hexanone (bp 110-115° C/13.3 Pa (0.1mm)).

d) In the manner described in Example I d, 372.9g of 6-(3-pyridinyl)-3-hexanone was reacted with 116.5g of sodium cyanoborohydride and 1426g of ammonium acetate in 6.51 of dry methanol for 3 days at room temperature, and then 4.51 of 6N HCl was added and the mixture stirred overnight. Distillation of the crude product gave 289.4g of (R,S)-α-ethyl- 3-pyridinebutanamine (bp 95-100° C/13.3 Pa 0.1 mm)).

## Example VII

a) As in Example V a, a solution of 367.4g of 1,3-dicyclohexylcarbodiimide in 500 ml of dimethylformamide was added to a stirred solution of 289g of (R,S)-α-ethyl-3-pyridinebutanamine, 259g of (R)-mandelic acid and 274g of 1-hydroxybenzotriazole in 1.71 of dimethylformamide maintained at -10° C during the addition. After stirring at -5° C for 3 hours, then at room temperature overnight. the mixture was recooled to 0° C for 2 hours. The precipitated solids were filtered and washed in turn with cold dimethylformamide (3 x 150 ml) and ethyl acetate (3 x 200ml). The solids, a mixture of 1,3-dicyclohexylurea (DCU) and the less soluble (R*,R)- mandelamide, was dispersed in 1N HCl (2l) and stirred at room temperature for 4 hours. The undissolved solids (DCU) were removed by filtration and were washed with 200 ml dilute HCl and with water. The filtrate was basified and the resulting crystalline material was collected by filtration, washed with water and dried in vacuo to give 195.4g of [R-(R*,R*)]-α-hydroxy-N-[1-ethyl-4-(3-pyridinyl)butyl]benzeneacetamide, mp 161.5-163° C; $[\alpha]_{25@D}$ -14.9° (c 1.0, MeOH).

b) As in Example V c, a solution of 195g of [R-(R*,R*)]-α-hydroxy-N-[1-ethyl-4-(3-pyridinyl)butyl]-benzeneacetamide in 1.11 of 6N HCl was treated with 104 ml of conc. HCl and then was heated at reflux for 2 days. The crude amine, obtained by the normal work up, was distilled to give 109g of (R)-α- ethyl-3-pyridinebutanamine, (bp 105° C/26.7 (Pa 0.2mm)); $[\alpha]_D^{25}$ -11.9° (c 1.0, MeOH)

## Claims

1. Compounds of the general formula

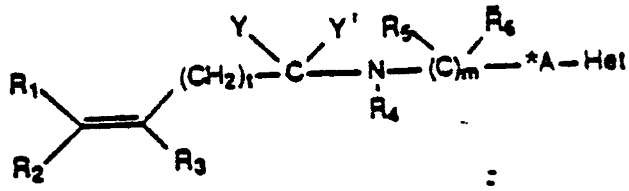

I

wherein Y and Y′ are hydrogen or taken together are O or S, *A is paraphenylene or *-$(CH_2)_n$-$(X)_s$-$(CH_2)_r$-, X is O, S or -CH=CH-, n and r, independently, are integers from 0 to 3, m is the integer 0 or 1, s is the integer 0 or 1, provided that when s is 1, n+m is at least 2, t is an integer from 0 to 10, $R_1$ and $R_2$, independently, are lower alkyl, lower alkenyl or aryl, or one of $R_1$ and $R_2$ is hydrogen and the other is

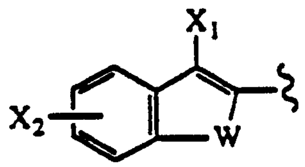

(a)

W is -$CX_3$=$CX_4$-, -$CH_2$-$CH_2$-, -$CH_2$-, O, S or -$NX_5$-, $X_1$ is lower alkyl, phenyl or phenyl with up to 3 substituents selected from lower alkoxy, lower alkyl and halogen, $X_2$, $X_3$ and $X_4$, independently, are hydrogen, lower alkyl, lower alkoxy or halogen, $X_5$ is lower alkyl, $R_3$ is hydrogen, lower alkyl or aryl, $R_4$ is hydrogen, lower alkyl, aryl, aryl-lower alkyl or acyl, $R_5$ is hydrogen or lower alkyl, $R_6$ is hydrogen, lower alkyl, lower cycloalkyl, Het-lower alkyl or aryl, Het is a monocyclic 6-membered heteroaromatic radical containing one or two nitrogen atoms, which radical may be substituted by lower alkyl, halogen or aryl, and the asterisk denotes the point of attachment, when $R_5$ and $R_6$ are different, enantiomers and racemic mixtures thereof, when $R_1$ and $R_2$ are different, geometric isomers thereof, and pharmaceutically acceptable acid addition salts thereof.

2. Compounds in accordance with claim 1, wherein Y and y′ are hydrogen or taken together are O or S, *A is paraphenylene or *-$(CH_2)_n$-$(X)_3$-$(CH_2)$r-, X is O, S or -CH=CH-, n and r, independently, are integers from 0 to 3, m is the integer 0 or 1, s is the integer O or 1, provided that when s is 1, n+m is at least 2, t is an integer from 0 to 10, $R_1$ and $R_2$, independently, are lower alkyl, lower alkenyl or aryl, $R_3$ is hydrogen, lower alkyl or aryl, $R_4$ is hydrogen, lower alkyl, aryl, aryl-lower alkyl, lower alkanoyl, aryl-lower alkanoyl or aroyl, $R_5$ is hydrogen or lower alkyl, $R_6$ is hydrogen, lower alkyl, lower cycloalkyl, Het-lower alkyl or aryl, Het is a monocyclic 6-membered heteroaromatic radical, containing 1 or 2 nitrogen atoms, which radical may be substitued by lower alkyl, halogen or aryl, and the asterisk denotes the point of attachment.

3. Compounds in accordance with claim 1 or 2, wherein R and $R_2$, independently are lower alkyl or aryl, $R_3$ is hydrogen or lower alkyl, $R_4$ and $R_5$ are hydrogen, $R_6$ is hydrogen, lower alkyl or lower cycloalkyl, *A is *-$(CH_2)_n$-$(X)_s$-$(CH_2)_r$-, n+r is an integer from 2 to 6, m is the integer 1, s is the integer 0, t is an integer from 0 to 4, Het is a monocyclic 6-membered heteroaromatic radical containing one or two nitrogen atoms, which radical may be substituted by lower alkyl, and Y and Y′ are as defined in claim 1.

4. Compounds in accordance with claim 3, wherein $R_1$ is lower alkyl or aryl, $R_2$ is aryl, $R_3$ is hydrogen or lower alkyl, $R_6$ is hydrogen, lower alkyl or cyclopropy, Het is pyridinyl or pyrimidinyl unsubstituted or substituted by lower alkyl, n + r is 3, t is the integer 0 or 2, Y and Y′ are hydrogen or taken together are O.

5. Compounds in accordance with Claim 4, wherein $R_1$ is butyl, pentyl, hexyl, phenyl or phenyl with up to 3 substituents selected from halogen and lower alkoxy, $R_2$ is phenyl or phenyl with up to 3 substituents selected from halogen and lower alkoxy, $R_3$ is hydrogen, Het is 3-pyridinyl or 2-methyl-3-pyridinyl and t is 2.

6. Compounds in accordance with Claim 1, wherein $R_1$ is hydrogen, $R_2$ is

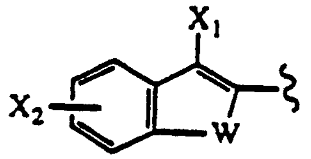

(a)

$R_3$ is hydrogen or lower alkyl, $R_4$ and $R_5$ are hydrogen, $R_6$ is hydrogen, lower alkyl or lower cycloalkyl, *A is *-$(CH_2)_n$-$(X)_s$-$(CH_2)_r$-, n+r is an integer from 2 to 6, m is the integer 1, s is the integer 0, t is an integer from 0 to 4, Het is a monocyclic 6-membered heteroaromatic radical containing one or two nitrogen atoms, which radical may be substituted by lower alkyl, and $X_1$, $X_2$, W, Y and Y′ are as defined in Claim 1.

7. Compounds in accordance with Claim 6, wherein $R_1$ is hydrogen, $R_2$ is

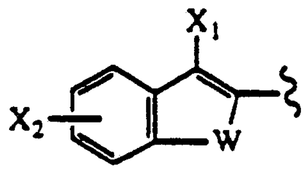

(a)

$R_3$ is hydrogen or lower alkyl, $R_6$ is hydrogen, lower alkyl or cyclopropyl, Het is pyridinyl or pyrimidinyl unsubstituted or substituted by lower alkyl, n + r is 3, Y and Y′ are hydrogen or taken together are O, and $X_1$, $X_2$ and W are as defined in claim 1.

8. Compounds in accordance with Claim 7, wherein $R_1$ is hydrogen, $R_2$ is

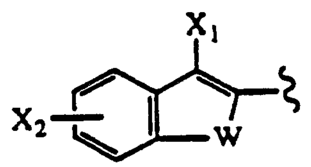

(a)

W is $-CX_3=CX_4-$, $-CH_2-$, O, S or $-NX_5-$, $X_1$ is butyl, pentyl, hexyl, phenyl or phenyl with up to 3 substituents selected from halogen and lower alkoxy, $X_2$, $X_3$ and $X_4$, independently, are hydrogen, lower alkoxy or halogen, $X_5$ is lower alkyl, $R_3$ is hydrogen, Het is 3-pyridinyl or 2-methyl-3-pyridinyl and Y and Y′ taken together are O.

9. 5,5-Bis(4-methoxyphenyl)-N-[4-(3-pyridinyl)butyl]-4-pentenamide.

10. 5,5-Diphenyl-N-[4-(3-pyridinyl)butyl]-4-pentenamide.

11. N-(5,5-Diphenyl-4-pentenyl)-3-pyridinebutanamine.

12. 5,5-Bis(4-fluorophenyl)-N-[4-(3-pyridinyl)butyl]-4-pentenamide.

13. [R-(E)]-3-(1-Butyl-6-methoxy-2-naphthalenyl)-N-[1-methyl-4-(3-pyridinyl)butyl]-2-propenamide.

14. [R-(E)]-3-(1-Butyl-4-methoxy-2-naphthalenyl)-N-[1-methyl-4-(3-pyridinyl)butyl]-2-propenamide.

15. [R-(E)]-3-(6-Methoxy-3-pentylinden-2-yl)-N-[1-methyl-4-(3-pyridinyl)butyl]-2-propenamide.

16. [R-(E)]-3-(6-Methoxy-3-pentylbenzo[b]thien-2-yl)-N-[1-methyl-4-(3-pyridinyl)butyl]-2-propenamide.

17. [R-(E)]-3-(6-Methoxy-3-pentylbenzofuran-2-yl)-N-[1-methyl-4- (3-pyridinyl)butyl]-2-propenamide.

18. R-(E)]-3-(6-Methoxy-1-methyl-3-pentylindol-2-yl)-N-[1-ethy 1- 4-(3-pyridinyl)butyl]-2-propenamide.

19. Compounds in accordance with any one of claims 1-18 for use as therapeutically active substances.

20. Compounds in accordance with any one of claims 1-18 for use as platelet activating factor (PAF) antagonists.

21. A process for the manufacture of compounds in accordance with any one of claims 1-18 and their pharmaceutically acceptable acid addition salts, which process comprises

a) reacting a carboxylic acid of the general formula

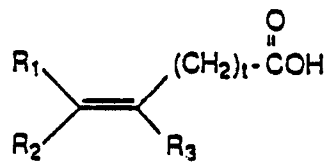

II

wherein $R_1$, $R_2$, $R_3$ and t are as defined in claim 1, in the presence of a coupling agent or a reactive derivative of a carboxylic acid of the formula II above with an amine of the general formula

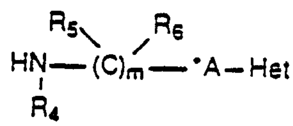

V

wherein $R_4$, $R_5$, $R_6$, *A, Het and m are as defined in claim 1, or

b) reacting a compound of the general formula

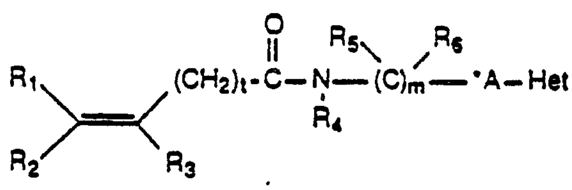

Ia

wherein $R_1$, $R_2$, $R_3$, $R_4$, $R_5$, $R_6$, *A, Het, m and t are as defined in claim 1, with phosphorous pentasulfide, or

c) reacting a compound of the general formula

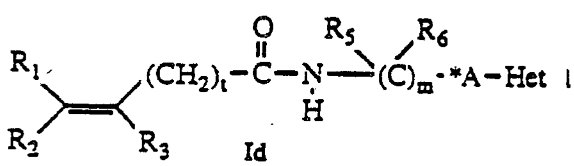

Id

or

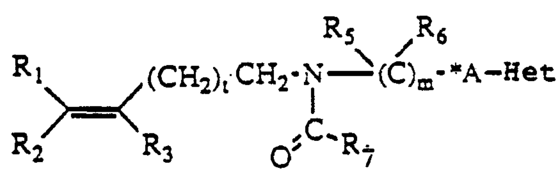

If

wherein $R_1$, $R_2$, $R_3$, $R_5$, $R_6$, *A, Het, m and t are as defined in claim 1 and $R_7$ is $C_{1-6}$-alkyl, aryl-$C_{1-6}$-alkyl or aryl, with a reducing agent, or

d) reacting a compound of the general formula

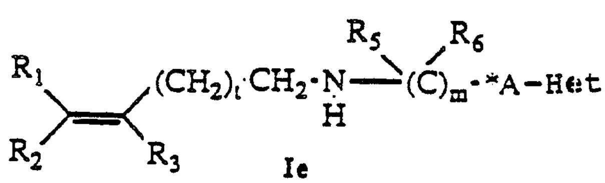

Ie

wherein $R_1$, $R_2$, $R_3$, $R_5$, $R_6$, *A, Het, m and t are

as defined in claim 1, with an acylating agent yielding the group $R_7$-CO-, wherein $R_7$ is as defined above, and

e) if desired, converting a compound into a pharmaceutically acceptable acid addition salt.

22. A pharmaceutical composition comprising a compound in accordance with any one of claims 1-18 and a therapeutically inert carrier material.

23. A pharmaceutical composition having platelet acting factor (PAF) antagonistic activity and comprising a compound in accordance with any one of claims 1-18 and a therapeutically inert carrier material.

24. The use of compounds in accordance with any one of claims 1-18 in the prevention or treatment of diseases.

25. The use of compounds in accordance with any one of claims 1-18 in the prevention or treatment of disease states characterized by excess platelet activating factor.

26. The use of compounds in accordance with any one of claims 1-18 in the prevention or treatment of cardiovascular diseases, pulmonary diseases. immunological disorders, inflammatory diseases, dermatological disorders, shocks or transplant rejections.

27. The use of compounds in accordance with any one of claims 1-18 in the manufacture of pharmaceutical compositions having platelet activating factor (PAF) antagonistic activity.

28. The use of compounds in accordance with any one of claims 1-18 for the manufacture of pharmaceutical compositions useful in the prevention or treatment of cardiovascular diseases, pulmonary diseases, immunological disorders, inflammatory diseases, dermatological disorders, shocks or transplant rejections.

Claims for the following Contracting States : GR; ES

1. A process for the manufacture of compounds of the general formula

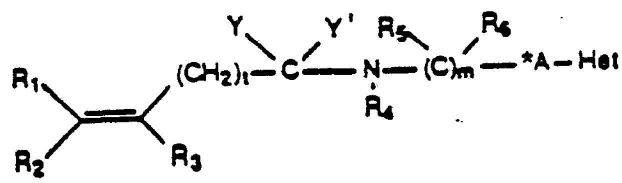

I

wherein Y and Y′ are hydrogen or taken together are O or S, *A is paraphenylene or *-$(CH_2)_n$-$(X)_s$-$(CH_2)_r$-, X is O, S or -CH=CH-, n and r, independently, are integers from 0 to 3, m is the integer 0 or 1, s is the integer 0 or 1, provided that when s is 1, n+m is at least 2, t is an integer from 0 to 10, $R_1$ and $R_2$, independently, are lower alkyl, lower alkenyl or aryl, or one of $R_1$ and $R_2$ is hydrogen and the other is

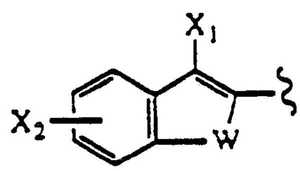

(a

W is -$CX_3$=$CX_4$-, -$CH_2$-$CH_2$-, -$CH_2$-, O, S or -$NX_5$-,
$X_1$ is lower alkyl, phenyl or phenyl with up to 3 substituents selected from lower alkoxy, lower alkyl and halogen, $X_2$, $X_3$ and $X_4$, independently, are hydrogen, lower alkyl, lower alkoxy or halogen, $X_5$ is lower alkyl, $R_3$ is hydrogen, lower alkyl or aryl, $R_4$ is hydrogen, lower alkyl, aryl, aryl-lower alkyl or acyl, $R_5$ is hydrogen or lower alkyl, $R_6$ is hydrogen, lower alkyl, lower cycloalkyl, Het-lower alkyl or aryl, Het is a monocyclic 6-membered heteroaromatic radical containing one or two nitrogen atoms, which radical may be substituted by lower alkyl, halogen or aryl, and the asterisk denotes the point of attachment,
when $R_5$ and $R_6$ are different, of enantiomers and racemic mixtures thereof, when $R_1$ and $R_2$ are different, of geometric isomers thereof, and of pharmaceutically acceptable acid addition salts thereof, which process comprises

a) reacting a carboxylic acid of the general formula

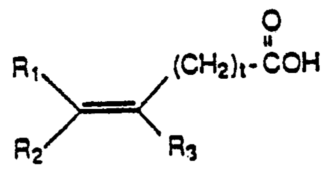

II

wherein $R_1$, $R_2$, $R_3$ and t are as defined above, in the presence of a coupling agent or a reactive derivative of a carboxylic acid of the formula II above with an amine of the general formula

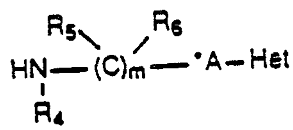

V

wherein $R_4$, $R_5$, $R_6$, *A, Het and m are as defined above, or

b) reacting a compound of the general formula

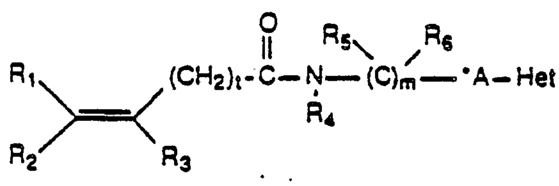

Ia

wherein $R_1$, $R_2$, $R_3$, $R_4$, $R_5$, $R_6$, *A, Het, m and t are as defined above, with phosphorous pentasulfide, or

c) reacting a compound of the general formula

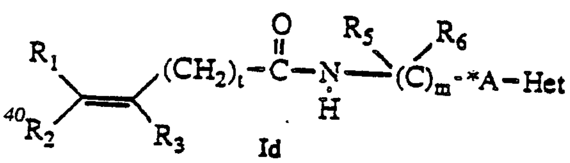

Id

or

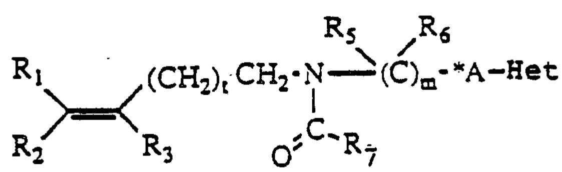

If

wherein $R_1$, $R_2$, $R_3$, $R_5$, $R_6$, *A, Het, m and t are as defined above and $R_7$ is $C_{1-6}$-alkyl, aryl-$C_{1-6}$-alkyl or aryl, with a reducing agent, or

d) reacting a compound of the general formula

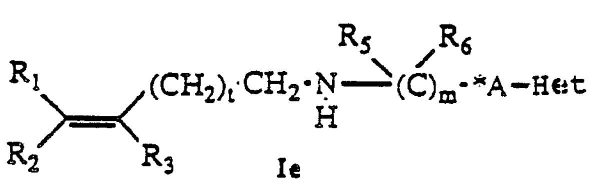

Ie

wherein $R_1$, $R_2$, $R_3$, $R_5$, $R_6$, *A, Het, m and t are as defined above with an acylating agent yielding the group $R_7$-CO-, wherein $R_7$ is as defined above, and

e) if desired, converting a compound of formula I obtained into a pharmaceutically acceptable acid addition salt.

2. A process in accordance with claim 1, wherein Y and Y′ are hydrogen or taken together are O or S, *A is paraphenylene or *-$(CH_2)_n$-$(X)_3$-$(CH_2)$r-, X is O, S or -CH=CH-, n and r, independently, are integers from 0 to 3, m is the integer 0 or 1, s is the integer 0 or 1, provided that when s is 1, n+m is at least 2, t is an integer from 0 to 10, $R_1$ and $R_2$, independently, are lower alkyl, lower alkenyl or aryl, $R_3$ is hydrogen, lower alkyl or aryl, $R_4$ is hydrogen, lower alkyl, aryl, aryl-lower alkyl, lower alkanoyl, aryl-lower alkanoyl or aroyl, $R_5$ is hydrogen or lower alkyl, $R_6$ is hydrogen, lower alkyl, lower cycloalkyl, Het-lower alkyl or aryl, Het is a monocyclic 6-membered heteroaromatic radical, containing 1 or 2 nitrogen atoms, which radical may be substituted by lower alkyl, halogen or aryl, and the asterisk denotes the point of attachment.

3. A process in accordance with claim 1 or 2, wherein $R_1$ and $R_2$, independently are lower alkyl or aryl, $R_3$ is hydrogen or lower alkyl, $R_4$ and $R_5$ are hydrogen, $R_6$ is hydrogen, lower alkyl or lower cycloalkyl, *A is *-$(CH_2)_n$-$(X)_s$-$(CH_2)_r$- n+r is an integer from 2 to 6, m is the integer 1, s is the integer 0, t is an integer from 0 to 4, Het is a monocyclic 6-membered heteroaromatic radical containing one or two nitrogen atoms, which radical may be substituted by lower alkyl, and Y and Y′ are as defined in claim 1.

4. A process in accordance with claim 3, wherein $R_1$ is lower alkyl or aryl, $R_2$ is aryl, $R_3$ is hydrogen or lower alkyl, $R_6$ is hydrogen, lower alkyl or cyclopropy, Het is pyridinyl or pyrimidinyl unsubstituted or substituted by lower alkyl, n + r is 3, t is the integer 0 or 2, Y and Y′ are hydrogen or taken together are O.

5. A process in accordance with Claim 4, wherein $R_1$ is butyl, pentyl, hexyl, phenyl or phenyl with up to 3 substituents selected from halogen and lower alkoxy, $R_2$ is phenyl or phenyl with up to 3 substituents selected from halogen and lower alkoxy, $R_3$ is hydrogen, Het is 3-pyridinyl or 2-methyl-3-pyridinyl and t is 2.

6. A process in accordance with Claim 1, wherein $R_1$ is hydrogen, $R_2$ is

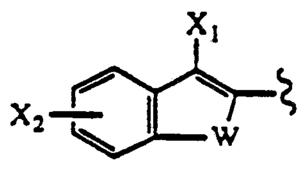

(a)

$R_3$ is hydrogen or lower alkyl, $R_4$ and $R_5$ are hydrogen, $R_6$ is hydrogen, lower alkyl or lower cycloalkyl, *A is *-$(CH_2)_n$-$(X)_s$-$(CH_2)_r$-, n+r is an integer from 2 to 6, m is the integer 1, s is the integer 0, t is an integer from 0 to 4, Het is a monocyclic 6-membered heteroaromatic radical containing one or two nitrogen atoms, which radical may be substituted by lower alkyl, and $X_1$, $X_2$, W, Y and Y′ are as defined in Claim 1.

7. A process in accordance with Claim 6, wherein $R_1$ is hydrogen, $R_2$ is

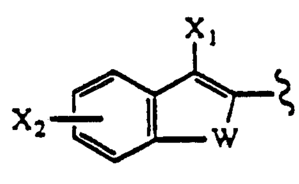

(a)

$R_3$ is hydrogen or lower alkyl, $R_6$ is hydrogen, lower alkyl or cyclopropyl, Het is pyridinyl or pyrimidinyl unsubstituted or substituted by lower alkyl, n + r is 3, Y and Y′ are hydrogen or taken together are O, and $X_1$, $X_2$ and W are as defined in claim 1.

8. A process in accordance with Claim 7, wherein $R_1$ is hydrogen, $R_2$ is

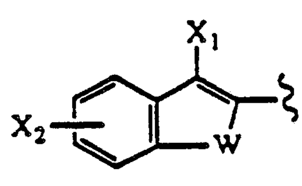

(a;

W is -$CX_3$=$CX_4$-, -$CH_2$-, O, S or -$NX_5$-, $X_1$ is butyl, pentyl, hexyl, phenyl or phenyl with up to 3 substituents

selected from halogen and lower alkoxy, $X_2$, $X_3$ and $X_4$, independently, are hydrogen, lower alkoxy or halogen, $X_5$ is lower alkyl, $R_3$ is hydrogen, Het is 3-pyridinyl or 2-methyl-3-pyridinyl and Y and Y′ taken together are O.

9. A process in accordance with claim 1, wherein 5,5-Bis(4-methoxyphenyl)-N-[4-(3-pyridinyl)butyl]-4-pentenamide is prepared.

10. A process in accordance with claim 1, wherein 5,5-Diphenyl-N-[4-(3-pyridinyl)butyl]-4-pentenamide is prepared.

11. A process in accordance with claim 1, wherein N-(5,5-Diphenyl-4-pentenyl)-3-pyridinebutanamine is prepared.

12. A process in accordance with claim 1, wherein 5,5-Bis(4-fluorophenyl)-N-[4-(3-pyridinyl)butyl]-4-pentenamide is prepared.

13. A process in accordance with claim 1, wherein [R-(E)]-3-(1-Butyl-6-methoxy 2-naphthalenyl)-N-[1-methyl-4-(3-pyridinyl)butyl]-2-propenamide is prepared.

14. A process in accordance with claim 1, wherein [R-(E)]-3-(1-Butyl-4-methoxy-2-naphthalenyl)-N-[1-methyl-4-(3-pyridinyl)butyl]-2-propenamide is prepared.

15. A process in accordance with claim 1, wherein 15. [R-(E)]-3-(6-Methoxy-3-pentylinden-2-yl)-N-[1-methyl-4-(3-pyridinyl)butyl]-2-propenamide is prepared.

16. A process in accordance with claim 1, wherein [R-(E)]-3-(6-Methoxy-3-pentylbenzo[b]thien-2-yl)-N-[1-methyl4-(3-pyridinyl)butyl]-2-propenamide is prepared.

17. A process in accordance with claim 1, wherein [R-(E)]-3-(6-Methoxy-3-pentylbenzofuran-2-yl)-N-[1-methyl-4-(3-pyridinyl)butyl]-2-propenamide is prepared.

18. A process in accordance with claim 1, wherein R-(E)]-3-(6-Methoxy-1-methyl-3-pentylindol-2-yl)-N-[1-ethyl-4-(3-pyridinyl)butyl]-2-propenamide is prepared.

19. A process for the manufacture of a pharmaceutical composition, particularly to be used in the control or prevention of disease states characterized by excess platelet activating factor or of cardiovascular dieseases, pulmonary diseases, immunological disorders, inflammatory diseases, dermatological disorders, shocks or transplant rejections, which comprises bringing a compound of formula I, as defined in claim 1 or a pharmaceutically acceptable acid addition salt thereof, and, if desired, one or more other therapeutically active substances together with a therapeutically inert carrier into a galenical administration form.

20. The use of a compound of formula I as defined in claim 1 or a pharmaceutically acceptable acid addition salt thereof for the manufacture of pharmaceutical compositions which exhibit activity as platelet activating factor antagonist.